(19) Europäisches Patentamt
European Patent Office
Office européen des brevets

(11) EP 4 545 524 A1

(12) **EUROPEAN PATENT APPLICATION**
published in accordance with Art. 153(4) EPC

(43) Date of publication:
**30.04.2025 Bulletin 2025/18**

(21) Application number: **23826588.8**

(22) Date of filing: **25.06.2023**

(51) International Patent Classification (IPC):
***C07D 413/14*** (2006.01) ***C07D 417/14*** (2006.01)
***A61K 31/422*** (2006.01) ***A61K 31/427*** (2006.01)
***A61K 31/433*** (2006.01) ***A61P 37/02*** (2006.01)
***A61P 35/02*** (2006.01) ***A61P 25/00*** (2006.01)
***A61P 29/00*** (2006.01)

(52) Cooperative Patent Classification (CPC):
**A61K 31/422; A61K 31/427; A61K 31/433; A61K 31/454; A61K 31/4545; A61K 31/496; A61K 31/5377; A61P 25/00; A61P 29/00; A61P 35/00; A61P 35/02; A61P 37/02; C07D 413/14; C07D 417/14**

(86) International application number:
**PCT/CN2023/102216**

(87) International publication number:
**WO 2023/246944 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.06.2022 CN 202210731254**

(71) Applicant: **Shanghai Institute of Materia Medica, Chinese Academy of Sciences**
**Shanghai 201203 (CN)**

(72) Inventors:
- **CHEN, Xiaohua**
  **Shanghai 201203 (CN)**
- **LI, Jia**
  **Shanghai 201203 (CN)**
- **NIE, Huijun**
  **Shanghai 201203 (CN)**
- **ZHOU, Yubo**
  **Shanghai 201203 (CN)**
- **HU, Tengfei**
  **Shanghai 201203 (CN)**
- **WANG, Yujie**
  **Shanghai 201203 (CN)**
- **XU, Gaoya**
  **Shanghai 201203 (CN)**
- **KAN, Weijuan**
  **Shanghai 201203 (CN)**
- **XU, Bingyue**
  **Shanghai 201203 (CN)**

(74) Representative: **Lavoix**
**Bayerstraße 83**
**80335 München (DE)**

(54) **SUBSTITUTED 4-AMINOISOINDOLINE COMPOUND, PREPARATION METHOD THEREFOR, PHARMACEUTICAL COMPOSITION THEREOF, AND USE THEREOF**

(57) The present invention relates to a substituted 4-aminoisoindoline compound, a preparation method therefor, a pharmaceutical composition thereof, and use thereof. The compound has a structure represented by formula (I). Specifically, the substituted 4-aminoisoindoline compound provided by the present invention has good anti-tumor activity as a $CRL4^{CRBN}$E3 ubiquitin ligase modulator, and can be used for preparing a medicament for the treatment of $CRL4^{CRBN}$E3 ubiquitin ligase-related diseases.

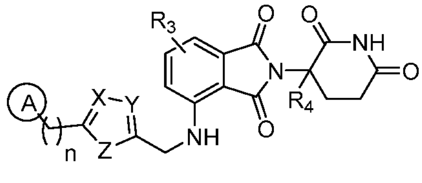

(I)

EP 4 545 524 A1

## Description

### TECHNICAL FIELD

The present invention relates to the field of medicinal chemistry, specifically to substituted 4-aminoisoindoline compounds, their preparation methods, pharmaceutical compositions, and applications.

### BACKGROUND OF THE INVENTION

In recent years, a strategy known as targeted protein degradation (TPD) has been developed to address the issues of resistance associated with small-molecule inhibitors and to design effective drug molecules for "undruggable" target proteins. TPD is a rapidly emerging technology in drug discovery that leverages the ubiquitin-proteasome system (UPS) to degrade disease-related proteins, thereby achieving therapeutic effects. This technology has become a research hotspot in the field of drug development, offering broad application prospects in both drug discovery and biomedical research. Currently, UPS-based targeted protein degradation technologies primarily include Proteolysis Targeting Chimeras (PROTACs) and molecular glues. Although the PROTAC field is rapidly advancing, it faces several challenges: 1. Most PROTACs have a molecular weight exceeding 1000 Da, resulting in poor bioavailability and cell permeability. This does not conform to Lipinski's rule of five, leading to suboptimal pharmacokinetic (PK) and pharmacodynamic (PD) properties. 2. The synthesis of PROTAC molecules is complex and costly. 3. PROTAC molecules exert their effects through a catalytic cycle, making it difficult to accurately evaluate their PK and PD properties using traditional methods. Currently, no mature PK and PD evaluation system exists for PROTAC molecules. These challenges underscore the need for further advancements in TPD technologies to overcome the limitations of PROTACs and enhance their therapeutic potential.

Molecular glues are small molecules that induce protein-protein interactions, enabling precise temporal control over various biological processes. These molecules act on protein interfaces to form novel protein-protein interactions. By bringing a target protein into the physical proximity of a regulatory protein, molecular glues reduce the reliance on active binding pockets within the target protein, thereby significantly expanding the druggable proteome. Molecular glue-mediated proximity-induced protein degradation offers inherent advantages over PROTACs: 1. Molecular glues have smaller molecular weights compared to PROTACs, facilitating the optimization of physicochemical properties and better adherence to Lipinski's rule of five. 2. Molecular glues are more conducive to improving bioavailability and pharmacokinetic properties compared to PROTACs. 3. Molecular glues are relatively simpler to synthesize, reducing the complexity and cost of production. 4. From a functional perspective, while the mechanisms of action for target proteins and PROTACs are typically well-defined, the discovery of molecular glues often leads to the identification of novel modes of action and new drug targets. These attributes highlight the potential of molecular glues as a transformative approach in drug discovery, offering unique opportunities for expanding therapeutic strategies.

Thalidomide, lenalidomide, and pomalidomide, collectively referred to as immunomodulatory drugs (IMiDs), have been recently identified to function through a molecular glue mechanism as part of their clinical therapeutic strategy. Their direct target is cereblon (CRBN) (Science, 2010, 327, 1345; Science, 2014, 343, 301; Science, 2014, 343, 305; Nature, 2015, 523, 183). Immunomodulatory drugs target the $CRL4^{CRBN}$E3 ubiquitin ligase and bind to CRBN, causing subtle conformational adjustments at the protein-binding interface. This enables the recruitment of different substrate proteins, which are subsequently ubiquitinated and marked for proteasomal degradation. The downstream effects of substrate protein degradation mediate the antiproliferative and immunomodulatory activities of immunomodulatory drugs. These findings reveal that different IMiD molecules exhibit distinct substrate degradation specificities upon interaction with the CRBN target, leading to varying pharmacological activities and entirely different therapeutic effects. This diversity enables their clinical application for different indications. For example, lenalidomide achieves efficacy in treating multiple myeloma by degrading IKZF1 and IKZF3, whereas its therapeutic effect on 5q deletion-associated myelodysplastic syndrome (MDS) is achieved by degrading CK1$\alpha$. With the development of new IMiD compounds, the indications for this class of molecules continue to expand. Currently, several molecular glue degraders within this category are in clinical development.

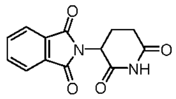
Thalidomide

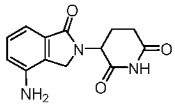
Lenalidomide

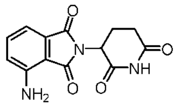
Pomalidomide

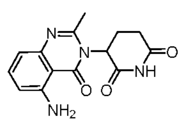
CC-122

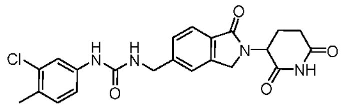
CC-885

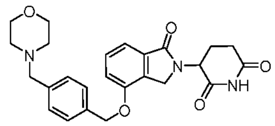
CC-220

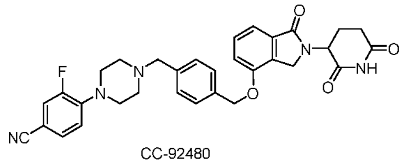
CC-92480

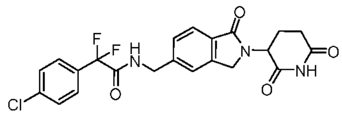
CC-90009

Currently, lenalidomide is primarily used for the treatment of multiple myeloma and myelodysplastic syndromes (MDS); however, its efficacy for other indications remains suboptimal. Meanwhile, its analogs, such as CC-122, CC-220, CC-92480, and CC-90009, are still in clinical research stages. Therefore, the development of structurally novel molecular glue degraders targeting the $CRL4^{CRBN}$E3 ubiquitin ligase has become a promising strategy. By modifying and optimizing the chemical structure to enhance ligand interactions and affinity with the ligase, substrate degradation efficiency can be improved, thereby enhancing therapeutic outcomes in tumors and expanding the indications of molecular glue degraders. From a mechanistic perspective, structural modifications of IMiDs often induce conformational changes in CRBN, which can lead to the discovery of novel modes of action and new drug targets. The development of new molecular glue degraders targeting the $CRL4^{CRBN}$ E3 ubiquitin ligase enables the recruitment and degradation of new substrate proteins, potentially facilitating the treatment of additional indications. In summary, developing novel molecular glue degraders targeting the $CRL4^{CRBN}$E3 ubiquitin ligase not only improves tumor treatment outcomes but also broadens their therapeutic indications. This approach addresses unmet medical needs and holds significant research value and practical significance.

## Summary of the invention

The object of the present invention is to provide a structurally novel molecular glue degrader targeting the $CRL4^{CRBN}$E3 ubiquitin ligase, which enhances tumor treatment efficacy and broadens the therapeutic indications of molecular glue degraders.

In a first aspect, the present invention provides a compound of Formula (I), or a pharmaceutically acceptable salt, tautomer, stereoisomer, or prodrug thereof:

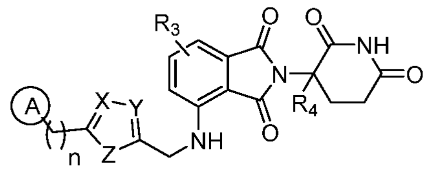

(I)

wherein, n is 0 or 1;

X and Y are each independently selected from CH or N, and X and Y are not both CH simultaneously;

Z is selected from O, S, or N;

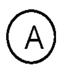

is selected from: C6-C10 aryl, C4-C10 cycloalkyl, 4-10 membered heterocyclyl, 5-10 membered heteroaryl, wherein the C6-C10 aryl, C4-C10 cycloalkyl, 4-10 membered heterocyclyl, and 5-10 membered heteroaryl may optionally be substituted with one or more substituents selected from the group consisting of: hydrogen, deuterium, halogen, cyano, nitro, hydroxy, carboxy, amide, 4-10 membered heterocyclyl, $C_1$-$C_6$ alkyl, $C_1$-$C_6$ alkoxy, halogen-substituted $C_1$-$C_6$ alkyl, halogen-substituted $C_1$-$C_6$ alkoxy, substituted or unsubstituted phenyl, $C_2$-$C_6$ alkenyl, C3-C8 heterocyclyl-O-, C3-C8 cycloalkyl-O-, hydroxy-substituted $C_1$-$C_6$ alkoxy, $C_1$-$C_6$ alkoxy-substituted $C_1$-$C_6$ alkoxy, -C(O)-$R_1$, -(CH2)m-$R_2$; wherein the substituted or unsubstituted phenyl may optionally be substituted with one or more substituents selected from the group consisting of: hydrogen, halogen, cyano, nitro, hydroxy, carboxy, 4-10 membered heterocyclyl, C1-C6 alkyl, C1-C6 alkoxy, halogen-substituted C1-C6 alkyl, halogen-substituted C1-C6 alkoxy.

$R_1$ is selected from: halogen-substituted or unsubstituted C1-C6 alkyl, C4-C12 cycloalkyl, C6-C10 aryl, 4-10 membered heterocyclyl, -CH2-(C4-C12 cycloalkyl), wherein the C4-C12 cycloalkyl, C6-C10 aryl, 4-10 membered heterocyclyl, and -CH2-(C4-C12 cycloalkyl) may optionally be substituted with one or more substituents selected from the group consisting of: hydrogen, deuterium, halogen, cyano, nitro, hydroxy, carboxy, 4-10 membered heterocyclyl, C1-C6 alkyl, C1-C6 alkoxy, halogen-substituted C1-C6 alkyl, halogen-substituted C1-C6 alkoxy, halogen-substituted or unsubstituted phenyl;

$R_2$ is C6-C10 aryl, and the C6-C10 aryl may optionally be substituted with one or more substituents selected from the group consisting of: hydrogen, halogen, cyano, nitro, hydroxy, carboxy, 4-10 membered heterocyclyl, $C_1$-$C_6$ alkyl, $C_1$-$C_6$ alkoxy, halogen-substituted $C_1$-$C_6$ alkyl, halogen-substituted $C_1$-$C_6$ alkoxy;
m is 0 or 1;
$R_3$ is hydrogen or halogen;
$R_4$ is hydrogen, deuterium, or fluorine.

In another preferred embodiment, n is 0.
In another preferred embodiment, Z is selected from O or S.
In another preferred embodiment, X is N, Y is C, and Z is O; or X is N, Y is C, and Z is S.
In another preferred embodiment,

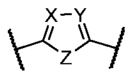

is selected from:

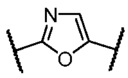, 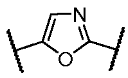,

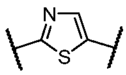, 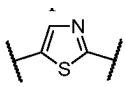, 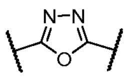, 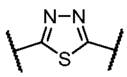.

In another preferred embodiment,

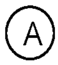

is

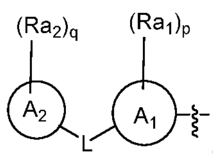,

wherein A1 is selected from: phenyl, C4-C6 cycloalkyl, 4-6 membered heterocyclyl, 5-6 membered heteroaryl;

L is none, CO, or $CH_2$;
$A_2$ is selected from: none, $C_1$-$C_6$ alkyl, halogen-substituted $C_1$-$C_6$ alkyl, phenyl, C4-C6 cycloalkyl, 4-6 membered heterocyclyl, 5-6 membered heteroaryl;
$Ra_1$ and $Ra_2$ are each independently selected from: hydrogen, deuterium, halogen, cyano, nitro, hydroxy, carboxy, amide, 4-10 membered heterocyclyl, $C_1$-$C_6$ alkyl, $C_1$-$C_6$ alkoxy, halogen-substituted $C_1$-$C_6$ alkyl, halogen-substituted $C_1$-$C_6$ alkoxy, substituted or unsubstituted phenyl, $C_2$-$C_6$ alkene, C3-C8 heterocyclyl-O-, C3-C8 cycloalkyl-O-, hydroxy-substituted $C_1$-$C_6$ alkoxy, $C_1$-$C_6$ alkoxy-substituted $C_1$-$C_6$ alkoxy;wherein the substituted or unsubstituted phenyl is optionally substituted with one or more substituents selected from the group consisting of: hydrogen,

halogen, cyano, nitro, hydroxy, carboxy, 4-10 membered heterocyclyl, $C_1$-$C_6$ alkyl, $C_1$-$C_6$ alkoxy, halogen-substituted $C_1$-$C_6$ alkyl, halogen-substituted $C_1$-$C_6$ alkoxy;
p and q are each independently 0, 1, 2, 3, 4, or 5.

In another preferred embodiment,

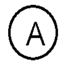

is selected from: phenyl, 5-6 membered heteroaryl, nitrogen-containing 4 or 6 membered heterocyclyl, and the phenyl, 5-6 membered heteroaryl, nitrogen-containing 4 or 6 membered heterocyclyl are optionally substituted with one or more substituents selected from the group consisting of: hydrogen, deuterium, halogen, cyano, morpholino, $C_1$-$C_6$ alkyl, $C_1$-$C_6$ alkoxy, halogen-substituted $C_1$-$C_6$ alkyl, halogen-substituted $C_1$-$C_6$ alkoxy, substituted or unsubstituted phenyl, $C_2$-$C_6$ alkene, C3-C8 heterocyclyl-O-, C3-C8 cycloalkyl-O-, hydroxy-substituted $C_1$-$C_6$ alkoxy, $C_1$-$C_6$ alkoxy-substituted $C_1$-$C_6$ alkoxy, -C(O)-$R_1$, -($CH_2$)-$R_2$, wherein the substituted or unsubstituted phenyl is optionally substituted with one or more substituents selected from the group consisting of: hydrogen, halogen, cyano, nitro, hydroxy, carboxy, 4-10 membered heterocyclyl, C1-C6 alkyl, C1-C6 alkoxy, halogen-substituted C1-C6 alkyl, halogen-substituted C1-C6 alkoxy; $R_1$ and $R_2$ have the definitions as mentioned above; Preferably, R1 is selected from: halogen-substituted or unsubstituted $C_1$-$C_6$ alkyl, C4-C12 cycloalkyl, phenyl, -$CH_2$-(C4-C12 cycloalkyl), and the C4-C12 cycloalkyl, phenyl, -$CH_2$-(C4-C12 cycloalkyl) are optionally substituted with one or more substituents selected from the group consisting of: hydrogen, deuterium, halogen, cyano, $C_1$-$C_6$ alkyl, $C_1$-$C_6$ alkoxy, halogen-substituted $C_1$-$C_6$ alkyl, halogen-substituted $C_1$-$C_6$ alkoxy, halogen-substituted or unsubstituted phenyl;
$R_2$ is phenyl, and the phenyl is optionally substituted with one or more substituents selected from the group consisting of: halogen, cyano, $C_1$-$C_6$ alkyl, $C_1$-$C_6$ alkoxy, halogen-substituted $C_1$-$C_6$ alkyl, halogen-substituted $C_1$-$C_6$ alkoxy.
In another preferred embodiment, the compound has the structure shown in formula II.

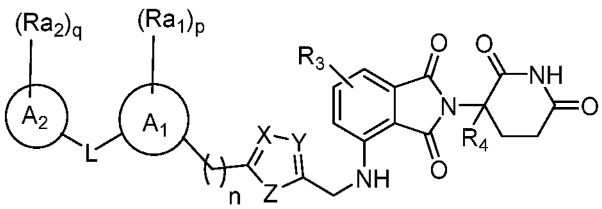

(II)

Wherein, n is 0 or 1;

X and Y are independently selected from: CH or N, and X and Y are not both CH simultaneously;
Z is selected from: O, S, or NH;
$R_3$ is hydrogen or halogen;
$R_4$ is hydrogen, deuterium, or fluorine;
$A_1$ is selected from: phenyl, C4-C6 cycloalkyl, 4-6 membered heterocyclyl, 5-6 membered heteroaryl;
L is absent, CO, or $CH_2$;
$A_2$ is selected from: absent, C1-C6 alkyl, phenyl, C4-C10 cycloalkyl, 4-6 membered heterocyclyl, 5-6 membered heteroaryl.

$Ra_1$ and $Ra_2$ are independently selected from: hydrogen, deuterium, halogen, cyano, nitro, hydroxyl, carboxyl, amide carbonyl, 4-10 membered heterocyclyl, $C_1$-$C_6$ alkyl, $C_1$-$C_6$ alkoxy, halogen-substituted $C_1$-$C_6$ alkyl, halogen-substituted $C_1$-$C_6$ alkoxy, substituted or unsubstituted phenyl, $C_2$-$C_6$ alkenyl, C3-C8 heterocyclyl-O-, C3-C8 cycloalkyl-O-, hydroxyl-substituted $C_1$-C6 alkoxy, and $C_1$-$C_6$ alkoxy-substituted $C_1$-$C_6$ alkoxy; wherein the substituted or unsubstituted phenyl is optionally substituted by one or more substituents selected from the group consisting of: hydrogen, halogen, cyano, nitro, hydroxyl, carboxyl, 4-10 membered heterocyclyl, C1-C6 alkyl, C1-C6 alkoxy, halogen-substituted C1-C6 alkyl, and halogen-substituted C1-C6 alkoxy ;
p and q are independently 0, 1, 2, 3, 4, or 5.
In another preferred embodiment, $A_1$ is selected from: phenyl, pyridyl, piperidyl, cyclohexyl, piperazine, and azetidine.
In another preferred embodiment, L and $A_2$ are both absent.

In another preferred embodiment, the compound has the structure shown in formula (I-1) to (I-4):

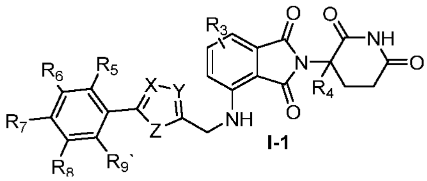

I-1

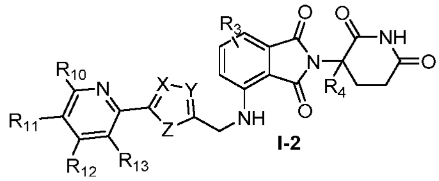

I-2

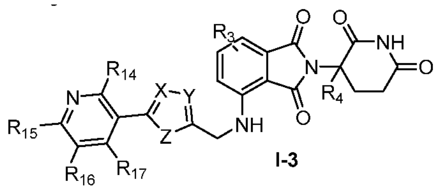

I-3

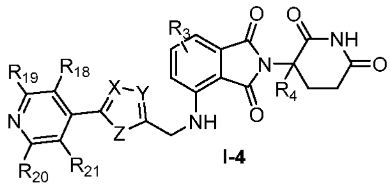

I-4

Where

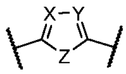

is selected from:

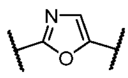,
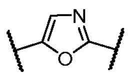,
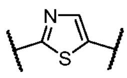,
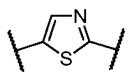,

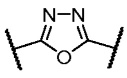,
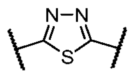;

$R_3$ is hydrogen or halogen;

$R_4$ is hydrogen, deuterium, or fluorine;

$R_5$, $R_6$, $R_7$, $R_8$, $R_9$, $R_{10}$, $R_{11}$, $R_{12}$, $R_{13}$, $R_{14}$, $R_{15}$, $R_{16}$, $R_{17}$, $R_{18}$, $R_{19}$, $R_{20}$, $R_{21}$ are each independently selected from: hydrogen, deuterium, halogen, cyano, C1-C6 alkyl, C1-C6 alkoxy, halogen-substituted C1-C6 alkyl, halogen-substituted C1-C6 alkoxy, phenyl, vinyl, C3-C8 heterocyclyl-O-, C3-C8 cycloalkyl-O-, hydroxyl-substituted $C_1$-$C_6$ alkoxy, $C_1$-$C_6$ alkoxy-substituted $C_1$-$C_6$ alkoxy;

In another preferred embodiment, $R_5$, $R_6$, $R_7$, $R_8$, and $R_9$ are all H or deuterium, or one is not hydrogen or deuterium, or two are not H or deuterium;

In another preferred embodiment, the compound has the structure shown in (I-5) or (I-6):

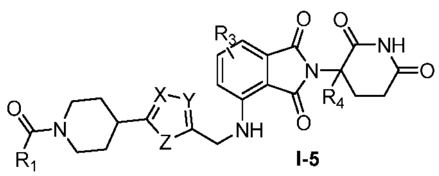

I-5

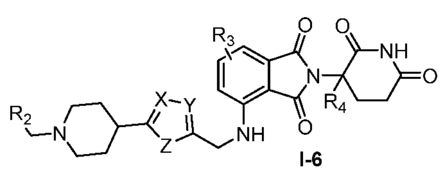

I-6

Where

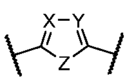

is selected from:

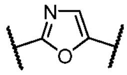, 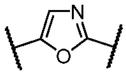, 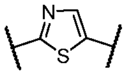, 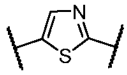,

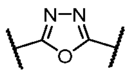, 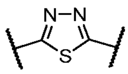;

$R_1$ is selected from: tert-butyl, cyclobutyl, cyclohexyl, phenyl,

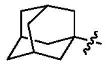 and 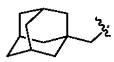,

and the cyclobutyl, cyclohexyl, and phenyl groups are optionally substituted with one or more substituents selected from the group consisting of: halogen, cyano, C1-C3 alkyl, trifluoromethyl, trifluoromethoxy, halogen-substituted or unsubstituted phenyl;

$R_2$ is phenyl, and the phenyl group is optionally substituted with one or more substituents selected from the group consisting of: fluoro, cyano, trifluoromethoxy;

$R_3$ is hydrogen or halogen;

$R_4$ is hydrogen, deuterium, or fluorine.

In another preferred embodiment, in formula (I-5), $R_1$ is selected from: tert-butyl, cyclobutyl, cyclohexyl, phenyl,

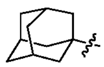, and 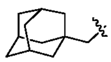,

and the cyclobutyl and phenyl groups are optionally substituted with one or more substituents selected from the group consisting of: halogen, cyano, trifluoromethyl, trifluoromethoxy, halogen-substituted or unsubstituted phenyl.

In another preferred embodiment, the prodrug has the structure shown in formula III:

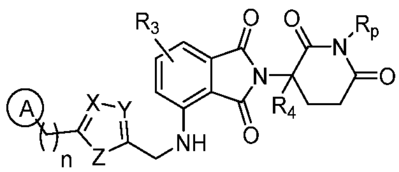

III

Wherein,

$R_p$ is $CH_2OCOC(CH_3)_3$;

$R_3$ is hydrogen or halogen;

$R_4$ is hydrogen, deuterium, or fluorine;

The definitions of X, Y, Z, A, and n are as described above.

In another preferred embodiment,

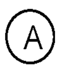

is selected from:

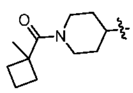, 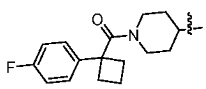,

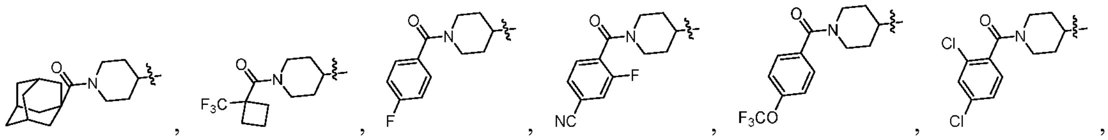
, , , , , ,

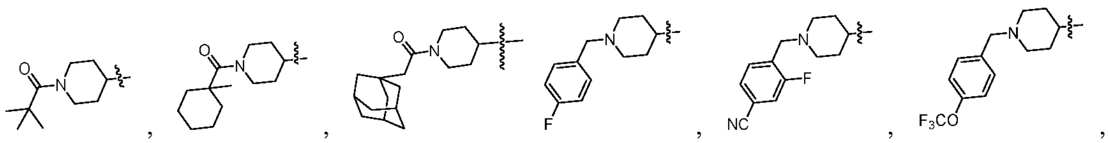
, , , , , ,

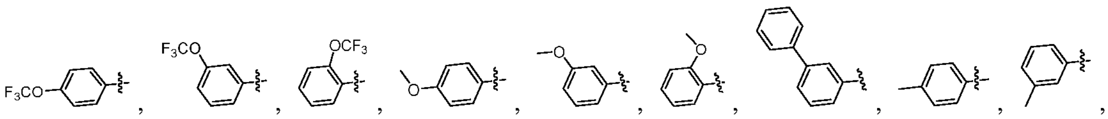
, , , , , , , , ,

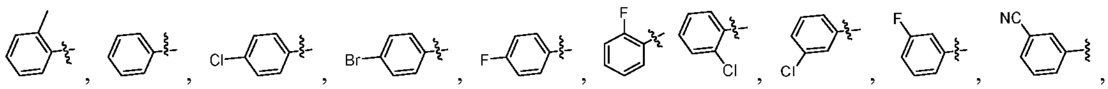
, , , , , , , , , ,

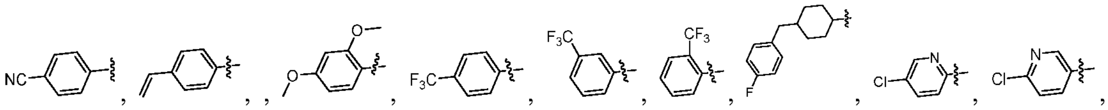
, , , , , , , , , ,

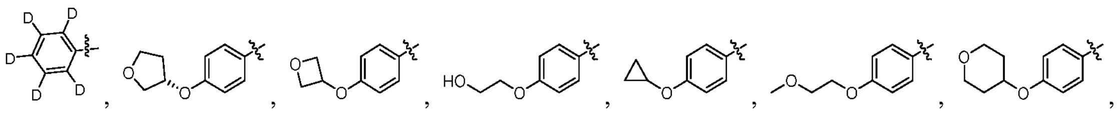
, , , , , , ,

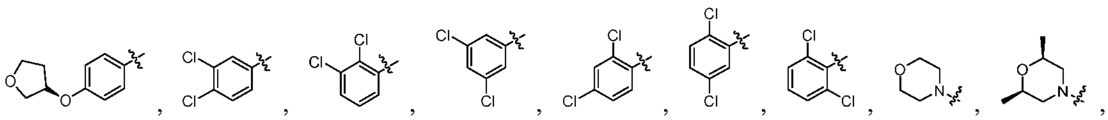
, , , , , , , , ,

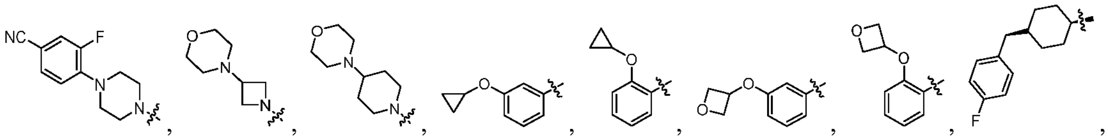
, , , , , , , ,

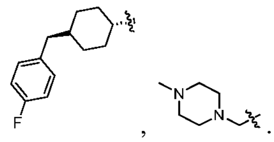
, .

In another preferred embodiment, the compound is selected from the following compounds:

| Comp. No. | Compound structure | Comp. No. | Compound structure |
|---|---|---|---|
| 1 | 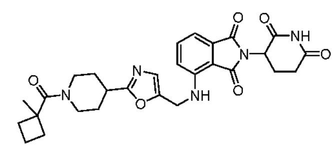 | 2 | 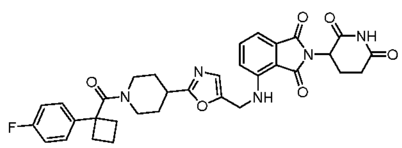 |

(continued)

| Comp. No. | Compound structure | Comp. No. | Compound structure |
|---|---|---|---|
| **3** | 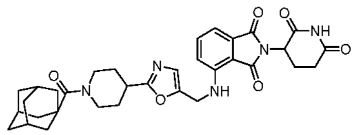 | **4** | 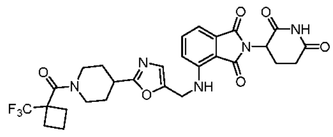 |
| **5** | 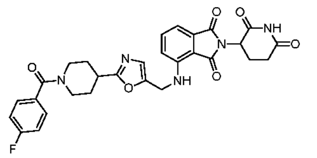 | **6** | 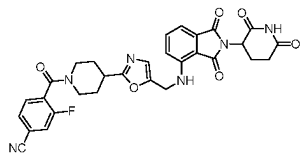 |
| **7** | 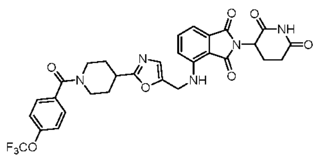 | **8** | 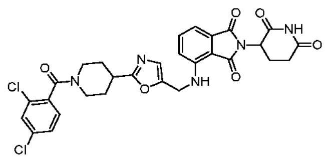 |
| **9** | 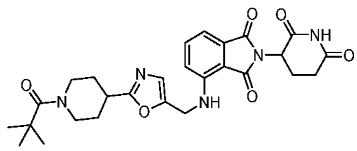 | **10** | 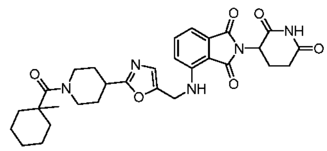 |
| **11** | 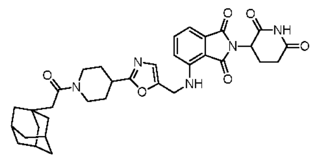 | **12** | 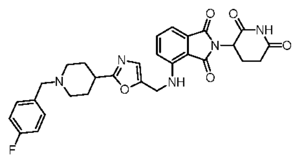 |
| **13** | 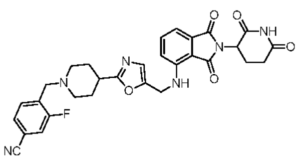 | **14** | 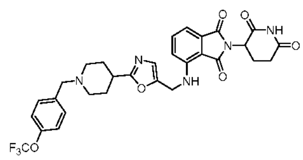 |
| **15** | 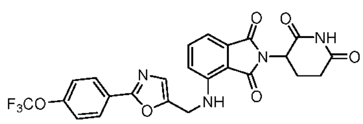 | **16** | 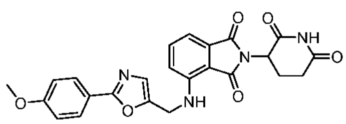 |
| **17** | 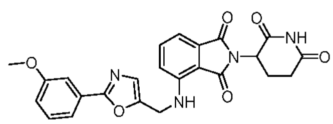 | **18** | 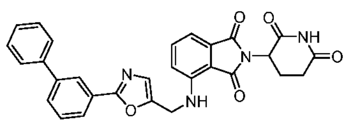 |
| **19** | 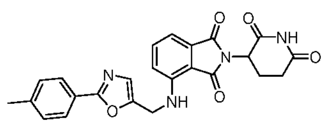 | **20** | 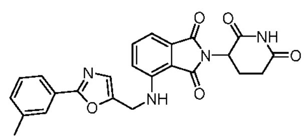 |
| **21** | 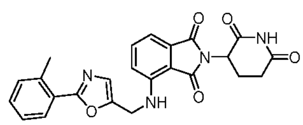 | **22** | 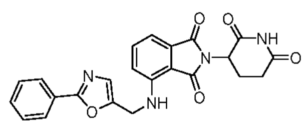 |

(continued)

| Comp. No. | Compound structure | Comp. No. | Compound structure |
|---|---|---|---|
| **23** | 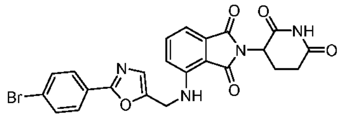 | **24** | 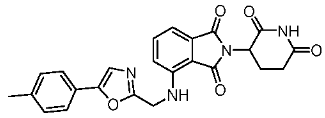 |
| **25** | 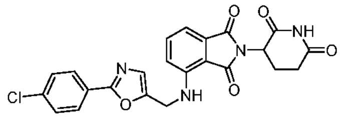 | **26** | 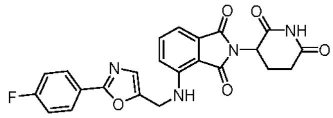 |
| **27** | 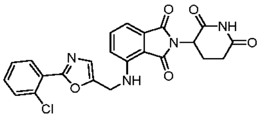 | **28** | 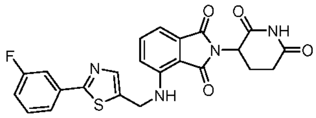 |
| **29** | 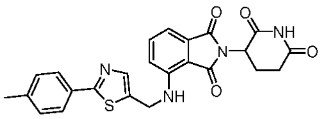 | **30** | 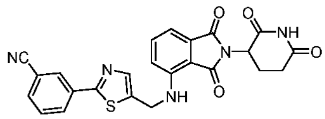 |
| **31** | 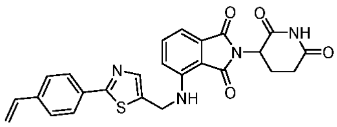 | **32** | 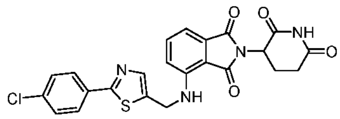 |
| **33** | 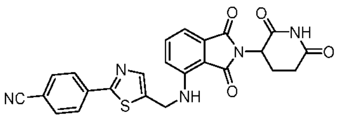 | **34** | 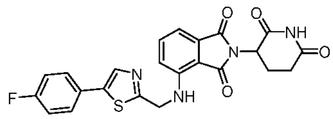 |
| **35** | 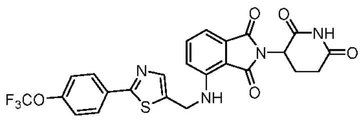 | **36** | 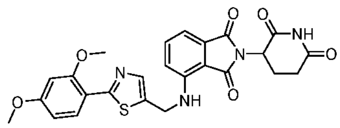 |
| **37** | 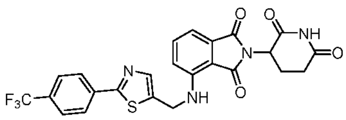 | **38** | 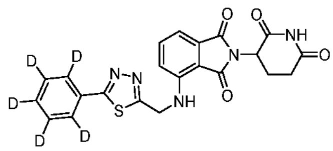 |
| **39** | 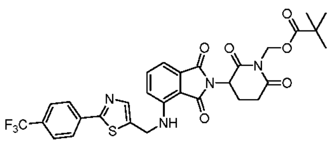 | **40** | 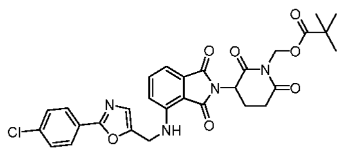 |
| **41** | 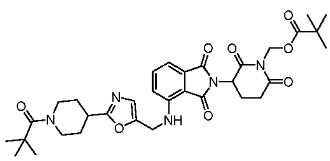 | **42** | 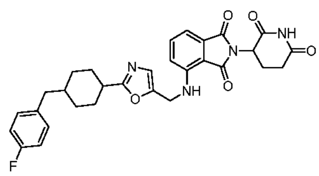 |
| **43** | 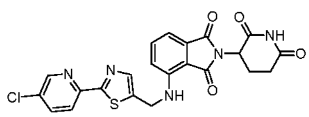 | **44** | 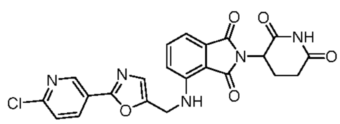 |

(continued)

| Comp. No. | Compound structure | Comp. No. | Compound structure |
|---|---|---|---|
| 45 | 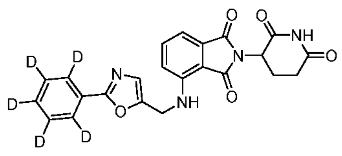 | 46 | 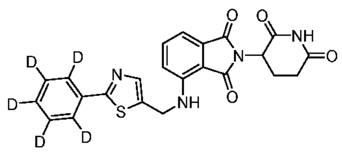 |
| 47 | 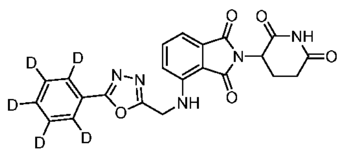 | 48 | 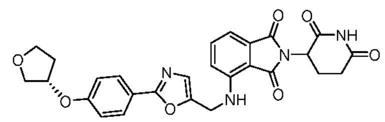 |
| 49 | 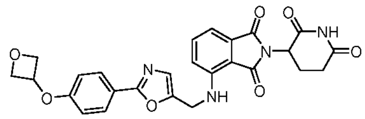 | 50 | 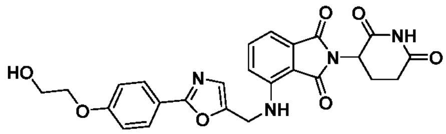 |
| 51 | 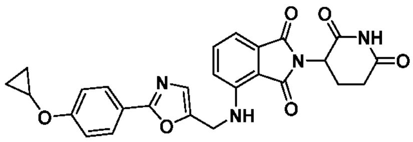 | 52 | 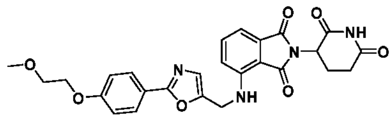 |
| 53 | 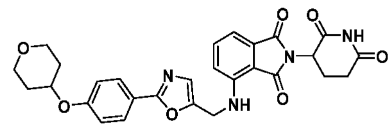 | 54 | 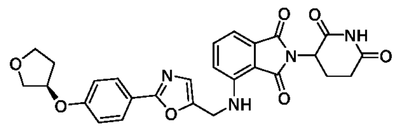 |
| 55 | 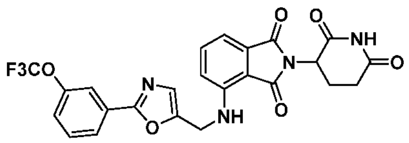 | 56 | 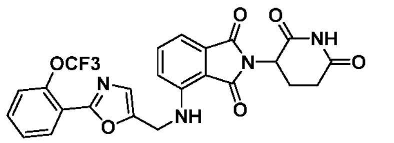 |
| 57 | 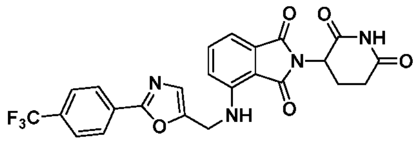 | 58 | 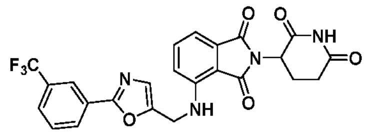 |
| 59 | 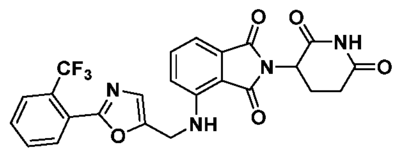 | 60 | 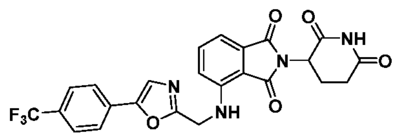 |
| 61 | 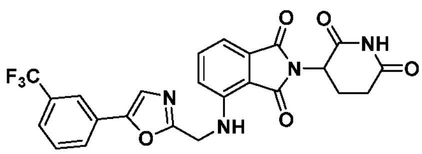 | 62 | 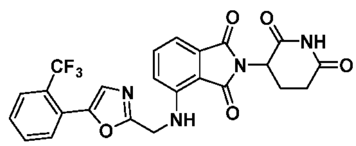 |
| 63 | 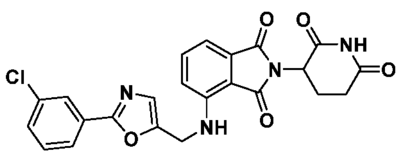 | 64 | 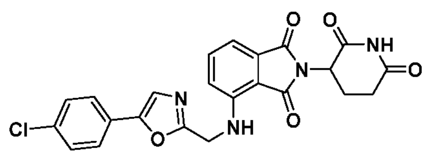 |

(continued)

| Comp. No. | Compound structure | Comp. No. | Compound structure |
|---|---|---|---|
| 65 | 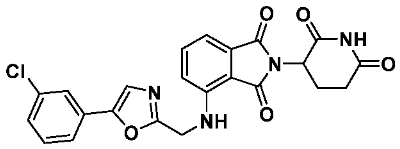 | 66 | 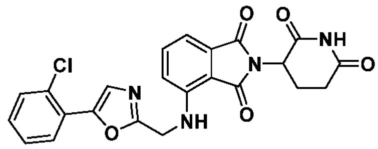 |
| 67 | 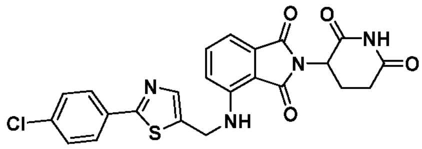 | 68 | 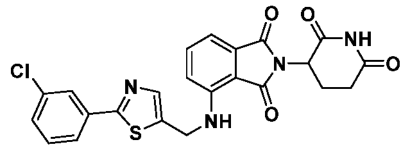 |
| 69 | 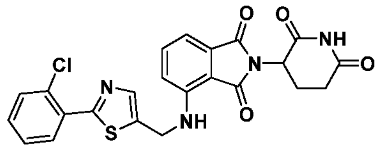 | 70 | 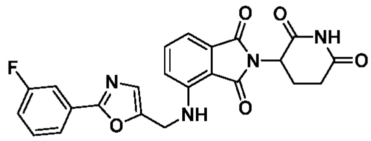 |
| 71 | 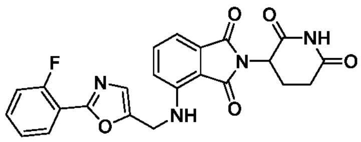 | 72 | 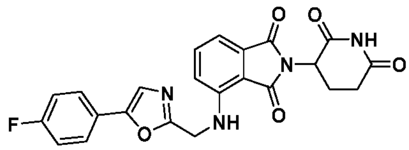 |
| 73 | 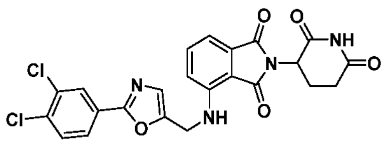 | 74 | 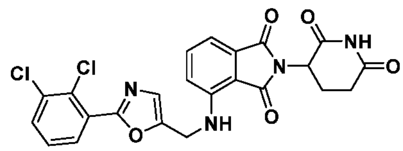 |
| 75 | 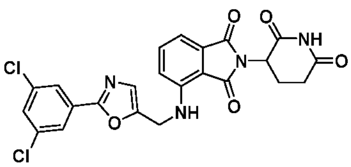 | 76 | 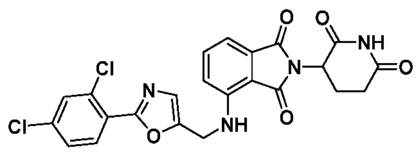 |
| 77 | 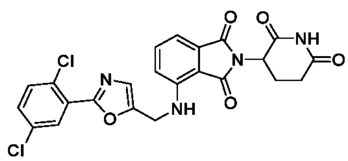 | 78 | 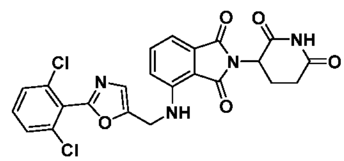 |
| 79 | 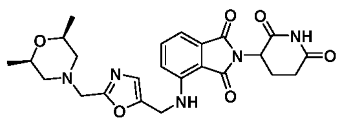 | 80 | 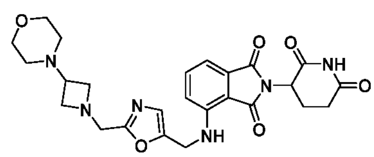 |
| 81 | 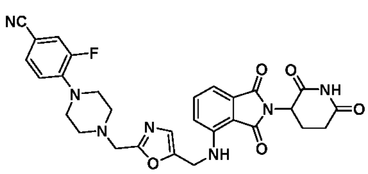 | 82 | 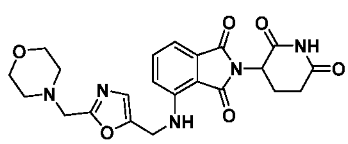 |
| 83 | 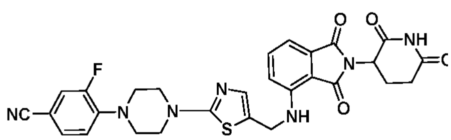 | 84 | 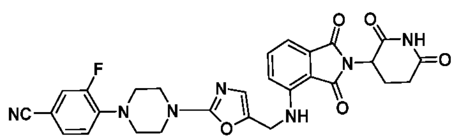 |

(continued)

| Comp. No. | Compound structure | Comp. No. | Compound structure |
|---|---|---|---|
| 85 | 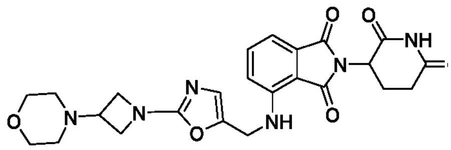 | 86 | 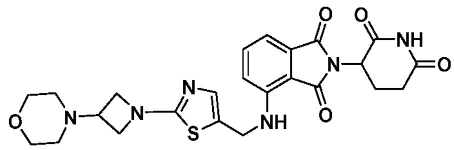 |
| 87 | 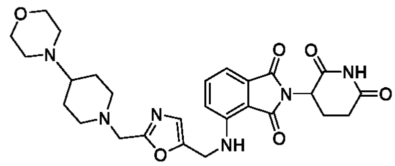 | 88 | 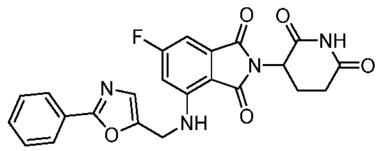 |
| 89 | 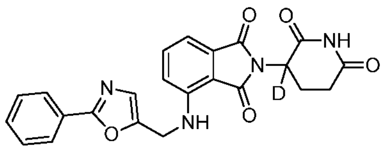 | 90 | 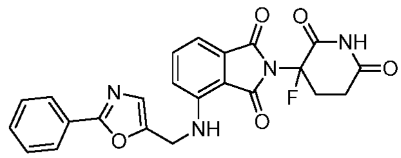 |
| 91 | 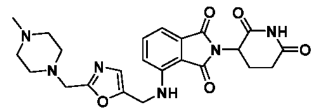 | 92 | 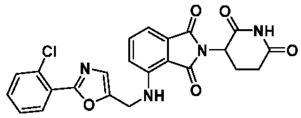 |
| 93 | 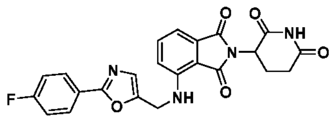 | 94 | 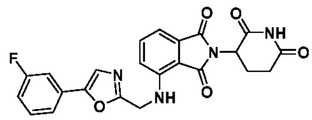 |
| 95 | 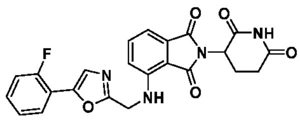 | 96 | 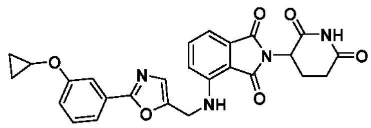 |
| 97 | 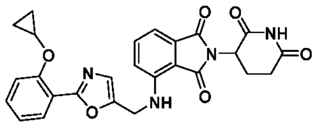 | 98 | 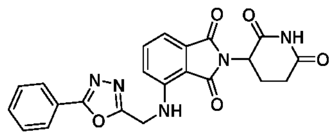 |
| 99 | 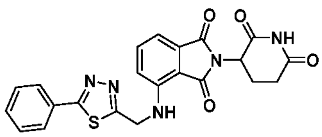 | 100 | 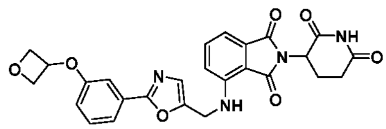 |
| 101 | 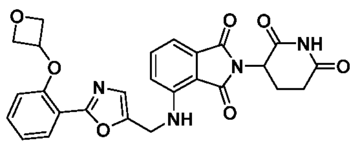 | 102 | 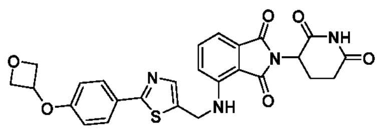 |
| 103 | 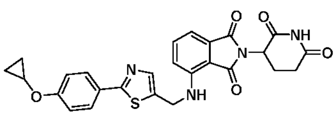 | 104 | 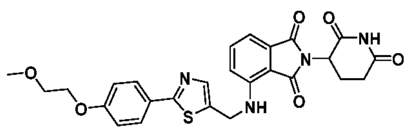 |

(continued)

| Comp. No. | Compound structure | Comp. No. | Compound structure |
|---|---|---|---|
| **105** | 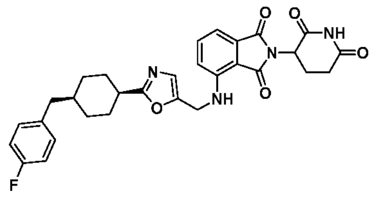 | **106** | 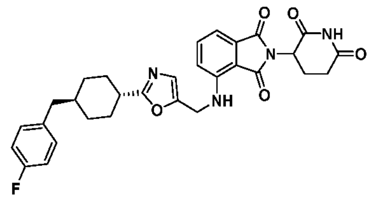 |

The third aspect of the present invention provides a method for preparing the compound of the first aspect, wherein the method is selected from one of the following methods:

Synthesis Method 1:

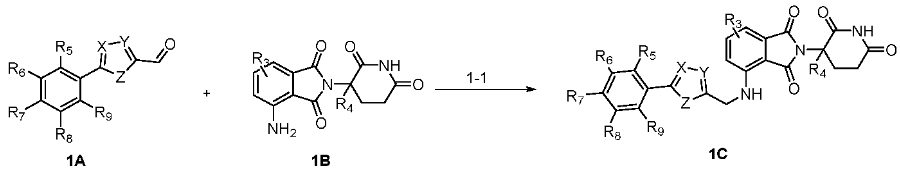


X and Y are each selected from CH or N, and X and Y cannot both be CH simultaneously;
Z is selected from O, S, or NH;
The definitions of $R_3$, $R_4$, $R_5$, $R_6$, $R_7$, $R_8$, and $R_9$ are as described above;
Step 1-1: Compound 1A and compound 1B react in the presence of phenylsilane and dibutyltin dichloride to yield compound 1C; or compound 1A and compound 1B react in the presence of acetic acid and sodium borohydride to yield compound 1C;

Synthesis Method 2:

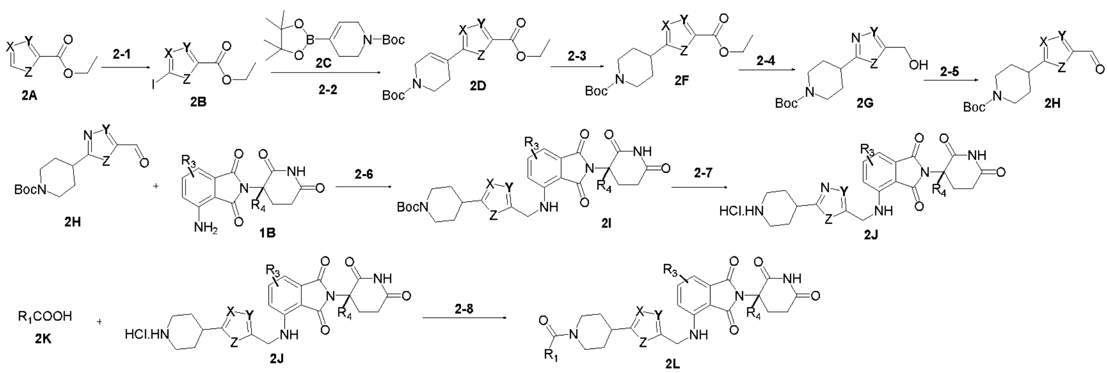


X is N, Y is CH;
Z is O;
The definitions of $R_1$, $R_3$, and $R_4$ are as described above;
Step 2-1: Compound 2A reacts with 1,2-diiodoethane to yield compound 2B;
Step 2-2: Compound 2B undergoes a coupling reaction with compound 2C to yield compound 2D;
Step 2-3: Compound 2D is hydrogenated to yield compound 2F;
Step 2-4: Compound 2F is reduced with sodium borohydride to yield compound 2G;
Step 2-5: Compound 2G reacts with an oxidizing agent to yield compound 2H;
Step 2-6: Compound 2H reacts with compound 1B in the presence of phenylsilane and dibutyltin dichloride to yield

compound 2I;
Step 2-7: Compound 2I reacts in the presence of hydrochloric acid to yield compound 2J;
Step 2-8: Compound 2K and compound 2J react in the presence of HATU and N,N-diisopropylethylamine to yield compound 2L;

Synthesis Method 3:

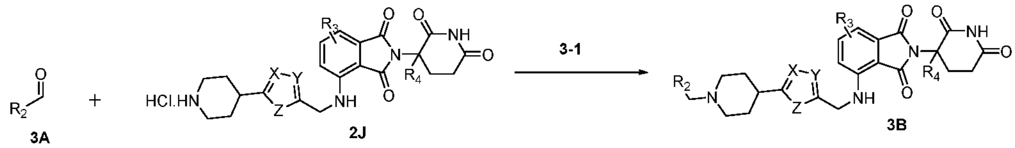

3A + 2J → 3B

X is N, Y is CH;
Z is O;
The definitions of $R_2$, $R_3$, and $R_4$ are as described above;
Step 3-1: Compound 3A reacts with compound 2J in the presence of zinc chloride, triethylamine, and sodium cyanoborohydride to yield compound 3B;

Synthesis Method 4:

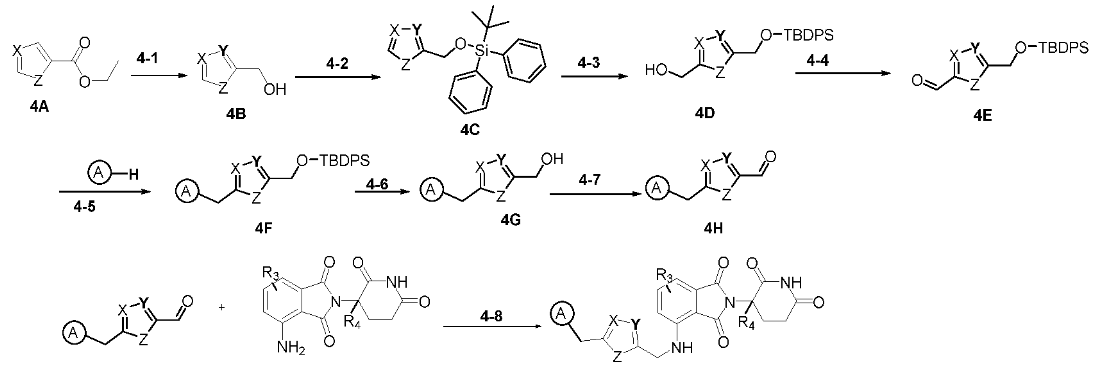

4A 4B 4C 4D 4E
4F 4G 4H
4H 1B 4I

X is N, Y is CH;
Z is O;
The definition of

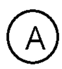

is as described above, preferably,

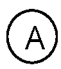

is a nitrogen-containing 4-membered or 6-membered heterocyclyl;
Step 4-1: Compound 4A reacts with lithium aluminum hydride to yield compound 4B;
Step 4-2: Compound 4B reacts with tert-butyl diphenylchlorosilane to yield compound 4C;
Step 4-3: In the presence of a borane-tetrahydrofuran complex and n-butyl lithium, compound 4C reacts with 4-formylmorpholine to yield compound 4D;
Step 4-4: Compound 4D is oxidized using Dess-Martin reagent to yield compound 4E;

Step 4-5: Compound 4E reacts with

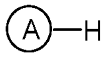

in the presence of sodium borohydride in acetic acid through reductive amination to yield compound 4F;
Step 4-6: In the presence of tetrabutylammonium fluoride in tetrahydrofuran solution, compound 4F undergoes deprotection to yield compound 4G;
Step 4-7: Compound 4G is oxidized using Dess-Martin reagent to yield compound 4H;
Step 4-8: In the presence of acetic acid and sodium borohydride, compound 4H reacts with compound 1B to yield compound 4I;

Synthesis Method 5:

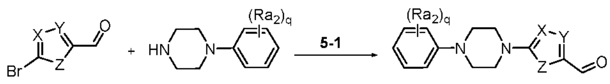


5A 5B 5C

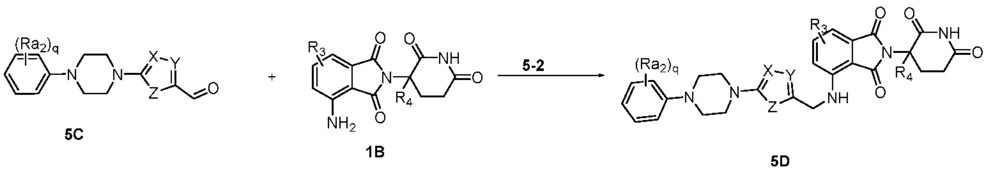


5C 1B 5D

X and Y are each selected from CH or N, and X and Y cannot both be CH;
Z is selected from O, S, or NH;
The definitions of $R_3$, $R_4$, $Ra_2$, and q are as described above;
Step 5-1: In the presence of a palladium catalyst, compound 5A undergoes a Buchwald-Hartwig amination reaction with compound 5B to yield compound 5C; Step 5-2: In the presence of acetic acid and sodium borohydride, compound 5C undergoes reductive amination with compound 1B to yield compound 5D.

The third aspect of the present invention provides a pharmaceutical composition, wherein the pharmaceutical composition comprises a therapeutically effective amount of the compound described in the first aspect, or its pharmaceutically acceptable salts, tautomers, stereoisomers, prodrugs, pharmaceutically acceptable salts of prodrugs, and pharmaceutically acceptable carriers.

In another preferred embodiment, the pharmaceutical composition further comprises one or more additional pharmaceutically active ingredients that exhibit synergistic effects in the prevention or treatment of specific diseases or disorders, or that alleviate or eliminate the toxic side effects caused by one or more other pharmaceutically active ingredients in the prevention or treatment of specific diseases or disorders.

In another preferred embodiment, the pharmaceutical composition further comprises one or more compounds selected from dexamethasone, rituximab, trastuzumab, PD-1 inhibitors, PDL-1 inhibitors, pemetrexed, topotecan, doxorubicin, bortezomib, gemcitabine, dacarbazine, clarithromycin, vincristine, cytarabine, prednisone, docetaxel, chlorambucil injection, HDAC inhibitors, kinase-targeted inhibitors, androgen receptor inhibitors, androgen biosynthesis inhibitors, erythropoiesis-stimulating agents, minocycline, Elotuzumab, Palbociclib, Nivolumab, Pembrolizumab, Panobinostat, Ublituximab, Romidepsin, Eltrombopag, CAR-T, and melphalan.

The fourth aspect of the present invention provides the use of a compound as described in the first aspect, its pharmaceutically acceptable salts, tautomeric forms, stereoisomers, prodrugs, or pharmaceutically acceptable salts in the preparation of a medicament for the prevention or treatment of diseases associated with the CRL4CRBN E3 ubiquitin ligase. Preferably, the diseases associated with the CRL4CRBN E3 ubiquitin ligase are cancer, pain, central nervous system diseases, or immune system diseases.

In another preferred embodiment, the diseases are solid tumors or hematological malignancies. Preferably, the diseases are selected from: myelodysplastic syndrome, multiple myeloma, mantle cell lymphoma, non-Hodgkin lymphoma, chronic lymphocytic leukemia, chronic myelomonocytic leukemia, myelofibrosis, Burkitt lymphoma, Hodgkin lymphoma, diffuse large B-cell lymphoma, follicular lymphoma, ciliary body and chronic melanoma, iris melanoma, recurrent bilateral ocular melanoma, T-cell lymphoma, erythroid lymphoma, monocytic and mononuclear cell leukemia, myeloid leukemia, central nervous system lymphoma, meningioma, spinal cord tumors, non-small cell lung cancer,

ovarian cancer, skin cancer, renal cell carcinoma, astrocytoma, amyloidosis, type I complex regional pain syndrome, malignant melanoma, radiculopathy, glioblastoma, gliosarcoma, malignant glioma, refractory plasma cell myeloma, extraocular extending melanoma, papillary and follicular thyroid carcinoma, breast cancer, prostate cancer, hepatocellular carcinoma, or primary macroglobulinemia.

The fifth aspect of the present invention provides a method for preventing or treating diseases associated with the $CRL4^{CRBN}$ E3 ubiquitin ligase, comprising the step of administering to a subject in need thereof a compound as described in the first aspect of the present invention, its pharmaceutically acceptable salts, tautomers, stereoisomers, or prodrugs, or a pharmaceutical composition as described in the third aspect of the present invention, thereby preventing or treating the disease.

In another preferred embodiment, the subject is a human or a non-human mammal, such as a rat or mouse.

In another preferred embodiment, the disease is a solid tumor, hematological malignancy, or the like.

It should be understood that within the scope of the present invention, the technical features described above and the technical features described in detail below (e.g., in the embodiments) can be combined with each other to form new or preferred technical solutions. Due to space limitations, they will not be listed here in detail.

## DESCRIPTION OF FIGURES

Figure 1 illustrates a structural diagram and stereoconformation diagram showing that the compounds of the present invention can form intramolecular hydrogen bonds and form a specific dihedral angle with the five-membered heteroaromatic ring through a methylene group.

## DETAILED DESCRIPTION OF THE INVENTION

Through extensive and in-depth research, the inventors have unexpectedly developed a structurally novel $CRL4^{CRBN}$ E3 ubiquitin ligase molecular glue degrader. Due to the formation of intramolecular hydrogen bonds, this degrader demonstrates superior activity in cells such as multiple myeloma MM.1S cells, mantle cell lymphoma Mino cells, and acute myeloid leukemia MV-4-11 cell lines compared to marketed drugs, clinical-stage drugs, and thalidomide-related compounds in the prior art that have comparable structures but do not form intramolecular hydrogen bonds or specific dihedral angles. Based on this, the inventors have completed the present invention.

## TERM

When substituents are described using conventional chemical formulas written from left to right, the substituents also include those that are chemically equivalent when the structure is written from right to left. For example, $-CH_2O-$ is equivalent to $-OCH_2-$.

One skilled in the art will understand that the combinations of substituents expected in the present invention are those that are stable or chemically feasible. Substituents on the relevant structures of the invention, including substituted and unsubstituted groups, such as "optionally" or "selectively" substituted by a certain substituent, refer to combinations that include both substituted and unsubstituted forms.

When multiple substituents are mentioned in the invention, R substituents may be the same or different, meaning that when a structure has multiple substituents, the combination of R substituents can be selected from various different types of substituents.

The term "substituted" only applies to sites where substitution by a substituent is possible, excluding those where substitution cannot occur according to existing chemical knowledge. "Substitution" refers to one or more hydrogen atoms on a specific group being replaced by a particular substituent. The specific substituents are those described earlier or those appearing in the examples. Unless otherwise specified, any substituted group may have a substituent selected from a particular group at any of its substitutable positions, and these substituents may be the same or different at each position. Cyclic substituents, such as heterocyclic alkyls, may be linked to another ring, such as a cycloalkyl group, forming a bicyclic system where two rings share a common carbon atom.

The term "halogen" refers to fluorine, chlorine, bromine, or iodine.

The term "$C_1$-$C_6$ alkyl" refers to a straight-chain or branched saturated hydrocarbon group containing 1 to 6 carbon atoms. Non-limiting examples include methyl, ethyl, propyl, isopropyl, butyl, isobutyl, sec-butyl, and tert-butyl.

The term "$C_1$-$C_6$ alkoxy" refers to a straight-chain or branched alkoxy group containing 1 to 6 carbon atoms. Non-limiting examples include methoxy, ethoxy, propoxy, isopropoxy, and butoxy.

The term "aryl" refers to a 6-14 membered fully carbon-based monocyclic or fused polycyclic group with a conjugated $\pi$-electron system. Preferably, the aryl group contains 6 to 10 members (i.e., C6-C10 aryl), more preferably phenyl and naphthyl, and most preferably phenyl. The aryl ring can be fused to a heteroaryl, heterocyclic, or cycloalkyl ring, where the ring connected to the parent structure is the aryl ring.

The term "halogen-substituted $C_1$-$C_6$ alkoxy" refers to a straight-chain or branched $C_1$-$C_6$ alkoxy group substituted with one or more halogens. Non-limiting examples include $-OCH_2F$, - $OCHF_2$, -OCF3.

The term "halogen-substituted $C_1$-$C_6$ alkyl" refers to a straight-chain or branched $C_1$-$C_6$ alkyl group substituted with one or more halogens. Non-limiting examples include 2-bromoethyl, 2-bromopropyl, etc.

The term "cycloalkyl" refers to a saturated or partially unsaturated monocyclic or polycyclic cyclic hydrocarbon substituent. Cycloalkyl includes C4-C12 cycloalkyl, preferably C4-C10 cycloalkyl, C4-C6 cycloalkyl, or C10-C12 cycloalkyl. Monocyclic cycloalkyls non-limiting examples include cyclopropyl, cyclobutyl, cyclopentenyl, cyclohexyl, while polycyclic cycloalkyls include spiro, fused, and bridged cycloalkyls, such as

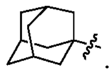

The term "4-10 membered heterocyclyl" refers to a ring containing one or more saturated and/or partially saturated rings, including 4 to 10 ring atoms, where one or more (e.g., 2 or 3) ring atoms are selected from nitrogen, oxygen, sulfur, or S(O)m (where m is an integer from 0 to 2) heteroatoms, with the remaining ring atoms being carbon. The 4-10 membered heterocyclic group is preferably a 4-6 membered heterocyclic group, such as epoxide, tetrahydrofuran, pyrrolidinyl, tetrahydropyran, piperidyl, piperazinyl, morphelinyl, thiazolyl.

The term "C3-C8 heterocyclic group" refers to a saturated and/or partially saturated ring containing 3 to 8 ring carbon atoms, which may also include one or more (e.g., 2 or 3) heteroatoms selected from nitrogen, oxygen, sulfur, or S(O)m (where m is an integer from 0 to 2). The C3-C8 heterocyclic group includes heterocyclic groups containing 3 to 5 ring carbon atoms and may contain one or more (e.g., 2 or 3) heteroatoms selected from nitrogen, oxygen, or sulfur, such as oxiranyl, tetrahydrofuran, pyrrolidinyl, tetrahydropyran, piperidyl, piperazinyl, morphelinyl, thiazolyl.

The term "C2-C6 alkenyl" refers to a hydrocarbon chain group composed only of carbon and hydrogen atoms, containing at least one double bond, having 2 to 6 carbon atoms, and being connected to the rest of the molecule by one or more single bonds, either as a straight or branched chain. Examples include, but are not limited to, vinyl, propyl, butenyl, styrenyl, and phenylethynyl.

The term "heteroaryl" refers to a 5-14 membered aromatic ring system containing 1 to 4 heteroatoms as ring atoms, with the remaining ring atoms being carbon. The heteroatoms include oxygen, sulfur, and nitrogen. It is preferably a 5-10 membered ring, more preferably 5 or 6 members, such as thiophene, furan, pyridine, pyrrole, N-alkylpyrrole, pyrimidine, pyrazine, imidazole, tetrazole, etc.

"Hydrate" refers to a compound that contains water.

"Pharmaceutically acceptable salts" refers to salts formed by the reaction of a drug molecule with a corresponding organic or inorganic acid, or organic or inorganic base. Examples include hydrochloride, formate, trifluoroacetate, succinate, methanesulfonate, etc.

In the present invention, unless specifically stated otherwise, the terms used have the generally understood meanings as recognized by those skilled in the art.

## ACTIVE INGREDIENT

As used herein, the term "compound of the present invention" or "active ingredient" refers to the compound shown in Formula I, and also includes its pharmaceutically acceptable salts, tautomers, stereoisomers, and prodrugs.

The compound shown in Formula I can exist in different tautomeric forms, all of which are included within the scope of the present invention.

The term "tautomer" refers to structural isomers that interconvert via low energy barrier transitions, typically involving the movement of hydrogen atoms or protons accompanied by the transformation of single bonds and adjacent double bonds.

The compounds of the present invention may exist in specific geometric or stereoisomeric forms. All such compounds contemplated by the present invention, including cis and trans isomers, (-)- and (+)-enantiomers, (R)- and (S)-enantiomers, non-corresponding isomers, racemates, and other mixtures, are within the scope of this invention. Alkyl or other substituents may also contain additional asymmetric carbon atoms. All of these isomers and their mixtures are included within the scope of the present invention.

The compound shown in Formula (I) may contain one or more asymmetric or chiral centers and, therefore, may exist in different stereoisomeric forms. The compounds of the present invention include all stereoisomeric forms, including but not limited to diastereomers, enantiomers, atropisomers, and mixtures thereof (such as racemates), all of which are included within the scope of the present invention.

Unless otherwise specified, the term "enantiomer" refers to stereoisomers that are mirror images of each other.

Unless otherwise specified, the term "diastereomer" refers to stereoisomers that have two or more chiral centers

and are not mirror images of each other.

"Racemate" refers to a mixture of two stereoisomers that are mirror images of each other, with opposite optical activities, resulting in the cancellation of the optical activity.

The compounds of the present invention, including the general formula and those in the examples, contain chiral centers. Therefore, the single configuration or racemate of the compounds in the present invention is also within the scope of this application. Moreover, prior art provides evidence that compounds with thalidomide-like structures, including their R-configurations, S-configurations, and racemates, will convert to a 1:1 racemate in vitro and in vivo cell experiments. Therefore, one skilled in the art can expect that the enantiomers or racemates of the compounds in the present invention will achieve equivalent technical effects.

In the present invention, if a single configuration of an example compound is needed, it can be obtained from the single configuration intermediate 1B through the synthesis method 1 in the compound preparation method, thus obtaining the enantiomer. Additionally, the non-diastereomeric example compounds can also be obtained by preparing them from the single configuration intermediate 1B, so non-diastereomers, such as compound 48 and compound 54, can form non-diastereomeric isomers. All these different isomeric forms are considered stereoisomers.

The compounds of the present invention may contain non-natural isotopic ratios of atoms at one or more atoms constituting the compound. For example, the compounds may be radiolabeled with isotopes such as deuterium (D), tritium (T), carbon-12 ($^{12}C$), carbon-13 ($^{13}C$), and carbon-14 ($^{14}C$). For instance, hydrogen may be replaced by deuterium to form deuterated drugs. The bond between deuterium and carbon is stronger than that between ordinary hydrogen and carbon, and compared to undeuterated drugs, deuterated drugs offer advantages such as reduced toxicity, increased drug stability, enhanced efficacy, and prolonged biological half-life. All isotopic variations of the compounds of the present invention, whether radioactive or not, are included within the scope of the present invention.

As used herein, and unless otherwise specified, the term "prodrug" refers to a derivative of a compound that contains a bioactive functional group, such that under biological conditions (in vitro or in vivo), the bioactive functional group may cleave from the compound or undergo other reactions to provide the active compound. Typically, the prodrug is inactive or less active than the

compound itself, such that the activity is only exerted after the compound is cleaved from the bioactive functional group under biological conditions. The bioactive functional group can undergo hydrolysis or oxidation under biological conditions to provide the compound. For example, the prodrug may contain a group that can be biologically hydrolyzed. Examples of biologically hydrolyzable groups include, but are not limited to, biologically hydrolyzable phosphates, biologically hydrolyzable esters, biologically hydrolyzable amides, biologically hydrolyzable carbonates, biologically hydrolyzable carbamates, and biologically hydrolyzable ureas. Reviews on prodrugs can be found, for example, in J. Rautio et al., Nature Reviews Drug Discovery 2008, 7, 255-270, and Prodrugs: Challenges and Rewards (V. Stella et al., eds., Springer, 2007).

The compounds of the present invention containing basic groups can be formulated into pharmaceutically acceptable salts, including inorganic acid salts and organic acid salts. Suitable acids for forming salts include, but are not limited to, inorganic acids such as hydrochloric acid, hydrobromic acid, hydrofluoric acid, sulfuric acid, nitric acid, and phosphoric acid; organic acids such as formic acid, acetic acid, propionic acid, oxalic acid, malonic acid, succinic acid, fumaric acid, maleic acid, lactic acid, malic acid, tartaric acid, citric acid, bitter acid, methanesulfonic acid, benzenesulfonic acid, and benzenesulfonic acid; as well as acidic amino acids such as aspartic acid and glutamic acid.

The present invention also includes any new intermediates disclosed herein.

## PHARMACEUTICAL COMPOSITION AND ADMINISTRATION METHOD

In this application, the term "pharmaceutical composition" refers to a formulation of the compounds of the present invention with a medium typically used in the art for delivering bioactive compounds to mammals (such as humans). This medium includes a pharmaceutically acceptable carrier. The purpose of the pharmaceutical composition is to facilitate administration to the organism and promote the absorption of the active ingredient to exert its biological activity.

The compound of formula (I) may be co-administered with other known drugs that treat or improve similar conditions. When co-administered, the dosing regimen and dosage of the original drug can remain unchanged while the compound of formula (I) is administered concurrently or subsequently. When the compound of formula (I) is taken together with one or more other drugs, it is preferred to use a pharmaceutical composition containing one or more known drugs and the compound of formula (I). Drug combinations also include those where the compound of formula (I) and one or more known drugs are taken during overlapping time periods. When the compound of formula (I) is co-administered with one or more other drugs, the dosage of the compound of formula (I) or the known drug may be lower than when they are used alone.

The term "pharmaceutically acceptable" as used herein refers to substances (such as carriers or diluents) that do not affect the biological activity or properties of the compounds of the present invention, and are relatively non-toxic, meaning that such substances can be administered to an individual without causing adverse biological reactions or

interacting with any component in the composition in an adverse manner.

In this application, the term "pharmaceutically acceptable carrier" includes, but is not limited to, any adjuvant, carrier, excipient, flow agent, sweetener, diluent, preservative, dye/colorant, flavoring agent, surfactant, wetting agent, dispersing agent, suspending agent, stabilizer, isotonic agent, solvent, or emulsifier that is approved by relevant governmental regulatory authorities for use in humans or animals.

Drugs or active ingredients that may be co-administered with the compound of formula (I) include, but are not limited to, dexamethasone, rituximab, trastuzumab, PD-1 inhibitors, PDL-1 inhibitors, pemetrexed, topotecan, doxorubicin, bortezomib, gemcitabine, dacarbazine, clarithromycin, vincristine, cytarabine, prednisone, docetaxel, chlorambucil injection, HDAC inhibitors, kinase-targeted inhibitors, androgen receptor inhibitors, androgen biosynthesis inhibitors, erythropoietin, minocycline, Elotuzumab, Palbociclib, Nivolumab, Pembrolizumab, Panobinostat, Ublituximab, Romidepsin, Eltrombopag, CAR-T, and methotrexate, either alone or in combination.

A typical formulation is prepared by mixing the compound represented by formula (I) of the present invention with a carrier, diluent, or excipient. Suitable carriers, diluents, or excipients are well-known to those skilled in the art and include substances such as carbohydrates, waxes, watersoluble and/or expandable polymers, hydrophilic or hydrophobic substances, gelatin, oils, solvents, water, and the like. The specific carrier, diluent, or excipient used will depend on the mode of administration and the intended purpose of the compound of the present invention. Solvents are typically selected based on those considered safe and effective for administration to mammalian animals by those skilled in the art. Generally, safe solvents are non-toxic aqueous solvents, such as pharmaceutical-grade water, and other non-toxic solvents that are soluble in or miscible with water. Suitable aqueous solvents include water, ethanol, propylene glycol, polyethylene glycol (e.g., PEG400, PEG300), and the like, either alone or in combination. The formulation may also include one or more buffers, stabilizers, surfactants, wetting agents, lubricants, emulsifiers, suspending agents, preservatives, antioxidants, light blockers, flow agents, processing aids, colorants, sweeteners, flavoring agents, or other known additives to manufacture or use the compound represented by formula (I) in an acceptable form.

When the compound of the present invention, as represented by formula (I), is combined with at least one other drug, the two or more drugs may be administered separately or in combination, preferably in the form of a pharmaceutical composition. The compound of formula (I) or the pharmaceutical composition of the present invention can be administered to the subject by any known method, including oral, intravenous, rectal, vaginal, transdermal, other local or systemic administration, either separately or together.

These pharmaceutical compositions may also contain one or more buffers, stabilizers, surfactants, wetting agents, lubricants, emulsifiers, suspending agents, preservatives, antioxidants, light blockers, flow agents, processing aids, colorants, sweeteners, flavoring agents, or other known additives to manufacture or use the pharmaceutical composition in an acceptable form.

The pharmaceutical composition of the present invention is preferably administered orally. Solid dosage forms for oral administration may include capsules, tablets, powders, or granules. In solid dosage forms, the compound of the present invention or its pharmaceutical composition is mixed with at least one inert excipient, diluent, or carrier. Suitable excipients, diluents, or carriers include substances such as sodium citrate or dicalcium phosphate, or starch, lactose, sucrose, mannitol, silica, etc.; binders such as carboxymethyl cellulose, alginates, gelatin, polyvinylpyrrolidone, sucrose, gum arabic, etc.; wetting agents such as glycerin; disintegrants such as agar, calcium carbonate, potato or cassava starch, alginic acid, specific complex silicates, sodium carbonate, etc.; solution retardants such as paraffin; absorption enhancers such as quaternary ammonium compounds; adsorbents such as kaolin, bentonite, etc.; lubricants such as talc, calcium stearate, magnesium stearate, solid polyethylene glycol, sodium lauryl sulfate, etc. In the case of capsules and tablets, the dosage form may also include buffers. Similar types of solid compositions may also be used as fillers in soft and hard gelatin capsules, which use lactose and high molecular weight polyethylene glycol as excipients.

Liquid dosage forms for oral administration include pharmaceutically acceptable emulsions, solutions, suspensions, syrups, and tinctures. In addition to the compound of the present invention or its composition, these liquid dosage forms may contain commonly used inert diluents such as water or other solvents; solubilizers and emulsifiers such as ethanol, isopropyl alcohol, ethyl carbonate, ethyl acetate, benzyl alcohol, benzyl benzoate, propylene glycol, 1,3-butanediol, dimethylformamide; oils (e.g., cottonseed oil, peanut oil, corn germ oil, olive oil, castor oil, sesame oil, etc.); glycerin; tetrahydrofurfuryl alcohol; fatty acid esters of polyethylene glycol and dehydrated sorbitol; or mixtures of several of these substances.

In addition to these inert diluents, the composition may also include excipients such as wetting agents, emulsifiers, suspending agents, sweeteners, flavoring agents, and fragrances, in one or more of these forms.

For suspensions, in addition to the compound or composition of the present invention, the formulation may further contain carriers such as suspending agents, including ethoxylated isostearyl alcohol, polyoxyethylene sorbitan, dehydrated sorbitan esters, microcrystalline cellulose, pseudo-boehmite, bentonite, agar, and astragalus gum, or mixtures of several of these substances.

Compositions for rectal or vaginal administration are preferably suppositories, which can be prepared by mixing the compound or composition of the present invention with a suitable nonirritating excipient or carrier, such as cocoa butter,

polyethylene glycol, or suppository wax, which are solid at room temperature but liquid at body temperature, and can melt in the rectum or vagina to release the active compound.

The compounds or pharmaceutical compositions of the present invention may also be administered using other topical dosage forms, including creams, powders, sprays, and inhalants.

The pharmaceutical composition may be mixed with pharmaceutically acceptable excipients, diluents, or carriers, and any necessary preservatives, buffers, or propellants, under sterile conditions. Ophthalmic formulations, ophthalmic ointments, powders, and solutions are also intended to be included within the scope of the present invention.

The terms "administer," "apply," "deliver," and the like, as used in this application, refer to methods that deliver the compound or composition to the desired site for biological activity. These methods include, but are not limited to, oral administration, enteral administration (such as through the duodenum), parenteral injections (including intravenous, subcutaneous, intraperitoneal, intramuscular, intra-arterial injections or infusions), topical administration, and rectal administration. Skilled practitioners are familiar with the methods of administration that can be used for the compounds and methods described herein, such as those discussed in Goodman and Gilman, The Pharmacological Basis of Therapeutics, current edition; Pergamon; and Remington's, Pharmaceutical Sciences (current edition), Mack Publishing Co., Easton, Pa. **In** preferred embodiments, the compounds and compositions discussed herein are administered orally.

The terms used in this invention, such as "drug combination," "drug co-administration," "combination therapy," "administering other treatments," "administering other therapeutic agents," and the like, refer to pharmaceutical treatments achieved by mixing or combining more than one active ingredient, including both fixed and non-fixed combinations of active ingredients. The term "fixed combination" refers to administering at least one compound of the present invention and at least one synergistic agent to the patient simultaneously in the form of a single entity or dosage form. The term "non-fixed combination" refers to administering at least one compound of the present invention and at least one synergistic formulation to the patient either simultaneously or sequentially at variable intervals in separate entities. These terms also apply to combination therapies, such as administering three or more active ingredients.

The pharmaceutical compositions of the present invention contain an effective and safe amount of the compound of the present invention or its pharmaceutically acceptable salts, along with pharmaceutically acceptable excipients or carriers. The term "effective and safe amount" refers to an amount of the compound sufficient to produce a noticeable improvement in the condition being treated, without causing severe side effects. Typically, the pharmaceutical composition contains 1-2000 mg of the compound per dose, preferably 10-1000 mg per dose. More preferably, the "dose" refers to a capsule or tablet.

The compounds or pharmaceutical compositions of the present invention can be used in the preparation of medicines for the treatment or prevention of diseases involving the CRL4CRBNE3 ubiquitin ligase. Such diseases, non-limiting in nature, include tumors, central nervous system disorders, and immune diseases.

**In** one preferred embodiment, the diseases include, but are not limited to, cancer, pain (including but not limited to complex regional pain syndrome), skin diseases, immunodeficiency diseases, central nervous system damage, and functional disorders.

In another preferred embodiment, the cancers include, but are not limited to, skin cancers (e.g., melanoma), lymphatic cancers, breast cancer, cervical cancer, uterine cancer, gastrointestinal cancers, lung cancer, ovarian cancer, prostate cancer, colorectal cancer, rectal cancer, oral cancer, brain tumors, head and neck cancers, throat cancer, testicular cancer, kidney cancer, pancreatic cancer, spleen cancer, liver cancer, bladder cancer, laryngeal cancer, and cancers related to AIDS. The compounds provided by the present invention are also effective for hematologic cancers and multiple myeloma, such as in the treatment of multiple myeloma, lymphoma, and acute and chronic leukemias. The compounds provided by the present invention can also be used for the prevention or treatment of primary and metastatic tumors.

The pharmaceutical molecules of the present invention are used in the treatment of diseases selected from the following: myelodysplastic syndromes, multiple myeloma, mantle cell lymphoma, non-Hodgkin lymphoma, chronic lymphocytic leukemia, chronic monocytic leukemia, myelofibrosis, Burkitt lymphoma, Hodgkin lymphoma, diffuse large B-cell lymphoma, follicular lymphoma, ciliary and chronic melanoma, iris melanoma, recurrent bilateral melanomas, T-cell lymphoma, erythroid lymphoma, monocytic and mononuclear leukemia, myelogenous leukemia, central nervous system lymphoma, meningiomas, spinal tumors, non-small cell lung cancer, ovarian cancer, skin cancer, renal cell carcinoma, astrocytomas, amyloidosis, Type I complex regional pain syndrome, malignant melanoma, radiculopathy, glioblastomas, gliosarcomas, malignant gliomas, refractory plasmacytomas, extraocular melanomas, papillary and follicular thyroid cancers, breast cancer, prostate cancer, hepatocellular carcinoma, or primary macroglobulinemia.

**Method for Preparing the Compound**

The following schemes describe methods for preparing the compound represented by Formula I. In certain cases, the order of steps in the reaction schemes can be altered to promote the reaction or avoid the formation of unwanted by-products. The compounds of the present invention can also optionally be prepared by combining various synthetic

methods described in this specification or known in the art, which can be easily carried out by those skilled in the art to which the invention pertains.

Generally, in the preparation process, the reactions are typically carried out in inert solvents at room temperature to reflux temperatures (e.g., 0 °C to 150 °C, preferably 10 °C to 100 °C). The reaction time is typically from 0.1 hours to 60 hours, preferably 0.5 to 48 hours.

The method is preferably selected from one of the following methods: Starting compounds 1A, 2B, 2D, and 2F can be synthesized according to references CN111285850A, WO2017193063 A1.

Synthesis Method 1:

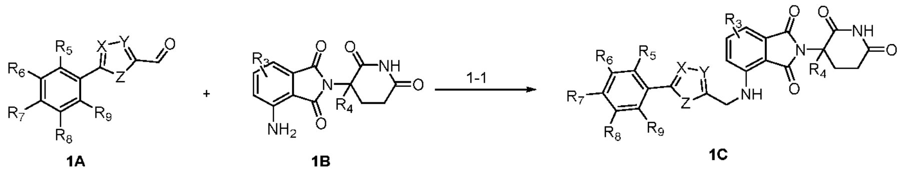


X and Y are each selected from CH or N, and X and Y cannot both be CH simultaneously;

Z is selected from O, S, or NH;

The definitions of $R_3$, $R_4$, $R_5$, $R_6$, $R_7$, $R_8$, and $R_9$ are as described above;

Step 1-1: Compound 1A and compound 1B react in the presence of phenylsilane and dibutyltin dichloride to yield compound 1C; or compound 1A and compound 1B react in the presence of acetic acid and sodium borohydride to yield compound 1C;

Synthesis Method 2:

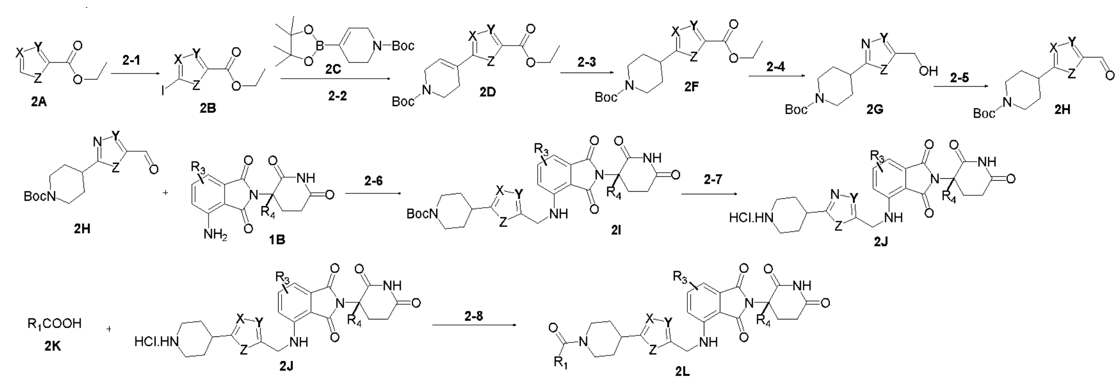


X is N, Y is CH;

Z is O;

The definitions of $R_1$, $R_3$, and $R_4$ are as described above;

Step 2-1: Compound 2A reacts with 1,2-diiodoethane to yield compound 2B;

Step 2-2: Compound 2B undergoes a coupling reaction with compound 2C to yield compound 2D;

Step 2-3: Compound 2D is hydrogenated to yield compound 2F;

Step 2-4: Compound 2F is reduced with sodium borohydride to yield compound 2G;

Step 2-5: Compound 2G reacts with an oxidizing agent to yield compound 2H;

Step 2-6: Compound 2H reacts with compound 1B in the presence of phenylsilane and dibutyltin dichloride to yield compound 2I;

Step 2-7: Compound 2I reacts in the presence of hydrochloric acid to yield compound 2J;

Step 2-8: Compound 2K and compound 2J react in the presence of HATU and N,N-diisopropylethylamine to yield compound 2L;

Synthesis Method 3:

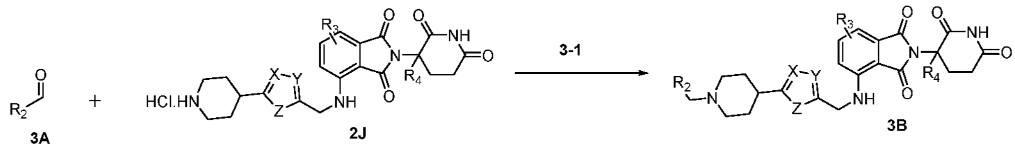

3A 2J 3B

X is N, Y is CH;
Z is O;
The definitions of $R_2$, $R_3$, and $R_4$ are as described above;
Step 3-1: Compound 3A reacts with compound 2J in the presence of zinc chloride, triethylamine, and sodium cyanoborohydride to yield compound 3B;

Synthesis Method 4:

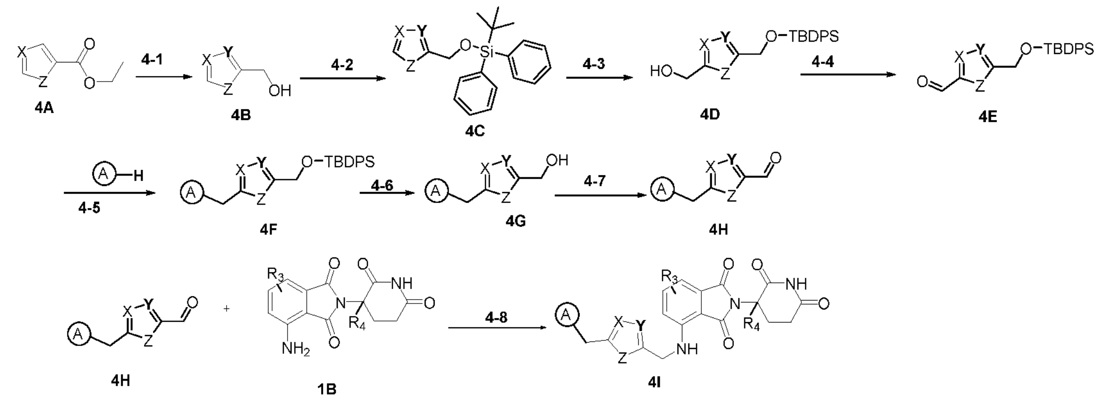

4A 4B 4C 4D 4E 4F 4G 4H 4H 1B 4I

X is N, Y is CH;
Z is O;
The definition of

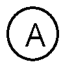

is as described above, preferably,

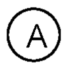

is a nitrogen-containing 4-membered or 6-membered heterocyclic group;
Step 4-1: Compound 4A reacts with lithium aluminum hydride to yield compound 4B;
Step 4-2: Compound 4B reacts with tert-butyl diphenylchlorosilane to yield compound 4C;
Step 4-3: In the presence of a borane-tetrahydrofuran complex and n-butyl lithium, compound 4C reacts with 4-formylmorpholine to yield compound 4D;
Step 4-4: Compound 4D is oxidized using Dess-Martin reagent to yield compound 4E;
Step 4-5: Compound 4E reacts with

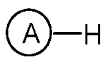

in the presence of sodium borohydride in acetic acid through reductive amination to yield compound 4F;

Step 4-6: In the presence of tetrabutylammonium fluoride in tetrahydrofuran solution, compound 4F undergoes deprotection to yield compound 4G;

Step 4-7: Compound 4G is oxidized using Dess-Martin reagent to yield compound 4H;

Step 4-8: In the presence of acetic acid and sodium borohydride, compound 4H reacts with compound 1B to yield compound 4I;

Synthesis Method 5:

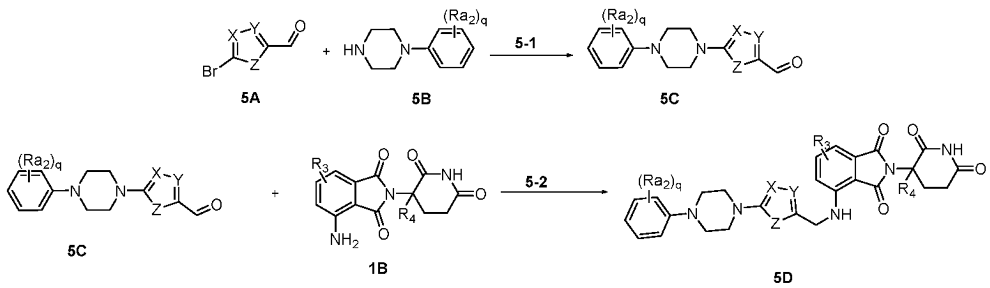


5A 5B 5C

5C 1B 5D

X and Y are each selected from CH or N, and X and Y cannot both be CH; Z is selected from O, S, or NH;

The definitions of $R_3$, $R_4$, $Ra_2$, and q are as described above;

Step 5-1: In the presence of a palladium catalyst, compound 5A undergoes a Buchwald-Hartwig amination reaction with compound 5B to yield compound 5C;

Step 5-2: In the presence of acetic acid and sodium borohydride, compound 5C undergoes reductive amination with compound 1B to yield compound 5D.

The main advantages of the present invention are:

1. The compounds of the present invention exhibit excellent cellular activity, particularly in multiple myeloma MM.1S cells, mantle cell lymphoma Mino cells, and acute myeloid leukemia MV-4-11 cell lines, outperforming marketed drugs, clinical investigational drugs, and compounds disclosed in the prior art that are comparable in structure but do not contain intramolecular hydrogen bonds or specific dihedral angles.

2. The compounds of the present invention have better pharmacokinetics and pharmacodynamics. Pharmacokinetic and pharmacodynamic experiments conducted in mice reveal that the compounds with tested results in the present invention outperform compounds disclosed in the prior art that are comparable in structure but do not contain intramolecular hydrogen bonds. These examples demonstrate the advantages of forming intramolecular hydrogen bonds or specific dihedral angles, and are not limited to other compounds in the present invention which also share the same advantages.

The present invention is further illustrated with specific examples. It should be understood that these examples are provided for the purpose of illustrating the invention and are not intended to limit the scope of the invention. In the following examples, experimental methods that are not specifically mentioned are generally carried out under conventional conditions or according to the conditions recommended by the manufacturer. Unless otherwise specified, percentages and parts are calculated by weight.

In all examples, the $^1H$ NMR spectra were recorded on a 400 MHz Bruker or 500 MHz Bruker NMR spectrometer, with chemical shifts expressed in $\delta$ (ppm); mass spectrometry was measured by MS UPLC-MS (ESI), where the UPLC model is Waters HPLC H-CLASS and the MS (ESI) model is Waters SQ Detector 2. Anhydrous tetrahydrofuran was dried and deoxygenated by refluxing with benzophenone/metal sodium; anhydrous toluene and anhydrous dichloromethane were dried by refluxing with calcium chloride. Solvents for column chromatography, such as petroleum ether, ethyl acetate, and dichloromethane, were purchased from China National Pharmaceutical Group Chemical Reagents Co., Ltd.; the silica gel plates (HSGF254) used for thin-layer chromatography were purchased from China National Pharmaceutical Group Chemical Reagents Co., Ltd.; and the silica gel (200-300 mesh) used for compound separation was selected from China National Pharmaceutical Group Chemical Reagents Co., Ltd. The raw materials in this invention can be obtained

commercially, such as the main reagents purchased from China National Pharmaceutical Group Chemical Reagents Co., Ltd., or prepared by methods known in the art or according to the methods described in the present invention.

## Example

### Synthesis of Key Intermediates:

**Intermediate 1: 2-(1-(tert-Butoxycarbonyl)-1,2,3,6-tetrahydropyridin-4-yl)oxazole-5-carboxylic acid ethyl ester**

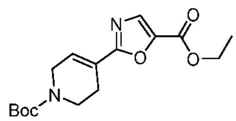

The compound 2-iodo-oxazole-5-carboxylic acid ethyl ester (500 mg, 1.87 mmol), N-Boc-1,2,5,6-tetrahydropyridin-4-boronic ester (636 mg, 2.06 mmol), and triphenylphosphine palladium (216 mg, 0.187 mmol) were dissolved in 16 mL of dioxane, and 4 mL of $Na_2CO_3$ (396 mg, 3.74 mmol) aqueous solution was added. The reaction mixture was purged with nitrogen three times, then heated to 90 °C under nitrogen protection for 4 hours. After the reaction was complete, the mixture was cooled to room temperature and diluted with ethyl acetate. The mixture was washed sequentially with water and saturated sodium chloride solution, separated, and the organic phase was dried over anhydrous sodium sulfate, filtered, and concentrated under reduced pressure. The resulting brown oily substance (424 mg) was obtained after silica gel column chromatography with a yield of 70%. $^1H$ NMR (400 MHz, CDCl3) δ 7.73 (s, 1H), 6.91 (s, 1H), 4.43 - 4.33 (m, 2H), 4.15 (s, 2H), 3.61 (t, J = 4.8 Hz, 2H), 2.64 (s, 2H), 1.48 (s, 9H), 1.38 (t, J = 6.7 Hz, 3H).

**Intermediate 2: 2-(1-(tert-Butoxycarbonyl)-piperidin-4-yl)oxazole-5-carboxylic acid ethyl ester**

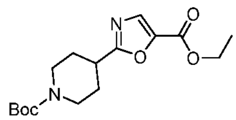

420 mg of ethyl 2-(1-(tert-butoxycarbonyl)-1,2,3,6-tetrahydropyridin-4-yl)oxazole-5-carboxylate was dissolved in 5 mL of tetrahydrofuran. A catalytic amount of Raney nickel was added, the atmosphere was replaced with nitrogen, and then replaced with hydrogen three times. The reaction system was allowed to undergo hydrogenation at room temperature and atmospheric pressure overnight. Upon completion of the reaction, the mixture was filtered through diatomaceous earth, the filter residue was washed with 30 mL of tetrahydrofuran, and the filtrate was concentrated under reduced pressure to obtain the product, which was directly used in the next step.

**Intermediate 3: 4-(5-(Hydroxymethyl)oxazole-2-yl)piperidine-1-carboxylic acid tert-butyl ester**

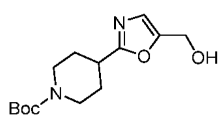

437 mg (1.67 mmol) of 2-(1-(tert-Butoxycarbonyl)-piperidin-4-yl)oxazole-5-carboxylic acid ethyl ester was dissolved in 10 mL of anhydrous ethanol. 256 mg (6.68 mmol) of sodium borohydride was added, and the reaction was allowed to proceed at room temperature for 4 hours. Afterward, the reaction was quenched with water, and the mixture was extracted with ethyl acetate. The layers were separated, and the organic phase was dried over anhydrous sodium sulfate. The solution was then filtered, and the filtrate was concentrated under reduced pressure. The product was obtained as a colorless oily substance (300 mg), corresponding to a yield of 64%. $^1H$ NMR (500 MHz, CDCl3) δ 6.90 (s, 1H), 4.64 (s, 2H), 4.12 (dd, J = 14.3, 7.1 Hz, 2H), 2.98-2.85 (m, 3H), 2.04-1.95 (m, 2H), 1.81 - 1.72 (m, 2H), 1.46 (s, 9H).

**Intermediate 4: tert-Butyl 4-(5-formyloxazol-2-yl)piperidine-1-carboxylate**

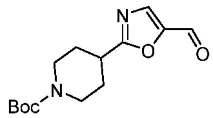

Tert-Butyl 4-(5-(hydroxymethyl)oxazol-2-yl)piperidine-1-carboxylate (300 mg, 1.06 mmol) was dissolved in 8 mL of dry dichloromethane. Under an ice bath, Dess-Martin periodinane (541 mg, 1.28 mmol) was added. After the addition, the reaction was allowed to warm to room temperature and stirred for 3 hours. Upon completion, the reaction was quenched with sodium thiosulfate solution and filtered. The filtrate was extracted with dichloromethane, and the organic phase was washed sequentially with sodium thiosulfate solution and saturated sodium bicarbonate solution. The organic layer was dried, concentrated under reduced pressure, and purified by silica gel column chromatography to afford 290 mg of a white solid with a yield of 98%. $^1$H NMR (400 MHz, CDCl3) δ 9.74 (s, 1H), 7.78 (s, 1H), 4.11 (s, 2H), 3.00 (dt, *J* = 23.4, 11.2 Hz, 3H), 2.08 (d, *J* = 12.9 Hz, 2H), 1.83 (dd, *J* = 22.3, 10.3 Hz, 2H), 1.47 (s, 9H).

**Intermediate 5: tert-Butyl 4-(5-(((2-(2,6-dioxopiperidin-3-yl)-1,3-dioxoisoindolin-4-yl)amino)methyl)oxazol-2-yl) piperidine-1-carboxylate**

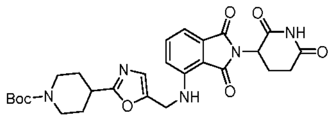

tert-Butyl 4-(5-formyloxazol-2-yl)piperidine-1-carboxylate (300 mg, 1.07 mmol), racemic pomalidomide (292 mg, 1.07 mmol), phenylsilane (132 μL, 1.07 mmol), and dibutyltin dichloride (232 μL, 1.07 mmol) were dissolved in 8 mL of dry tetrahydrofuran. The reaction mixture was heated to 80 °C and stirred overnight. Upon completion of the reaction, the mixture was concentrated under reduced pressure and purified by silica gel column chromatography to afford 262 mg of a yellow solid with a yield of 46%. $^1$H NMR (400 MHz, DMSO) δ 11.10 (s, 1H), 7.59 (t, *J* = 7.6 Hz, 1H), 7.21 (d, *J* = 8.4 Hz, 1H), 7.08 (d, *J* = 6.9 Hz, 1H), 7.02 (d, *J* = 5.8 Hz, 1H), 6.97 (s, 1H), 5.06 (dd, *J* = 12.5, 4.8 Hz, 1H), 4.60 (d, *J* = 5.7 Hz, 2H), 3.85 (d, *J* = 12.6 Hz, 2H), 2.93 (dt, *J* = 24.9, 11.5 Hz, 4H), 2.64-2.53 (m, 1H), 2.08 - 1.98 (m, 1H), 1.91 (d, *J* = 12.6 Hz, 2H), 1.49 (dd, *J* = 23.5, 11.9 Hz, 2H), 1.39 (s, 9H).

**Intermediate 6: 2-(2,6-Dioxopiperidin-3-yl)-4-(((2-(piperidin-4-yl)oxazol-5-yl)methyl)amino)isoindoline-1,3-dione hydrochloride**

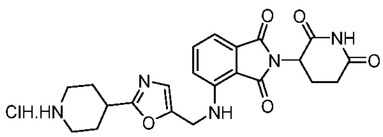

The compound tert-butyl 4-(5-(((2-(2,6-dioxopiperidin-3-yl)-1,3-dioxoisoindolin-4-yl)amino)methyl)oxazol-2-yl) piperidine-1-carboxylate (178 mg) was dissolved in 6 mL of hydrogen chloride in dioxane. The reaction mixture was stirred at room temperature for 5 hours. Upon completion of the reaction, the mixture was concentrated under reduced pressure to afford the product.

**Intermediate 7: N-(Prop-2-en-1-yl)-4-(trifluoromethoxy)benzamide**

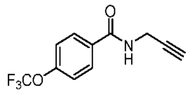

4-(Trifluoromethoxy)benzoic acid (2 g, 9.7 mmol), propargylamine (445 mg, 8.1 mmol), and HATU (3.7 g, 9.7 mmol) were dissolved in 30 mL of DMF. The mixture was stirred at room temperature, and N,N-diisopropylethylamine (8 mL, 48.6 mmol) was added. After approximately 0.5 hours, the reaction was monitored by TLC. Upon completion, the reaction was quenched with water, and the mixture was extracted with ethyl acetate. The organic layer was washed several times with saturated sodium chloride solution, dried, and concentrated. The product was purified by column chromatography to yield 1.6 g of product with a yield of 68%.

**Intermediate 8: 2-(4-(Trifluoromethoxy)phenyl)oxazole-5-carbaldehyde**

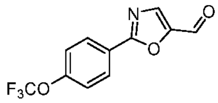

N-(Prop-2-en-1-yl)-4-(trifluoromethoxy)benzamide (671 mg, 2.76 mmol) and iodine (140 mg, 0.55 mmol) were added to a 100 mL round-bottom flask. The atmosphere was exchanged three times with oxygen, and the reaction was irradiated with 420 nm LED light for 10 hours. The reaction was monitored by TLC. Upon completion, the mixture was concentrated under reduced pressure and purified by column chromatography to afford 488 mg of a yellow solid with a yield of 69%. $^{1}H$ NMR (500 MHz, DMSO) δ 9.80 (s, 1H), 8.32 (s, 1H), 8.20 (d, J = 8.9 Hz, 2H), 7.57 (d, J = 8.1 Hz, 2H).

**Intermediate 9: 2-([1,1'-Biphenyl]-3-yl)oxazole-5-carbaldehyde**

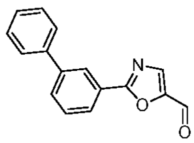

N-(1-Hydroxy-2-propyl)-[1,1'-biphenyl]-3-carboxamide (300 mg, 1.28 mmol) was used as the starting material, and the synthesis method was the same as that for Intermediate 8. The reaction afforded 93 mg of a yellow solid with a yield of 31%. $^{1}H$ NMR (400 MHz, CDCl3) δ 9.84 (s, 1H), 8.42 (s, 1H), 8.17 (d, *J* = 7.7 Hz, 1H), 7.98 (s, 1H), 7.79 (d, *J* = 7.7 Hz, 1H), 7.67 (s, 2H), 7.60 (t, *J* = 7.8 Hz, 1H), 7.49 (t, *J* = 7.5 Hz, 2H), 7.41 (t, *J* = 7.3 Hz, 1H).

**Intermediate 10: 2-(P-Tolyl)oxazole-5-carbaldehyde**

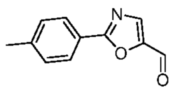

4-Methyl-N-(1-hydroxy-2-propyl)benzamide (500 mg, 2.89 mmol) was used as the starting material, and the synthesis method was the same as that for Intermediate 8. The reaction afforded 0.255 g of a yellow solid with a yield of 51%. $^{1}H$ NMR (400 MHz, DMSO) δ 9.79 (s, 1H), 8.32 (s, 1H), 7.99 (d, *J* = 7.6 Hz, 2H), 7.42 (d, *J* = 7.7 Hz, 2H).

**Intermediate 11: 2-(4-Methoxyphenyl)oxazole-5-carbaldehyde**

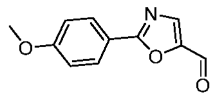

4-Methoxy-N-(1-hydroxy-2-propyl)benzamide (500 mg, 2.46 mmol) was used as the starting material, and the synthesis method was the same as that for Intermediate 8. The reaction afforded a light yellow solid (0.402 g) with a yield of 51%. UPLC-MS (ESI) calculated for: $C_{11}H_9NO_3$ calculated for $[M + H]^+$: 204.20, found: 204.06.

**Intermediate 12: 2-(4-Chlorophenyl)oxazole-5-carbaldehyde**

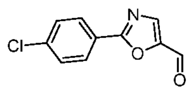

4-Chloro-N-(prop-2-yn-1-yl)benzamide (500 mg, 2.58 mmol) was used as the starting material, and the synthesis method was the same as that for Intermediate 8. The reaction afforded a light yellow solid (289 mg) with a yield of 52%. $^1$H NMR (400 MHz, DMSO) δ 9.82 (s, 1H), 8.35 (s, 1H), 8.10 (d, *J*= *7.5* Hz, 2H), 7.68 (d, *J* = 7.6 Hz, 2H).

**Intermediate 13: 2-(4-Fluorophenyl)oxazole-5-carbaldehyde**

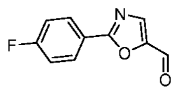

4-Fluoro-N-(1-hydroxy-2-propyl)benzamide (1 g, 5.64 mmol) was used as the starting material, and the synthesis method was the same as that for Intermediate 8. The reaction afforded a light yellow solid (647 mg) with a yield of 60%. $^1$H NMR (400 MHz, DMSO) δ 9.81 (s, 1H), 8.34 (s, 1H), 8.15 (d, J = 6.0 Hz, 2H), 7.45 (s, 2H).

**Intermediate 14: 2-(2-Chlorophenyl)oxazole-5-carbaldehyde**

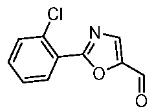

2-Chloro-N-(prop-2-yn-1-yl)benzamide (500 mg, 2.58 mmol) was used as the starting material, and the synthesis method was the same as that for Intermediate 8. The reaction afforded a light yellow solid (230 mg) with a yield of 43%. UPLC-MS (ESI) calculated for: $C_{10}H_6ClNO_2$ $[M + H]^+$: 208.61, found: $[M + H]^+$: 208.12.

**Intermediate 15: 5-(P-Tolyl)oxazole-2-carbaldehyde**

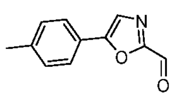

Para-tolualdehyde (3 g, 24.97 mmol) and para-toluenesulfonylmethyl isocyanide (5.12 g, 26.22 mmol) were dissolved in 50 mL of methanol, and potassium carbonate (4.14 g, 29.96 mmol) was added. The reaction was refluxed for 1.5 hours. After the reaction was monitored by TLC and completed, the mixture was cooled to room temperature and quenched with water. The mixture was extracted with ethyl acetate, and the organic layer was washed with saturated sodium chloride solution. The solution was dried over anhydrous sodium sulfate, concentrated under reduced pressure,

and purified by column chromatography to afford 2.5 g of product with a yield of 63%.

5-(P-Tolyl)oxazole (1 g, 6.28 mmol) was dissolved in 20 mL of ether, and under nitrogen protection at -78 °C, a solution of n-butyllithium (2.88 mL, 6.91 mmol) in hexane (2.4 mol/L) was added dropwise. After 30 minutes, DMF (535 μL, 6.91 mmol) in ether (2 mL) was added dropwise, and the reaction was continued at the same temperature for 30 minutes. The temperature was then raised to room temperature and allowed to react for 1.5 hours. Upon completion, the reaction was monitored by TLC, quenched with 2 M HCl to adjust the pH to 5-6, and the mixture was extracted with dichloromethane. The organic layer was separated, dried, concentrated under reduced pressure, and purified by column chromatography to afford a yellow solid (650 mg) with a yield of 55%. UPLC-MS (ESI): calculated for $C_{11}H_9NO_2$ $[M + H]^+$: 188.20, found: 188.16.

### Intermediate 16: 5-(4-Fluorophenyl)thiazole-2-carbaldehyde

Para-fluorophenylboronic acid, 5-bromothiazole, and tetrakis(triphenylphosphine)palladium were added to a 100 mL two-necked flask, followed by the addition of 24 mL of dioxane and 6 mL of an aqueous potassium carbonate solution (2.79 g, 20.13 mmol). The reaction vessel was degassed with nitrogen three times, and the reaction mixture was refluxed at 100 °C under a nitrogen atmosphere for 2 hours. After completion, the reaction was quenched with water at room temperature and extracted with ethyl acetate (2 × 50 mL). The organic layers were combined, washed with saturated sodium chloride solution, dried, and concentrated under reduced pressure. The crude product was purified by column chromatography to yield a reddish-brown oil (1.19 g).

The obtained 5-(4-fluorophenyl)thiazole (1.19 g, 6.64 mmol) was dissolved in 25 mL of anhydrous tetrahydrofuran (THF). Under nitrogen protection at -78 °C, a solution of n-butyllithium (2.5 mol/L, 2.92 mL, 7.3 mmol) in hexane was added dropwise over 30 minutes. DMF (568 μL, 7.30 mmol) in THF (5 mL) was then added dropwise, and the reaction was stirred at the same temperature for 2 hours. The reaction mixture was quenched with 2 M HCl to adjust the pH to 5-6, and extracted with dichloromethane. The organic layer was separated, dried, concentrated under reduced pressure, and purified by column chromatography to yield a yellow solid (743 mg) with a yield of 54%. $^1$H NMR (400 MHz, CDCl3): δ 9.96 (s, 1H), 8.21 (s, 1H), 7.64 (dd, J = 8.7, 5.1 Hz, 2H), 7.17 (t, J = 8.5 Hz, 2H).

### Intermediate 17: 2-(4-(Trifluoromethoxy)phenyl)thiazole-5-carbaldehyde

Para-trifluoromethoxyphenylboronic acid (355 mg, 1.72 mmol), 2-bromo-5-formylthiazole (300 mg, 1.56 mmol), and tetrakis(triphenylphosphine)palladium (180 mg, 0.156 mmol) were added to a 100 mL two-necked flask, followed by the addition of 10 mL of dioxane and 3 mL of an aqueous potassium carbonate solution (647 mg, 4.68 mmol). The reaction vessel was degassed with nitrogen three times, and the reaction mixture was refluxed at 100 °C under a nitrogen atmosphere for 2 hours. After completion, the reaction was quenched with water at room temperature and extracted with ethyl acetate (2 × 50 mL). The organic layers were combined, washed with saturated sodium chloride solution, dried, and concentrated under reduced pressure. The crude product was purified by column chromatography to yield a yellow solid (220 mg) with a yield of 52%. $^1$H NMR (400 MHz, DMSO) δ 10.09 (s, 1H), 8.80 (s, 1H), 8.21 (d, J = 8.7 Hz, 2H), 7.56 (d, J = 8.4 Hz, 2H).

### Intermediate 18: 3-(5-Formylthiazol-2-yl)benzonitrile

3-Cyanophenylboronic acid (253 mg, 1.72 mmol) and 2-bromo-5-formylthiazole (300 mg, 1.56 mmol) were used as raw materials. The synthesis followed the procedure of Intermediate 17. A brown solid (216 mg) was obtained with a yield of 65%. $^1$H NMR (400 MHz, DMSO) δ 10.07 (s, 1H), 8.79 (s, 1H), 8.48 (s, 1H), 8.36 (d, J = 8.0 Hz, 1H), 8.02 (d, J = 7.7 Hz, 1H), 7.74 (t, J = 7.9 Hz, 1H).

### Intermediate 19: 4-(5-Formylthiazol-2-yl)benzonitrile

4-Cyanophenylboronic acid (253 mg, 1.72 mmol) and 2-bromo-5-formylthiazole (300 mg, 1.56 mmol) were used as raw materials. The synthesis followed the procedure of Intermediate 17. A brown solid (235 mg) was obtained with a yield of 66%. $^1$H NMR (400 MHz, DMSO) δ 10.12 (s, 1H), 8.86 (s, 1H), 8.26 (d, J = 8.3 Hz, 2H), 8.04 (d, J = 8.3 Hz, 2H).

### Intermediate 20: 2-(4-(Trifluoromethyl)phenyl)thiazole-5-carbaldehyde

4-(Trifluoromethyl)phenylboronic acid (218 mg, 1.15 mmol) and 2-bromo-5-formylthiazole (200 mg, 1 mmol) were used as raw materials. The synthesis followed the procedure of Intermediate 17. A yellow solid (162 mg) was obtained with a yield of 40%. $^1$H NMR (400 MHz, CDCl3) δ 10.08 (s, 1H), 8.48 (s, 1H), 8.15 (d, J = 6.8 Hz, 2H), 7.75 (d, J = 6.8 Hz, 2H).

**Intermediate 21: 2-(2,4-Dimethoxyphenyl)thiazole-5-carbaldehyde**

2,4-Dimethoxyphenylboronic acid (313 mg, 1.72 mmol) and 2-bromo-5-formylthiazole (300 mg, 1.56 mmol) were used as raw materials. The synthesis followed the procedure of Intermediate 17. A yellow solid (249 mg) was obtained with a yield of 64%. UPLC-MS (ESI) calculated for $C_{12}H_{11}NO_3S$ $[M + H]^+$: 250.28, found: 250.52.

**Intermediate 22: 2-(3-Fluorophenyl)thiazole-5-carbaldehyde**

3-Fluorophenylboronic acid (88 mg, 0.63 mmol) and 2-bromo-5-formylthiazole (110 mg, 0.57 mmol) were used as raw materials. The synthesis followed the procedure of Intermediate 17. A pale yellow solid (94 mg) was obtained with a yield of 72%. $^1H$ NMR (500 MHz, DMSO) δ 10.10 (s, 1H), 8.81 (s, 1H), 7.95-7.92 (m, 1H), 7.92-7.89 (m, 1H), 7.62 (td, J = 8.0, 5.9 Hz, 1H), 7.50-7.44 (m, 1H).

**Intermediate 23: 2-(p-Tolyl)thiazole-5-carbaldehyde**

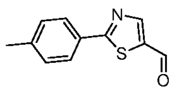

p-Tolylboronic acid (156 mg, 1.14 mmol) and 2-bromo-5-formylthiazole (200 mg, 1.04 mmol) were used as raw materials. The synthesis followed the procedure of Intermediate 17. A yellow solid (162 mg) was obtained with a yield of 70%. $^1H$ NMR (500 MHz, DMSO) δ 10.07 (s, 1H), 8.75 (s, 1H), 7.99-7.96 (m, 2H), 7.38 (d, J = 7.9 Hz, 2H), 2.39 (s, 3H).

**Intermediate 24: N-Methyl-1-(2-(4-(Trifluoromethoxy)phenyl)oxazol-5-yl)methanamine**

2-(4-(Trifluoromethoxy)phenyl)oxazole-5-carbaldehyde (200 mg, 0.778 mmol) was dissolved in 10 mL of methanol, and methylamine (40% methanol solution) was added at room temperature. The reaction proceeded for 1 hour. At 0 °C, sodium borohydride (59 mg, 1.55 mmol) was added and the reaction was stirred for 30 minutes at room temperature. Acetone (10 mL) was added to quench the reaction. After concentrating under reduced pressure, a white solid (190 mg) was obtained with a yield of 90%. $^1H$ NMR (500 MHz, DMSO) δ 8.06 (d, J = 8.8 Hz, 2H), 7.52 (d, J = 8.3 Hz, 2H), 7.17 (s, 1H), 3.76 (s, 2H), 2.30 (s, 3H).

**Intermediate 25: Oxazole-5-methanol**

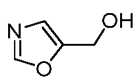

At 0 °C, a solution of ethyl oxazole-5-carboxylate (9 g, 63.78 mmol) in 10 mL of THF was added dropwise to a suspension of LiAlH4 (2.548 g, 63.78 mmol) in 100 mL of THF. The reaction mixture was stirred for 30 minutes. Upon completion of the reaction, water was added at 0 °C to quench the reaction. The mixture was filtered, and the solid residue was washed with ethyl acetate. The filtrate was concentrated under reduced pressure, and the crude product was purified by silica gel column chromatography to yield a red oily product (3.039 g) with a yield of 48.09%. 1H NMR (500 MHz, DMSO) δ 8.28 (s, 1H), 7.04 (s, 1H), 5.37 (t, J = 5.8 Hz, 1H), 4.47 (d, J = 5.8 Hz, 2H).

**Intermediate 26: 5-((((tert-Butyldiphenylsilyl)oxy)methyl)oxazole**

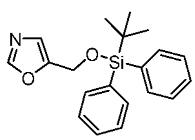

Oxazole-5-methanol (1.15 g, 11.61 mmol) and imidazole (1.580 g, 23.21 mmol) were dissolved in 20 mL of dry DMF. tert-Butyldiphenylsilyl chloride (3.142 mL, 12.77 mmol) was added, and the reaction mixture was stirred for 1 hour. Upon completion, water was added to quench the reaction. The mixture was extracted with ethyl acetate, and the organic layer was washed several times with saturated sodium chloride solution, dried, and concentrated. The crude

product was purified by column chromatography to yield a colorless oily product (3.918 g) with a yield of 100%. $^1$H NMR (500 MHz, DMSO) δ 8.33 (s, 1H), 7.66-7.61 (m, 4H), 7.51-7.43 (m, 6H), 7.01 (s, 1H), 4.74 (s, 2H), 0.99 (s, 9H).

**Intermediate 27: (5-(((tert-Butyldiphenylsilyl)oxy)methyl)oxazol-2-yl)methanol**

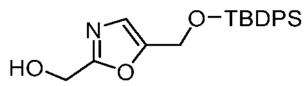

Compound 5-((((tert-butyldiphenylsilyl)oxy)methyl)oxazole (3.918 g, 11.61 mmol) was dissolved in 30 mL of dry THF and degassed with nitrogen three times. At 0 °C, borane tetrahydrofuran complex (15.3 mL, 15.30 mmol) was added, and the mixture was stirred for 1 hour. The reaction mixture was then cooled to -78 °C, and a solution of n-butyllithium in hexane (6.116 mL, 15.29 mmol) was added dropwise. The mixture was stirred at -78 °C for 2 hours, followed by the slow addition of 4-formylmorpholine (1.6 mL, 15.29 mmol). The reaction was allowed to warm to -25 °C and quenched with 35 mL of 5% v/v acetic acid in ethanol solution. The mixture was stirred at room temperature for 30 minutes, concentrated under reduced pressure, and the residue was dissolved in ethyl acetate. The organic layer was washed sequentially with saturated sodium bicarbonate and saturated sodium chloride solutions, dried over anhydrous sodium sulfate, filtered, and concentrated under reduced pressure. The crude product was purified by silica gel column chromatography to yield a colorless oily product (2.724 g) with a yield of 63.84%. $^1$H NMR (500 MHz, DMSO) δ 7.65-7.62 (m, 4H), 7.50-7.43 (m, 6H), 6.91 (s, 1H), 5.63 (t, J = 6.2 Hz, 1H), 4.71 (s, 2H), 4.46 (d, J = 6.2 Hz, 2H), 0.99 (d, J = 5.0 Hz, 9H).

**Intermediate 28: 5-(((tert-Butyldiphenylsilyl)oxy)methyl)oxazole-2-carbaldehyde**

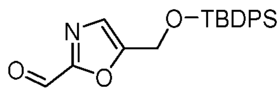

Compound (5-(((tert-butyldiphenylsilyl)oxy)methyl)oxazol-2-yl)methanol (1.254 g, 3.412 mmol) was dissolved in 10 mL of dichloromethane. At 0 °C, Dess-Martin periodinane (1.592 g, 3.753 mmol) was added. The reaction was allowed to proceed at room temperature for 1 hour. Upon completion, 1 N NaOH was added to quench the reaction. The aqueous layer was extracted with dichloromethane, and the combined organic layers were washed with saturated sodium chloride solution, dried over anhydrous sodium sulfate, filtered, and concentrated under reduced pressure. The crude product was purified by silica gel column chromatography to yield a colorless oily product (500 mg) with a yield of 40.09%. $^1$H NMR (500 MHz, DMSO) δ 9.66 (s, 1H), 7.64 (dd, J = 7.9, 1.5 Hz, 4H), 7.49-7.43 (m, 7H), 4.85 (s, 2H), 1.01 (s, 9H).

**Intermediate 29: (2R,6S)-4-((5-(((tert-Butyldiphenylsilyl)oxy)methyl)oxazol-2-yl)methyl)-2,6-dimethylmorpholine**

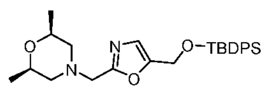

Compound 5-(((tert-butyldiphenylsilyl)oxy)methyl)oxazole-2-carbaldehyde (500 mg, 1.368 mmol) was dissolved in 5 mL of 1,2-dichloroethane. cis-2,6-Dimethylmorpholine (204 μL, 1.642

mmol) was added, and at 0 °C, sodium triacetoxyborohydride (580 mg, 2.736 mmol) was introduced. The reaction mixture was stirred at room temperature for 6 hours. Upon completion of the reaction, the mixture was quenched with ice water and extracted twice with dichloromethane. The organic phases were combined, washed with saturated sodium chloride solution, dried over anhydrous sodium sulfate, and filtered. The filtrate was concentrated under reduced

pressure, and the crude product was purified by silica gel column chromatography to yield an oily product (527 mg) with a yield of 82.60%. $^1$H NMR (500 MHz, CDCl3) δ 7.67 (dd, *J* = 8.0, 1.4 Hz, 4H), 7.47 - 7.37 (m, 6H), 6.77 (s, 1H), 4.67 (s, 2H), 3.72 (dt, *J* = 13.6, 6.9 Hz, 2H), 3.65 (s, 2H), 2.75 (d, *J* = 10.8 Hz, 2H), 1.91 (t, *J* = 10.6 Hz, 2H), 1.14 (d, *J* = 6.3 Hz, 6H), 1.05 (s, 9H).

**Intermediate 30: (2-(((2R,6S)-2,6-dimethylmorpholin-4-yl)methyl)oxazol-5-yl)methanol**

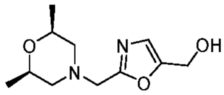

Compound (2R,6S)-4-((5-(((tert-butyldiphenylsilyl)oxy)methyl)oxazol-2-yl)methyl)-2,6-dimethylmorpholine (527 mg, 1.13 mmol) was dissolved in 5 mL of dry tetrahydrofuran. The reaction mixture was cooled to 0 °C, and a solution of tetrabutylammonium fluoride in tetrahydrofuran (1.7 mL, 1.70 mmol) was added. The reaction was warmed to room temperature and stirred for 2 hours. Upon completion, water was added to quench the reaction. The mixture was extracted twice with ethyl acetate, and the combined organic phases were dried over anhydrous sodium sulfate, filtered, and concentrated under reduced pressure. The crude product was purified by silica gel column chromatography to obtain an oily product (170 mg) with a yield of 66.49%. $^1$H NMR (500 MHz, CDCl3) δ 6.96 (s, 1H), 4.67 (s, 2H), 3.72 (dqd, J = 12.5, 6.3, 2.0 Hz, 2H), 3.66 (s, 2H), 2.76 (d, J = 10.4 Hz, 2H), 1.94 - 1.87 (m, 2H), 1.15 (d, J = 6.3 Hz, 6H).

**Intermediate 31: 2-(((2R,6S)-2,6-dimethylmorpholin-4-yl)methyl)oxazol-5-carbaldehyde**

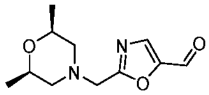

Compound (2-(((2R,6S)-2,6-dimethylmorpholin-4-yl)methyl)oxazol-5-yl)methanol (170 mg, 0.7513 mmol) was dissolved in 3 mL of dichloromethane. At 0 °C, Dess-Martin periodinane (351 mg, 0.8264 mmol) was added. The reaction was warmed to room temperature and stirred for 2 hours. Upon completion, 1N NaOH was added to quench the reaction. The aqueous phase was extracted with dichloromethane, and the combined organic phases were washed with saturated sodium chloride solution, dried over anhydrous sodium sulfate, filtered, and concentrated under reduced pressure. The crude product was purified by silica gel column chromatography to yield a colorless oily product (151 mg) with a yield of 89.62%. $^1$H NMR (500 MHz, CDCl3) δ 9.79 (s, 1H), 7.82 (s, 1H), 3.79 (s, 2H), 3.73 (dqd, J = 12.5, 6.3, 2.1 Hz, 2H), 2.79 (d, J = 10.2 Hz, 2H), 1.99 - 1.93 (m, 2H), 1.15 (d, J = 6.3 Hz, 6H).

**Intermediate 32: 3-Fluoro-4-(4-(5-formylthiazol-2-yl)piperazin-1-yl)benzonitrile**

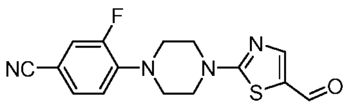

Compound 2-bromothiazole-5-carbaldehyde (100 mg, 0.521 mmol), 3-fluoro-4-(piperazin-1-yl)benzonitrile (128 mg, 0.625 mmol), 4,5-bis(diphenylphosphino)-9,9-dimethylxanthene (30 mg, 0.0521 mmol), tris(dibenzylideneacetone) dipalladium (15 mg, 0.0261 mmol), and cesium carbonate (238 mg, 0.729 mmol) were dissolved in 10 mL of toluene. The reaction mixture was purged with nitrogen gas three times and heated to 100 °C under a nitrogen atmosphere for 12 hours. Upon completion, the reaction was cooled to room temperature, diluted with ethyl acetate, and washed sequentially with water and saturated sodium chloride solution. The organic phase was dried over anhydrous sodium sulfate, filtered, and concentrated under reduced pressure. The crude product was purified by silica gel column chromatography to obtain a brown solid (102 mg) with a yield of 62%.

**Synthesis of Exemplary Compounds:**

**Synthetic Route 1:**

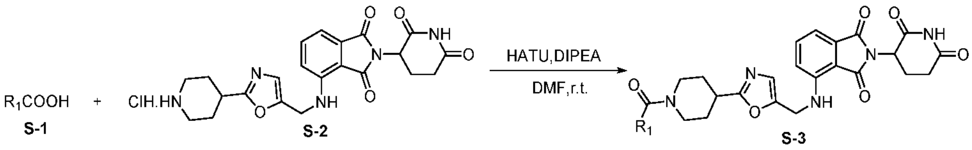


Wherein $R_1$ is as defined above, and S-2 represents the aforementioned intermediate. Reaction conditions are specified in the following specific examples.

**Example 1: 2-(2,6-Dioxopiperidin-3-yl)-4-((((2-(1-(1-methylcyclobutane-1-carbonyl)piperidin-4-yl)oxazol-5-yl) methyl)amino)isoindoline-1,3-dione**

2-(2,6-Dioxopiperidin-3-yl)-4-((((2-(piperidin-4-yl)oxazol-5-yl)methyl)amino)isoindoline-1,3-dione hydrochloride (40 mg, 0.084 mmol), 1-methylcyclobutanecarboxylic acid (11 mg, 0.092 mmol), and N,N,N',N'-tetramethyl-O-(7-azabenzotriazol-1-yl)hexafluorophosphate uronium (35 mg, 0.092 mmol) were dissolved in 3 mL of N,N-dimethylformamide. Under stirring, N,N-diisopropylethylamine (42 μL, 0.252 mmol) was added. The reaction mixture was stirred at room temperature for 1 hour. Completion of the reaction was monitored by LC-MS. Afterward, the reaction mixture was purified by HPLC to afford the desired yellow solid (21 mg) with a yield of 47%. $^1$H NMR (600 MHz, DMSO) δ 11.10 (s, 1H), 7.62 - 7.55 (m, 1H), 7.21 (d, *J* = 8.6 Hz, 1H), 7.08 (d, *J* = 7.1 Hz, 1H), 7.02 (t, *J* = 6.1 Hz, 1H), 6.98 (s, 1H), 5.06 (dd, *J* = 12.9, 5.4 Hz, 1H), 4.61 (d, *J* = 6.0 Hz, 2H), 4.19 (s, 1H), 3.51 (s, 1H), 3.05 (td, *J* = 10.8, 5.4 Hz, 2H), 2.92 - 2.83 (m, 1H), 2.77 (s, 1H), 2.59 (d, *J* = 18.1 Hz, 1H), 2.55 - 2.51 (m, 1H), 2.37 (q, *J* = 9.5 Hz, 2H), 2.06 - 1.99 (m, 1H), 1.97 - 1.85 (m, 3H), 1.78 (t, *J* = 9.5 Hz, 2H), 1.57 (dt, *J* = 69.6, 34.8 Hz, 3H), 1.32 (s, 3H).UPLC-MS (ESI) calculated for $C_{28}H_{31}N_5O_6$ [M + H ]$^+$: 534.23, found: 534.36.

**Example 2: 2-(2,6-Dioxopiperidin-3-yl)-4-((((2-(1-(1-(4-fluorophenyl)cyclobutane-1-carbonyl)piperidin-4-yl)oxazol-5-yl)methyl)amino)isoindoline-1,3-dione**

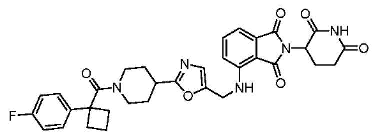

Using 1-(4-fluorophenyl)cyclobutane-1-carboxylic acid and Intermediate 6 as starting materials, the reaction was performed following the same synthetic route described in Synthesis Route 1 and Example 1. The reaction yielded a yellow solid (18 mg) with a yield of 35%. $^1$H NMR (500 MHz, DMSO) δ 11.11 (s, 1H), 7.61 - 7.54 (m, 1H), 7.38 (dd, *J* = 8.6, 5.4 Hz, 2H), 7.23 - 7.14 (m, 3H), 7.07 (d, *J* = 7.1 Hz, 1H), 7.02-6.97 (t, 1H), 6.92 (s, 1H), 5.06 (dd, *J* = 12.8, 5.4 Hz, 1H), 4.57 (d, *J* = 6.2 Hz, 2H), 4.26 (d, *J* = 11.6 Hz, 1H), 3.23 (d, *J* = 13.1 Hz, 1H), 2.96 - 2.84 (m, 2H), 2.83-2.73 (m, 3H), 2.68-2.55 (m, 2H), 2.44-2.34 (m, 2H), 2.26-2.15 (m, 1H), 2.05-1.97 (m, 1H), 1.87 (dd, *J* = 18.2, 9.8 Hz, 2H), 1.76 (dt, *J* = 15.3, 7.3 Hz, 1H), 1.54 (d, *J* = 9.8 Hz, 1H), 1.43-1.33 (m, 1H). UPLC-MS (ESI ) calculated for $C_{33}H_{32}FN_5O_6$ [M + H ]$^+$: 614.23, found: 614.45

Example 3: 4-((2-(1-((3r,5r,7r)-adamantane-1-carbonyl)piperidin-4-yl)oxazol-5-yl)methyl)amino)-2-(2,6-dioxopiperidin-3-yl)isoindoline-1,3-dione

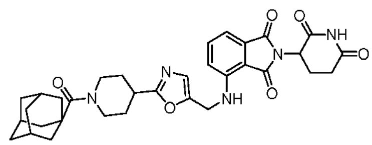

Using 1-adamantanecarboxylic acid and Intermediate 6 as starting materials, the reaction was performed

following the same synthetic route described in Synthesis Route 1 and Example 1. The reaction yielded a yellow solid (25 mg) with a yield of 50%. $^1$H NMR (500 MHz, DMSO) δ 11.11 (s, 1H), 7.59 (dd, *J* = 14.0, 6.0 Hz, 1H), 7.21 (d, *J* = 8.6 Hz, 1H), 7.08 (d, *J* = 7.1 Hz, 1H), 7.02 (dd, *J* = 12.6, 6.4 Hz, 1H), 6.98 (s, 1H), 5.06 (dd, *J* = 12.8, 5.3 Hz, 1H), 4.61 (d, *J* = 6.2 Hz, 1H), 4.25 (d, *J* = 13.2 Hz, 2H), 3.12 - 3.05 (m, 1H), 2.99 (t, *J* = 12.2 Hz, 2H), 2.93 - 2.84 (m, 1H), 2.65-2.52 (m, 2H), 2.06 - 1.99 (m, 1H), 1.99-1.91 (m, 5H), 1.88 (s, 5H), 1.80 (dd, *J* = 18.5, 8.2 Hz, 2H), 1.73 - 1.61 (m, 7H), 1.49 (dd, *J* = 21.3, 10.9 Hz, 2H). UPLC-MS (ESI ) calculated for $C_{33}H_{37}N_5O_6$ [M + H ]$^+$: 600.27 found: 600.41

**Example 4: 2-(2,6-Dioxopiperidin-3-yl)-4-((((2-(1-(1-(trifluoromethoxy)cyclobutane-1-carbonyl)piperidin-4-yl) oxazol-5-yl)methyl)amino)isoindoline-1,3-dione**

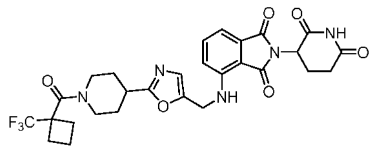

Using 2-bromo-6-trifluoromethylpyridine and Intermediate 6 as starting materials, the reaction was performed following the same synthetic route described in Synthesis Route 1 and Example 1. A yellow solid (20.5 mg) was obtained with a yield of 42%. $^1$H NMR (500 MHz, DMSO) δ 11.11 (s, 1H), 7.62 - 7.55 (m, 1H), 7.21 (d, J = 8.6 Hz, 1H), 7.08 (d, J = 7.1 Hz, 1H), 7.03 (t, J = 6.1 Hz, 1H), 6.98 (s, 1H), 5.06 (dd, J = 12.8, 5.4 Hz, 1H), 4.61 (d, J = 6.2 Hz, 2H), 4.26 (d, J = 12.5 Hz, 1H), 3.50 (d, J = 12.3 Hz, 1H), 3.17 - 3.05 (m, 2H), 2.94 - 2.82 (m, 2H), 2.7-2.53 (m, 4H), 2.46-2.32 (m, 3H), 2.06 - 1.90 (m, 5H), 1.8-1.71(m, 1H), 1.63-1.49 (m, 2H). UPLC-MS (ESI ) calculated for $C_{28}H_{28}F_3N_5O_7$ [M + H ]$^+$: 588.20, found:588.41.

**Example 5: 2-(2,6-Dioxopiperidin-3-yl)-4-((((2-(1-(4-fluorobenzoyl)piperidin-4-yl)oxazol-5-yl)methyl)amino)isoindoline-1,3-dione**

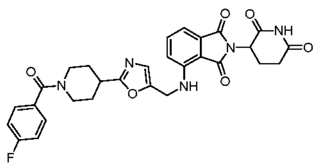

Using 4-fluorobenzoic acid and Intermediate 6 as starting materials, the reaction was performed following the same synthetic route described in Synthesis Route 1 and Example 1. A yellow solid (20 mg) was obtained with a yield of 43%. $^1$H NMR (600 MHz, DMSO) δ 11.10 (s, 1H), 7.62 - 7.57 (m, 1H), 7.48 - 7.43 (m, 2H), 7.26 (t, *J* = 8.9 Hz, 2H), 7.21 (d, *J* = 8.6 Hz, 1H), 7.08 (d, *J* = 7.1 Hz, 1H), 7.02 (t, *J* = 6.3 Hz, 1H), 6.99 (s, 1H), 5.06 (dd, *J* = 12.9, 5.4 Hz, 1H), 4.61 (d, *J* = 6.2 Hz, 2H), 4.32 (s, 1H), 3.55 (s, 1H), 3.17 - 3.01 (m, 3H), 2.88 (ddd, *J* = 17.1, 13.9, 5.4 Hz, 1H), 2.62 - 2.56 (m, 1H), 2.56 - 2.52 (m, 1H), 2.07-1.87 (m, 3H), 1.68-1.57 (m, 2H).UPLC-MS ( ESI ) calculated for $C_{29}H_{26}FN_5O_6$ [M + H ]$^+$: 560.19, found:560.73.

**Example 6: 4-(4-(5-(((2-(2,6-dioxopiperidin-3-yl)-1,3-dioxoisindole-4-yl)amino)methyl)oxazol-2-yl)piperidine-1-carbonyl)-3-fluorobenzonitrile**

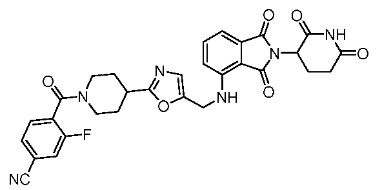

Using 4-cyano-2-fluorobenzoic acid and Intermediate 6 as starting materials, the reaction was performed following the same synthetic route described in Synthesis Route 1 and Example 1. A yellow solid (13 mg) was obtained with a yield of 26%. $^1$H NMR (500 MHz, DMSO) δ 11.11 (s, 1H), 8.00 (d, *J* = 9.2 Hz, 1H), 7.79 (d, *J* = 7.8 Hz, 1H), 7.63 - 7.55

(m, 2H), 7.21 (d, *J* = 8.6 Hz, 1H), 7.08 (d, *J* = 7.1 Hz, 1H), 7.03 (t, *J* = 5.9 Hz, 1H), 6.99 (s, 1H), 5.06 (dd, *J* = 12.8, 5.4 Hz, 1H), 4.61 (d, *J* = 6.2 Hz, 2H), 4.37 (d, *J* = 13.3 Hz, 1H), 3.36 (s, 1H), 3.22 - 3.12 (m, 2H), 3.06 (dd, *J* = 17.7, 7.0 Hz, 1H), 2.93 - 2.82 (m, 1H), 2.64 - 2.52 (m, 2H), 2.11 - 1.86 (m, 3H), 1.56 (d, *J* = 10.1 Hz, 2H). UPLC-MS ( ESI ) calculated for $C_{30}H_{25}FN_6O_6$ [M + H ]$^+$: 585.18, found: 585.25.

**Example 7: 2-(2,6-Dioxopiperidin-3-yl)-4-((((2-(1-(4-(trifluoromethoxy)benzoyl)piperidin-4-yl)oxazol-5-yl) methyl)amino)isoindoline-1,3-dione**

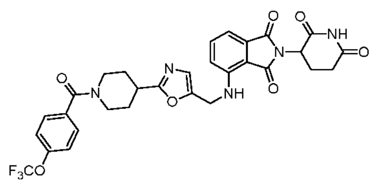

Using 4-(trifluoromethoxy)benzoic acid and Intermediate 6 as starting materials, the reaction was performed following the same synthetic route described in Synthesis Route 1 and Example 1. A yellow solid (20 mg) was obtained with a yield of 38%. $^1$H NMR (500 MHz, DMSO) δ 11.11 (s, 1H), 7.60 (s, 1H), 7.54 (d, *J* = 8.5 Hz, 2H), 7.43 (d, *J* = 8.2 Hz, 2H), 7.21 (d, *J* = 8.6 Hz, 1H), 7.08 (d, *J* = 7.1 Hz, 1H), 7.03 (s, 1H), 6.99 (s, 1H), 5.06 (dd, *J* = 12.8, 5.4 Hz, 1H), 4.61 (d, *J* = 6.1 Hz, 2H), 4.34 (s, 1H), 3.51 (s, 1H), 3.20-2.98 (m, 3H), 2.94 - 2.81 (m, 1H), 2.63 - 2.52 (m, 2H), 2.08 - 1.87 (m, 3H), 1.70-1.55 (m, 2H). UPLC-MS( ESI ) calculated for $C_{30}H_{26}F_3N_5O_7$ [M + H ]$^+$: 626.18, found: 626.26.

**Example 8: 4-((2-(1-(2,4-Dichlorobenzoyl) piperidin-4-yl) oxazole-5-yl)methyl) amino)-2-(2,6-dioxopiperidin-3-yl) isoindolin-1,3-dione**

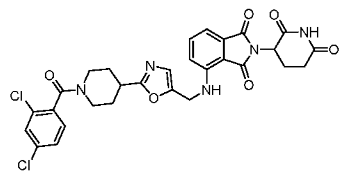

Using 2,4-dichlorobenzoic acid and intermediate 6 as starting materials, the procedure followed the same synthetic route as in Synthesis Route 1 and Example 1. A yellow solid (20 mg) was obtained with a yield of 39%. $^1$H NMR (500 MHz, DMSO) δ 11.11 (s, 1H), 7.72 (d, *J* = 1.7 Hz, 1H), 7.66 - 7.54 (m, 1H), 7.50 (ddd, *J* = 8.0, 3.8, 1.8 Hz, 1H), 7.42 (dd, *J* = 27.4, 8.2 Hz, 1H), 7.21 (d, *J* = 7.8 Hz, 1H), 7.08 (d, *J* = 7.1 Hz, 1H), 7.03 (t, *J* = 6.2 Hz, 1H), 6.99 (s, 1H), 5.06 (dd, *J* = 12.8, 5.4 Hz, 1H), 4.61 (d, *J* = 6.2 Hz, 2H), 4.37 (d, *J* = 13.2 Hz, 1H), 3.27 (d, *J* = 14.1 Hz, 1H), 3.10 (ddt, J= 29.8, 21.2, 10.3 Hz, 3H), 2.93 - 2.82 (m, 1H), 2.65-2.55 (m, 2H), 2.10-1.99 (m, 2H), 1.89 (t, *J* = 13.4 Hz, 1H), 1.71 - 1.48 (m, 2H).UPLC-MS ( ESI ) calculated for $C_{29}H_{25}Cl_2N_5O_6$ [M + H ]$^+$: 610.12, found: 610.28.

**Example 9: 2-(2,6-Dioxopiperidin-3-yl)-4-((2-(1-(1-n-pentylpyridine-4-yl)oxazole-5-yl)methyl)amino)indole-1,3-dione**

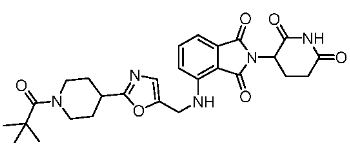

Using trimethylacetic acid and intermediate 6 as starting materials, following the same synthetic route as in Route 1 and Example 1, a yellow solid (20 mg) was obtained with a yield of 46%. $^1$H NMR (600 MHz, DMSO) δ 11.10 (s, 1H), 7.59 (s, 1H), 7.21 (d, *J* = 8.6 Hz, 1H), 7.08 (d, *J* = 7.1 Hz, 1H), 7.02 (s, 1H), 6.98 (s, 1H), 5.06 (dd, *J* = 12.9, 5.4 Hz, 1H), 4.61 (d, *J* = 6.2 Hz, 2H), 4.17 (d, *J* = 13.5 Hz, 2H), 3.12 - 3.04 (m, 1H), 2.99 (t, *J* = 12.0 Hz, 2H), 2.92 - 2.85 (m, 1H), 2.62-2.52 (m, 2H),

2.06 - 2.00 (m, 1H), 1.95 (dd, *J* = 13.1, 2.5 Hz, 2H), 1.51 (td, *J* = 14.5, 3.6 Hz, 2H), 1.18 (s, 9H). UPLC-MS (ESI ) calculated for $C_{27}H_{31}N_5O_6$ [M + H ]$^+$: 522.23, found: 522.44.

**Example 10: 2-(2,6-Dioxopiperidin-3-yl)-4-(((2-(1-(1-methylcyclohexylcarboxyl)pyridine-4-yl)oxazole-5-yl) methyl)amino)indoline-1,3-dione**

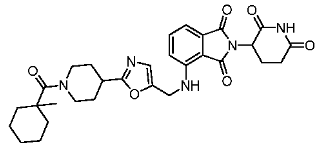

Using 1-methyl-1-cyclohexanecarboxylic acid and intermediate 6 as starting materials, following the same synthetic route as in Route 1 and Example 1, a yellow solid (18 mg) was obtained with a yield of 38%. $^1$H NMR (500 MHz, DMSO) δ 11.11 (s, 1H), 7.62 - 7.57 (m, 1H), 7.21 (d, *J* = 8.6 Hz, 1H), 7.08 (d, *J* = 7.1 Hz, 1H), 7.03 (s, 1H), 6.97 (s, 1H), 5.06 (dd, *J* = 12.8, 5.4 Hz, 1H), 4.61 (d, *J* = 6.2 Hz, 2H), 4.18 (d, *J* = 13.4 Hz, 2H), 3.13 - 3.04 (m, 1H), 2.98 (t, *J* = 12.0 Hz, 2H), 2.92 - 2.82 (m, 1H), 2.57 (ddd, *J* = 17.7, 15.6, 9.9 Hz, 2H), 2.07 - 1.99 (m, 1H), 1.94 (d, *J* = 10.3 Hz, 4H), 1.54 - 1.43 (m, 4H), 1.39 (dd, *J* = 13.2, 6.2 Hz, 3H), 1.32 - 1.20 (m, 4H), 1.15 (s, 3H).UPLC-MS (ESI ) calculated for $C_{30}H_{35}N_5O_6$ [M + H ]$^+$: 562.26, found:562.82.

**Example 11: 4-(((2-(1-(2-((3r,5r,7r)-adamantane-1-yl)acetyl)pyridine-4-yl)oxazole-5-yl)methyl)amino)indole-1,3-dione**

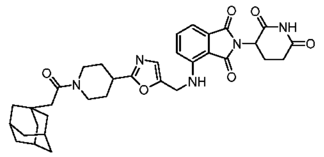

Using 1-adamantane acetic acid and intermediate 6 as starting materials, following the same synthetic route as in Route 1 and Example 1, a yellow solid (26 mg) was obtained with a yield of 50%.$^1$H NMR (500 MHz, DMSO) δ 11.11 (s, 1H), 7.62 - 7.56 (m, 1H), 7.21 (d, *J* = 8.6 Hz, 1H), 7.08 (d, *J* = 7.1 Hz, 1H), 7.03 (t, *J* = 6.3 Hz, 1H), 6.98 (s, 1H), 5.06 (dd, *J* = 12.8, 5.4 Hz, 1H), 4.61 (d, *J* = 6.2 Hz, 2H), 4.30 (d, *J* = 13.1 Hz, 1H), 3.93 (d, *J* = 14.0 Hz, 1H), 3.15 (t, *J* = 11.4 Hz, 1H), 3.08 - 3.02 (m, 1H), 2.92 - 2.83 (m, 1H), 2.75 (t, *J* = 12.0 Hz, 1H), 2.65 - 2.52 (m, 2H), 2.13 (d, *J* = 13.5 Hz, 1H), 2.04 (dd, *J* = 18.4, 9.4 Hz, 2H), 1.98 - 1.88 (m, 5H), 1.64 (d, *J* = 11.9 Hz, 3H), 1.61-1.55 (m, 10H), 1.43 (d, *J* = 13.1 Hz, 1H). UPLC-MS ( ESI ) calculated for $C_{34}H_{39}N_5O_6$ [M + H ]$^+$: 614.29, found:614.37.

**Example 12: 2-(2,6-Dioxopiperidin-3-yl)-4-((2-(1-(4-fluorobenzyl)pyridine-4-yl)oxazole-5-yl)methyl)amino)indole-1,3-dione**

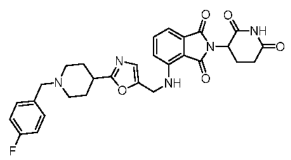

Using 4-fluorobenzaldehyde (9 mg, 0.076 mmol), intermediate 6 (30 mg, 0.063 mmol), and ZnCl2 (1M in THF, 66 μL) in tetrahydrofuran, stirring at room temperature for 3 hours, followed by the addition of sodium cyanoborohydride (8 mg, 0.126 mmol), the reaction was allowed to proceed overnight at room temperature. After monitoring the completion of the reaction by LC-Mass, ethyl acetate was added for dilution. The mixture was washed with saturated sodium chloride solution, separated, dried over anhydrous sodium sulfate, filtered, and the filtrate concentrated under reduced pressure. The crude product was purified by HPLC to yield a yellow

solid (10 mg, 29%).$^1$H NMR (500 MHz, DMSO) δ 11.11 (s, 1H), 7.62 - 7.57 (m, 1H), 7.32 (s, 2H), 7.20 (d, *J* = 8.6 Hz, 1H), 7.13 (t, *J* = 8.6 Hz, 2H), 7.08 (d, *J* = 7.1 Hz, 1H), 7.02 (t, *J* = 6.2 Hz, 1H), 6.96 (s, 1H), 5.06 (dd, *J* = 12.9, 5.4 Hz, 1H), 4.60 (d, *J* = 6.2 Hz, 2H), 3.49-3.39 (m, 2H), 2.92 - 2.83 (m, 1H), 2.81-2.70 (m, 3H), 2.65 - 2.53 (m, 2H), 2.11 - 1.97 (m, 3H), 1.91 (d, *J* = 11.4 Hz, 2H), 1.70-1.60 (m, 2H). UPLC-MS (ESI ) calculated for $C_{29}H_{28}FN_5O_5$ [M + H ]$^+$: 546.21, found:546.84.

**Example 13: 4-(((4-(5-((2-(2,6-dioxopiperidin-3-yl)-1,3-dioxoisoquinolin-4-yl)amino)methyl)oxazole-2-yl)pyridine-1-yl)methyl)aminomethyl)-3-fluorobenzonitrile**

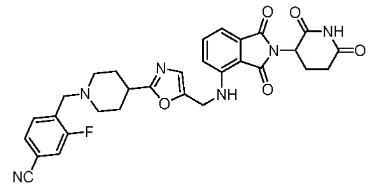

Using 4-cyano-2-fluorobenzaldehyde and intermediate 6 as starting materials, following the same synthetic route as Example 12, a yellow solid (13 mg) was obtained with a yield of 38%.$^1$H NMR (500 MHz, DMSO) δ 11.10 (s, 1H), 7.82 (dd, *J* = 9.8, 1.3 Hz, 1H), 7.68 (dd, *J* = 7.9, 1.4 Hz, 1H), 7.66 - 7.56 (m, 2H), 7.20 (d, *J* = 8.6 Hz, 1H), 7.08 (d, *J* = 7.1 Hz, 1H), 7.02 (t, *J* = 6.2 Hz, 1H), 6.96 (s, 1H), 5.06 (dd, *J* = 12.8, 5.4 Hz, 1H), 4.60 (d, *J* = 6.2 Hz, 2H), 3.58 (s, 2H), 2.88 (ddd, J = 17.1, 14.0, 5.4 Hz, 1H), 2.76 (dd, *J* = 7.6, 3.4 Hz, 3H), 2.65 - 2.55 (m, 2H), 2.13 (t, *J* = 10.4 Hz, 2H), 2.07 - 1.98 (m, 1H), 1.91 (d, *J* = 11.0 Hz, 2H), 1.73 - 1.61 (m, 2H).UPLC-MS (ESI ) calculated for $C_{30}H_{27}FN_6O_5$ [M + H ]$^+$: 571.20, found:571.72.

**Example 14: 2-(2,6-Dioxopiperidin-3-yl)-4-((((2-(1-(4-(Trifluoromethoxy) benzyl) piperidin-4-yl) oxazole-5-yl) methyl) amino) iso-dihydroindole-1,3-dione**

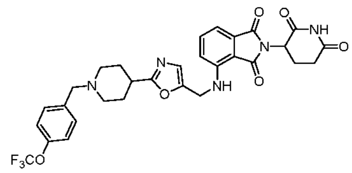

Using trifluoromethoxybenzaldehyde and intermediate 6 as starting materials, the synthetic procedure followed the same route as Example 12. A yellow solid (23 mg) was obtained with a yield of 60%.$^1$H NMR (500 MHz, DMSO) δ 11.11 (s, 1H), 7.62 - 7.56 (m, 1H), 7.42 (d, *J* = 8.5 Hz, 2H), 7.30 (d, *J* = 8.2 Hz, 2H), 7.20 (d, *J* = 8.6 Hz, 1H), 7.08 (d, *J* = 7.1 Hz, 1H), 7.01 (d, *J* = 6.5 Hz, 1H), 6.96 (s, 1H), 5.06 (dd, *J* = 12.8, 5.4 Hz, 1H), 4.60 (d, *J* = 6.1 Hz, 2H), 2.93 - 2.84 (m, 2H), 2.81 - 2.70 (m, 3H), 2.64 - 2.56 (m, 2H), 2.11 - 1.96 (m, 4H), 1.91 (d, *J* = 10.7 Hz, 2H), 1.66 (dd, *J* = 21.1, 11.1 Hz, 2H).UPLC-MS (ESI ) calculated for $C_{30}H_{28}F_3N_5O_6$ [M + H ]$^+$: 612.20, found:612.33.

**Example 15: 2-(2,6-Dioxopiperidin-3-yl)-4-((((2-(4-(Trifluoromethoxy) phenyl) oxazole-5-yl)methyl) amino) iso-indoline-1,3-dione**

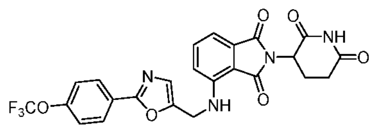

2-(4-(Trifluoromethoxy) phenyl) oxazole-5-carbaldehyde (46 mg, 0.18 mmol), racemic pomalidomide (50 mg, 0.18 mmol), phenylsilane (22 μL, 0.18 mmol), and dibutyltin dichloride (55 mg, 0.18 mmol) were added to a solution of tetrahydrofuran (6 mL) and heated to reflux overnight. The reaction was monitored by LC-Mass, and upon completion, the mixture was concentrated under reduced pressure and purified by HPLC to yield a yellow solid (12 mg), with a yield of 13%.$^1$H NMR (500 MHz, DMSO) δ 11.11 (s, 1H), 8.03 (d, *J* = 8.8 Hz, 2H), 7.62 (s, 1H), 7.52 (d, *J* = 8.3 Hz, 2H), 7.32 - 7.25 (m,

2H), 7.15 (s, 1H), 7.10 (d, *J* = 7.1 Hz, 1H), 5.07 (dd, *J* = 12.8, 5.4 Hz, 1H), 4.74 (d, *J* = 6.2 Hz, 2H), 2.89 (dd, *J* = 22.4, 8.8 Hz, 1H), 2.59 (d, *J* = 18.3 Hz, 2H), 2.07 - 1.99 (m, 1H).UPLC-MS (ESI ) calculated for $C_{24}H_{17}F_3N_4O_6$ [M + H ]$^+$: 515.11, found:515.29.

**Example 16: 2-(2,6-Dioxopiperidin-3-yl)-4-((((2-(4-Methoxyphenyl) oxazole-5-yl)methyl) amino) isoindole-1,3-dione**

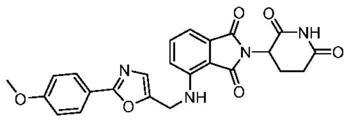

Using 2-(4-methoxyphenyl) oxazole-5-carbaldehyde and racemic pomalidomide as starting materials, the synthetic procedure followed the same route as Example 15. The product was purified by HPLC to obtain a yellow solid (8 mg) with a yield of 10%. $^1$H NMR (500 MHz, DMSO) δ 11.12 (s, 1H), 7.84 (d, *J* = 8.9 Hz, 2H), 7.66 - 7.59 (m, 1H), 7.30 (d, *J* = 8.6 Hz, 1H), 7.17 (s, 1H), 7.13 (s, 1H), 7.08 (dd, *J* = 14.6, 8.0 Hz, 3H), 5.07 (dd, *J* = 12.8, 5.4 Hz, 1H), 4.71 (d, *J* = 6.2 Hz, 2H), 3.81 (s, 3H), 2.95 - 2.84 (m, 1H), 2.58 (d, *J* = 19.4 Hz, 2H), 2.07 - 1.96 (m, 1H). UPLC-MS ( ESI ) calculated for $C_{24}H_{20}N_4O_6$ [M + H ]$^+$: 461.45, found: 461.27.

**Example 17: 2-(2,6-Dioxopiperidin-3-yl)-4-((((2-(3-Methoxyphenyl) oxazole-5-yl)methyl) amino) isoindole-1,3-dione**

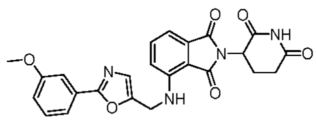

2-(3-methoxyphenyl) oxazole-5-carbaldehyde (74 mg, 0.37 mmol), racemic pomalidomide (100 mg, 0.37 mmol), phenylsilane (45 μL, 0.37 mmol), and dibutyltin dichloride (112 mg, 0.27 mmol) were added to a solution of tetrahydrofuran (6 mL) and heated to reflux overnight. The reaction was monitored by LC-Mass, and upon completion, the mixture was concentrated under reduced pressure and purified by HPLC to yield a yellow solid (40 mg), with a yield of 23%.$^1$H NMR (500 MHz, DMSO) δ 11.12 (s, 1H), 7.63 (dd, *J* = 8.4, 7.2 Hz, 1H), 7.49 (dd, *J* = 7.7, 1.1 Hz, 1H), 7.44 (d, *J* = 8.0 Hz, 1H), 7.41 - 7.39 (m, 1H), 7.31 (d, *J* = 8.6 Hz, 1H), 7.23 (s, 1H), 7.16 (t, *J* = 6.3 Hz, 1H), 7.08 (ddd, *J* = 5.9, 4.0, 3.5 Hz, 2H), 5.07 (dd, *J* = 12.8, 5.4 Hz, 1H), 4.73 (d, *J* = 6.1 Hz, 2H), 3.81 (s, 3H), 2.88 (ddd, *J* = 17.0, 13.9, 5.4 Hz, 1H), 2.61 - 2.53 (m, 2H), 2.06 - 1.99 (m, 1H). UPLC-MS (ESI ) calculated for $C_{24}H_{20}N_4O_6$ [M + H ]$^+$: 461.45, found:461.38.

**Example 18: 4-((((2-([1,1'-Biphenyl]-3-yl) oxazole-5-yl)methyl) amino)-2-(2,6-dioxopiperidin-3-yl) isoindole-1,3-dione**

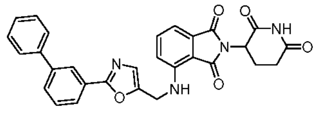

Using 2-([1,1'-biphenyl]-3-yl) oxazole-5-carbaldehyde and racemic pomalidomide as starting materials, the synthetic procedure followed the same route as Example 17. The product was purified by HPLC to obtain a yellow solid (28 mg) with a yield of 15%.$^1$H NMR (500 MHz, DMSO) δ 11.12 (s, 1H), 8.13 (t, *J* = 1.5 Hz, 1H), 7.91 (d, *J* = 7.8 Hz, 1H), 7.84 - 7.78 (m, 1H), 7.73 - 7.67 (m, 2H), 7.62 (q, *J* = 8.3 Hz, 2H), 7.51 (t, *J* = 7.7 Hz, 2H), 7.43 (d, *J* = 7.4 Hz, 1H), 7.33 (d, *J* = 8.7 Hz, 1H), 7.27 (s, 1H), 7.18 (s, 1H), 7.10 (d, *J* = 7.1 Hz, 1H), 5.07 (dd, *J* = 12.8, 5.4 Hz, 1H), 4.76 (d, *J* = 6.3 Hz, 2H), 2.88 (ddd, *J* = 17.0, 13.9, 5.4 Hz, 1H), 2.62 - 2.52 (m, 2H), 2.07 - 1.96 (m, 1H). UPLC-MS (ESI ) calculated for $C_{29}H_{22}N_4O_5$ [M + H ]$^+$: 507.52, found:507.28.

**Example 19: 2-(2,6-Dioxopiperidin-3-yl)-4-((((2-(Para-tolyl) oxazole-5-yl)methyl) amino) isoindoline-1,3-dione**

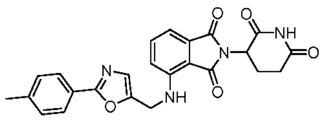

Using 2-(para-tolyl) oxazole-5-carbaldehyde and racemic pomalidomide as starting materials, the synthetic procedure followed the same route as Example 17. The product was purified by HPLC to obtain a yellow solid (42 mg) with a yield of 26%. $^{1}$H NMR (500 MHz, DMSO) δ 11.12 (s, 1H), 7.80 (d, *J* = 8.2 Hz, 2H), 7.62 (dd, *J* = 8.5, 7.2 Hz, 1H), 7.31 (dd, *J* = 12.2, 8.3 Hz, 3H), 7.20 (s, 1H), 7.14 (s, 1H), 7.09 (d, *J* = 7.1 Hz, 1H), 5.07 (dd, *J* = 12.8, 5.4 Hz, 1H), 4.72 (s, 2H), 2.88 (ddd, *J* = 17.0, 14.0, 5.4 Hz, 1H), 2.57 (dd, *J* = 25.6, 10.1 Hz, 2H), 2.35 (s, 3H), 2.06 - 1.98 (m, 1H). UPLC-MS ( ESI ) calculated for $C_{24}H_{20}N_4O_5$ [M + H ]$^+$: 445.45, found:445.33.

**Example 20: 2-(2,6-Dioxopiperidin-3-yl)-4-((((2-(Meta-tolyl) oxazole-5-yl)methyl) amino) isoindole-1,3-dione**

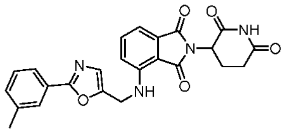

Using 2-(meta-tolyl) oxazole-5-carbaldehyde and racemic pomalidomide as starting materials, the synthetic procedure followed the same route as Example 17. The product was purified by HPLC to obtain a yellow solid (32 mg) with a yield of 20%.

**Example 21: 2-(2,6-Dioxopiperidin-3-yl)-4-((((2-(Ortho-tolyl) oxazole-5-yl)methyl) amino) isoindoline-1,3-dione**

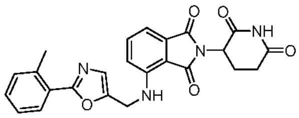

Using 2-(ortho-tolyl) oxazole-5-carbaldehyde and racemic pomalidomide as starting materials, the synthetic procedure followed the same route as Example 17. The product was purified by HPLC to obtain a yellow solid (25 mg) with a yield of 15%. $^{1}$H NMR (500 MHz, DMSO) δ 11.12 (s, 1H), 7.73 (s, 1H), 7.70 (d, *J* = 7.7 Hz, 1H), 7.65 - 7.59 (m, 1H), 7.39 (t, *J* = 7.7 Hz, 1H), 7.30 (t, *J* = 8.7 Hz, 2H), 7.22 (s, 1H), 7.15 (t, *J* = 6.2 Hz, 1H), 7.09 (d, *J* = 7.1 Hz, 1H), 5.07 (dd, *J* = 12.8, 5.4 Hz, 1H), 4.73 (d, *J* = 6.2 Hz, 2H), 2.93 - 2.82 (m, 1H), 2.58 (d, *J* = 19.8 Hz, 2H), 2.36 (s, 3H), 2.07 - 1.97 (m, 1H). UPLC-MS (ESI ) calculated for $C_{24}H_{20}N_4O_5$ [M + H ]$^+$: 445.45, found:445.22.

**Example 22: 2-(2,6-Dioxopiperidin-3-yl)-4-((((2-(Phenyl) oxazole-5-yl)methyl) amino) isoindole-1,3-dione**

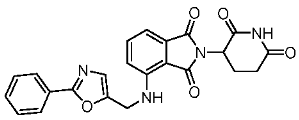

Using 2-phenyl oxazole-5-carbaldehyde and racemic pomalidomide as starting materials, the synthetic procedure followed the same route as Example 15. The product was purified by HPLC to obtain a yellow solid (31 mg) with a yield of 20%. $^{1}$H NMR (500 MHz, DMSO) δ 11.12 (s, 1H), 7.94 - 7.89 (m, 2H), 7.63 (dd, *J* = 8.4, 7.3 Hz, 1H), 7.55 - 7.48 (m, 3H), 7.30 (d, *J* = 8.6 Hz, 1H), 7.24 (s, 1H), 7.15 (t, *J* = 6.3 Hz, 1H), 7.10 (d, *J* = 7.1 Hz, 1H), 5.07 (dd, *J* = 12.8, 5.4 Hz, 1H), 4.74 (d, *J*

= 6.2 Hz, 2H), 2.88 (ddd, *J* = 17.0, 13.9, 5.4 Hz, 1H), 2.65 - 2.52 (m, 2H), 2.03 (ddd, *J* = 10.5, 5.4, 3.1 Hz, 1H). UPLC-MS (ESI ) calculated for $C_{23}H_{18}N_4O_5$ [M + H ]$^+$: 431.42, found: 431.28.

**Example 23: 4-((((2-(4-Bromophenyl) oxazole-5-yl)methyl) amino)-2-(2,6-dioxopiperidin-3-yl) isoindolin-1,3-dione**

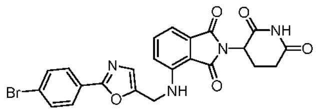

Using 2-(4-bromophenyl) oxazole-5-carbaldehyde and racemic pomalidomide as starting materials, the synthetic procedure followed the same route as Example 15. The product was purified by HPLC to obtain a yellow solid (21 mg) with a yield of 11%. $^1$H NMR (400 MHz, DMSO) δ 11.10 (s, 1H), 7.84 (d, *J* = 8.4 Hz, 2H), 7.73 (d, *J* = 8.5 Hz, 2H), 7.66 - 7.59 (m, 1H), 7.33 - 7.23 (m, 2H), 7.18 - 7.06 (m, 2H), 5.07 (dd, *J* = 12.8, 5.3 Hz, 1H), 4.73 (d, *J* = 4.7 Hz, 2H), 2.94 - 2.79 (m, 1H), 2.69 - 2.55 (m, 2H), 2.08 - 1.97 (m, 1H). UPLC-MS (ESI ) calculated for $C_{23}H_{17}BrN_4O_5$ [M + H ]$^+$: 510.32, found: 510.07.

**Example 24: 2-(2,6-Dioxopiperidin-3-yl)-4-((((5-(Para-tolyl) oxazole-2-yl)methyl) amino) isoindolin-1,3-dione**

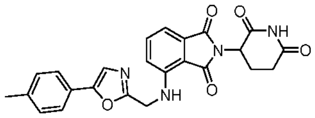

Using 5-(para-tolyl) oxazole-2-carbaldehyde (55 mg, 0.294 mmol) and pomalidomide (80 mg, 0.294 mmol) as starting materials, the synthetic procedure followed the same route as Example 15. The product was purified by HPLC to obtain a yellow solid (14 mg) with a yield of 11%.$^1$H NMR (500 MHz, DMSO) δ 11.13 (s, 1H), 7.61 (dd, *J* = 8.3, 7.3 Hz, 1H), 7.55 (t, *J* = 4.1 Hz, 3H), 7.30 - 7.20 (m, 4H), 7.10 (d, *J* = 7.1 Hz, 1H), 5.09 (dd, *J* = 12.7, 5.5 Hz, 1H), 4.78 (d, *J* = 6.2 Hz, 2H), 2.95 - 2.82 (m, 1H), 2.66 - 2.53 (m, 2H), 2.32 (s, 3H), 2.08 - 2.01 (m, 1H). UPLC-MS (ESI ) calculated for $C_{24}H_{20}N_4O_5$ [M + H ]$^+$: 445.45, found:445.22.

**Example 25: 4-((((2-(4-Chlorophenyl) oxazole-5-yl)methyl) amino)-2-(2,6-dioxopiperidin-3-yl) isoindole-1,3-dione**

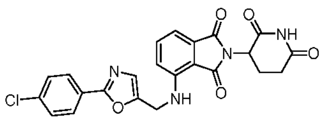

Using 2-(4-chlorophenyl) oxazole-5-carbaldehyde and racemic pomalidomide as starting materials, the synthetic procedure followed the same route as Example 17. The product was purified by HPLC to obtain a yellow solid (93 mg) with a yield of 55%.$^1$H NMR (500 MHz, DMSO) δ 11.14 (s, 1H), 7.96 - 7.87 (m, 2H), 7.67 - 7.59 (m, 3H), 7.31 (d, *J* = 8.6 Hz, 1H), 7.27 (s, 1H), 7.17 (t, *J* = 6.2 Hz, 1H), 7.11 (d, *J* = 7.1 Hz, 1H), 5.08 (dd, *J* = 12.8, 5.5 Hz, 1H), 4.75 (d, *J* = 6.3 Hz, 2H), 2.90 (ddd, *J* = 16.9, 14.0, 5.4 Hz, 1H), 2.68 - 2.57 (m, 2H), 2.04 (dd, *J* = 10.7, 5.7 Hz, 1H). UPLC-MS (ESI ) calculated for $C_{23}H_{17}ClN_4O_5$ [M + H ]$^+$: 465.86, found:465.37.

**Example 26: 2-(2,6-Dioxopiperidin-3-yl)-4-((((2-(4-Fluorophenyl) oxazole-5-yl)methyl) amino) isoindole-1,3-dione**

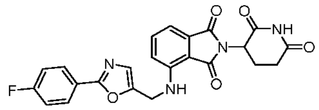

Using 2-(4-fluorophenyl) oxazole-5-carbaldehyde and racemic pomalidomide as starting materials, the synthetic procedure followed the same route as Example 17. The product was purified by HPLC to obtain a yellow solid (92 mg) with a yield of 56%.$^1$H NMR (500 MHz, DMSO) δ 11.12 (s, 1H), 8.00 - 7.91 (m, 2H), 7.62 (dd, *J* = 8.4, 7.2 Hz, 1H), 7.41 - 7.33 (m, 2H), 7.30 (d, *J* = 8.6 Hz, 1H), 7.23 (s, 1H), 7.15 (t, *J* = 6.4 Hz, 1H), 7.10 (d, *J* = 7.0 Hz, 1H), 5.07 (dd, *J* = 12.8, 5.4 Hz, 1H), 4.73 (d, *J* = 6.3 Hz, 2H), 2.88 (ddd, *J* = 17.0, 13.9, 5.4 Hz, 1H), 2.60 (dd, *J* = 17.5, 14.6 Hz, 2H), 2.06 - 1.98 (m, 1H).UPLC-MS (ESI) calculated for $C_{23}H_{17}FN_4O_5$ [M + H ]$^+$:449.41, found:449.54.

**Example 27: 4-((2-(2-Chlorophenyl)-5-oxazolyl)methyl)amino)-2-(2,6-Dioxopiperidin-3-yl)isoindolin-1,3-dione**

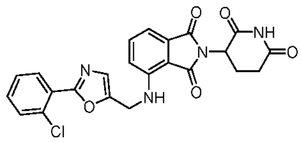

2-(2-Chlorophenyl)-5-oxazole-2-carbaldehyde and racemic pomalidomide were used as raw materials. The synthetic route followed that of Example 17. After purification by HPLC, 144 mg of the yellow product was obtained, yielding 86%.$^1$H NMR (500 MHz, DMSO) δ 11.10 (s, 1H), 7.92 (dd, *J* = 7.6, 1.9 Hz, 1H), 7.64 - 7.59 (m, 2H), 7.50 (dtd, *J* = 18.4, 7.4, 1.6 Hz, 2H), 7.31 (t, *J* = 4.3 Hz, 2H), 7.14 (d, *J* = 6.4 Hz, 1H), 7.10 (d, *J* = 7.0 Hz, 1H), 5.07 (dd, *J* = 12.8, 5.4 Hz, 1H), 4.76 (d, *J* = 6.2 Hz, 2H), 2.88 (ddd, *J* = 17.0, 14.0, 5.4 Hz, 1H), 2.65 - 2.54 (m, 2H), 2.06 - 1.96 (m, 1H). UPLC-MS (ESI ) calculated for $C_{23}H_{17}ClN_4O_5$ [M + H ]$^+$: 465.86, found:465.30.

**Example 28: 2-(2,6-Dioxopiperidin-3-yl)-4-((2-(3-Fluorophenyl)-5-thiazolyl)methyl)amino)-isoindolin-1,3-dione**

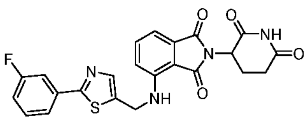

45 mg (0.22 mmol) of 2-(3-Fluorophenyl)-5-thiazole-2-carbaldehyde and 59 mg (0.22 mmol) of racemic pomalidomide were suspended in 4 mL of acetic acid and heated under reflux overnight. The reaction was cooled to room temperature, and sodium borohydride (9 mg, 0.22 mmol) was added. After a 2-hour reaction at room temperature, the mixture was concentrated under reduced pressure and diluted with ethyl acetate. The organic phase was washed successively with saturated sodium bicarbonate solution and saturated sodium chloride solution, then dried and concentrated. The product was purified by HPLC to yield 5.5 mg of yellow product, with a yield of 15.4%.$^1$H NMR (500 MHz, DMSO) δ 11.13 (s, 1H), 7.94 (s, 1H), 7.74 - 7.66 (m, 2H), 7.61 - 7.55 (m, 1H), 7.51 (dt, *J* = 14.0, 7.0 Hz, 1H), 7.38 (t, *J* = 6.3 Hz, 1H), 7.30 (td, *J* = 8.4, 2.1 Hz, 1H), 7.21 (d, *J* = 8.6 Hz, 1H), 7.07 (d, *J* = 7.1 Hz, 1H), 5.07 (dd, *J* = 12.8, 5.4 Hz, 1H), 4.85 (d, *J* = 6.3 Hz, 2H), 2.93 - 2.84 (m, 1H), 2.65 - 2.55 (m, 2H), 2.03 (ddd, *J* = 7.5, 6.8, 3.9 Hz, 1H). UPLC-MS ( ESI ) calculated for $C_{23}H_{17}FN_4O_4S$ [M + H ]$^+$: 465.47, found:465.19.

**Example 29: 2-(2,6-Dioxopiperidin-3-yl)-4-((2-(para-Tolyl)-5-thiazolyl)methyl)amino)-isoindolin-1,3-dione**

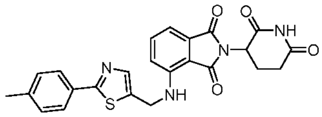

80 mg (0.39 mmol) of 2-(para-Tolyl)-5-thiazole-2-carbaldehyde and 108 mg (0.39 mmol) of racemic pomalidomide were used as raw materials. The synthetic route followed that of Example 28. After purification by HPLC, 25.4 mg of the yellow product was obtained, yielding 14%.$^1$H NMR (500 MHz, DMSO) δ 11.13 (s, 1H), 7.86 (s, 1H), 7.77 (d, *J* = 8.2 Hz, 2H), 7.58 (dd, *J* = 8.5, 7.2 Hz, 1H), 7.33 (t, *J* = 6.2 Hz, 1H), 7.27 (d, *J* = 8.0 Hz, 2H), 7.22 (d, *J* = 8.6 Hz, 1H), 7.07 (d, *J* = 7.1 Hz, 1H), 5.07 (dd, *J* = 12.8, 5.4 Hz, 1H), 4.82 (d, *J* = 6.1 Hz, 2H), 2.89 (ddd, *J* = 16.9, 13.9, 5.5 Hz, 1H), 2.59 (dd, *J* = 34.3, 14.6 Hz, 2H), 2.33 (s, 3H), 2.07 - 2.00 (m, 1H). UPLC-MS (ESI ) calculated for $C_{24}H_{20}N_4O_4S$ [M + H ]$^+$: 461.51, 实测值: 461.23.

**Example 30: 3-(5-((2-(2,6-Dioxopiperidin-3-yl)-1,3-dioxoisoindolin-4-yl)amino)methyl)-thiazol-2-yl)-benzonitrile**

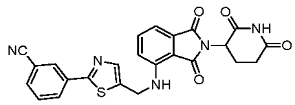

62 mg (0.29 mmol) of 3-(5-formylthiazol-2-yl)benzonitrile and 80 mg (0.29 mmol) of racemic pomalidomide were used as raw materials. The synthetic route followed that of Example 28. After purification by HPLC, 9 mg of the yellow product was obtained, yielding 7%.$^1$H NMR (500 MHz, DMSO) δ 11.13 (s, 1H), 8.31 (s, 1H), 8.21 (d, *J* = 8.4 Hz, 1H), 7.98 (s, 1H), 7.91 (d, *J* = 7.8 Hz, 1H), 7.67 (t, *J* = 7.9 Hz, 1H), 7.62 - 7.55 (m, 1H), 7.40 (t, *J* = 6.4 Hz, 1H), 7.22 (d, *J* = 8.6 Hz, 1H), 7.08 (d, *J* = 7.1 Hz, 1H), 5.07 (dd, *J* = 12.8, 5.4 Hz, 1H), 4.87 (d, *J* = 6.1 Hz, 2H), 2.94 - 2.83 (m, 1H), 2.65 - 2.55 (m, 2H), 2.07 - 1.99 (m, 1H).UPLC-MS (ESI ) calculated for $C_{24}H_{17}N_5O_4S$ [M + H ]$^+$: 472.49, found:472.20.

**Example 31: 2-(2,6-Dioxopiperidin-3-yl)-4-((2-(4-Vinylphenyl)-5-thiazolyl)methyl)amino)-isoindolin-1,3-dione**

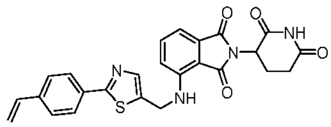

63 mg (0.29 mmol) of 2-(4-Vinylphenyl)-5-thiazole-2-carbaldehyde and 80 mg (0.29 mmol) of racemic pomalidomide were used as raw materials. The synthetic route followed that of Example 28. After purification by HPLC, 10 mg of the yellow product was obtained, yielding 7%.$^1$H NMR (500 MHz, DMSO) δ 11.13 (s, 1H), 7.90 (s, 1H), 7.86 (d, *J* = 8.3 Hz, 2H), 7.57 (t, *J* = 8.2 Hz, 3H), 7.35 (t, *J* = 6.3 Hz, 1H), 7.22 (d, *J* = 8.6 Hz, 1H), 7.07 (d, *J* = 7.1 Hz, 1H), 6.77 (dd, *J* = 17.6, 11.0 Hz, 1H), 5.92 (d, *J* = 17.7 Hz, 1H), 5.34 (d, *J* = 11.2 Hz, 1H), 5.07 (dd, *J* = 12.8, 5.4 Hz, 1H), 4.84 (d, *J* = 6.3 Hz, 2H), 2.96 - 2.80 (m, 1H), 2.65 - 2.53 (m, 2H), 2.10 - 2.00 (m, 1H). UPLC-MS ( ESI ) calculated for $C_{25}H_{20}N_4O_4S$ [M + H ]$^+$: 473.52, found:473.19.

**Example 32: 4-((2-(4-Chlorophenyl)-5-thiazolyl)methyl)amino)-2-(2,6-Dioxopiperidin-3-yl)isoindolin-1,3-dione**

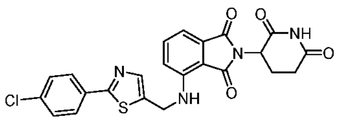

66 mg (0.29 mmol) of 2-(4-Chlorophenyl)-5-thiazole-2-carbaldehyde and 80 mg (0.29 mmol) of racemic pomalidomide were used as raw materials. The synthetic route followed that of Example 28. After purification by HPLC, 12 mg of the yellow product was obtained, yielding 9%.$^1$H NMR (500 MHz, DMSO) δ 11.13 (s, 1H), 7.96 - 7.85 (m, 3H), 7.58 (dd, *J* = 8.5, 7.2 Hz, 1H), 7.54 - 7.49 (m, 2H), 7.36 (t, *J* = 6.1 Hz, 1H), 7.21 (d, *J* = 8.6 Hz, 1H), 7.07 (d, *J* = 7.1 Hz, 1H), 5.07 (dd, *J* = 12.8, 5.4 Hz, 1H), 4.84 (d, *J* = 5.6 Hz, 2H), 2.89 (ddd, *J* = 17.1, 13.9, 5.4 Hz, 1H), 2.65 - 2.52 (m, 2H), 2.07 - 1.99 (m, 1H). UPLC-MS (ESI ) calculated for $C_{23}H_{17}ClN_4O_4S$ [M + H ]$^+$:481.92, found:481.13.

**Example 33: 4-(5-(((2-(2,6-dioxopiperidin-3-yl)-1,3-dioxoisoindolin-4-yl)amino)methyl)thiazol-2-yl)benzonitrile**

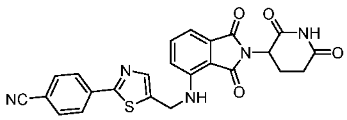

70 mg (0.33 mmol) of 4-(5-formylthiazol-2-yl)benzonitrile and 90 mg (0.33 mmol) of racemic pomalidomide were used as raw materials. The synthetic route followed that of Example 28. After purification by HPLC, 28 mg of the yellow product was obtained, yielding 18%.$^1$H NMR (400 MHz, DMSO) δ 11.11 (s, 1H), 8.07 (d, *J* = 8.3 Hz, 2H), 8.01 (s, 1H), 7.92 (d, *J* = 8.3 Hz, 2H), 7.62 - 7.55 (m, 1H), 7.39 (t, *J* = 6.2 Hz, 1H), 7.22 (d, *J* = 8.6 Hz, 1H), 7.08 (d, *J* = 7.1 Hz, 1H), 5.07 (dd, *J* = 12.9, 5.3 Hz, 1H), 4.87 (d, *J* = 6.2 Hz, 2H), 2.95 - 2.83 (m, 1H), 2.70 - 2.57 (m, 2H), 2.09 - 1.98 (m, 1H). UPLC-MS (ESI ) calculated for $C_{24}H_{17}N_5O_4S$ [M + H ]$^+$:472.49, found:472.19.

**Example 34: 2-(2,6-Dioxopiperidin-3-yl)-4-((5-(4-Fluorophenyl)-thiazol-2-yl)methyl)amino)-isoindolin-1,3-dione**

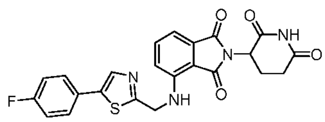

72 mg (0.348 mmol) of 5-(4-Fluorophenyl)-5-thiazole-2-carbaldehyde and 80 mg (0.29 mmol) of racemic pomalidomide were used as raw materials. The synthetic route followed that of Example 28. After purification by HPLC, 10.5 mg of the yellow product was obtained, yielding 8%.$^1$H NMR (500 MHz, DMSO) δ 11.14 (s, 1H), 8.12 (s, 1H), 7.68 (dd, *J* = 8.6, 5.3 Hz, 2H), 7.56 (dt, *J* = 12.1, 7.0 Hz, 2H), 7.25 (t, *J* = 8.8 Hz, 2H), 7.10 (dd, *J* = 7.5, 5.6 Hz, 2H), 5.10 (dd, *J* = 12.7, 5.3 Hz, 1H), 4.88 (d, *J* = 6.1 Hz, 2H), 2.94 - 2.86 (m, 1H), 2.60 (dd, *J* = 28.6, 10.3 Hz, 2H), 2.10 - 2.02 (m, 1H). UPLC-MS ( ESI ) calculated for $C_{23}H_{17}FN_4O_4S$ [M + H ]$^+$: 465.47, found:465.22.

**Example 35: 2-(2,6-Dioxopiperidin-3-yl)-4-((2-(4-(Trifluoromethoxy)-phenyl)-5-thiazolyl)methyl)amino)-isoindolin-1,3-dione**

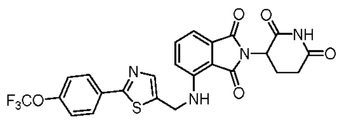

80 mg (0.29 mmol) of 2-(4-(Trifluoromethoxy)-phenyl)-5-thiazole-2-carbaldehyde and 80 mg (0.29 mmol) of racemic pomalidomide were used as raw materials. The synthetic route followed that of Example 28. After purification by HPLC, 20 mg of the yellow product was obtained, yielding 13%.$^1$H NMR (500 MHz, DMSO) δ 11.13 (s, 1H), 8.01 (dd, *J* = 8.7, 1.8 Hz, 2H), 7.94 (s, 1H), 7.59 (t, *J* = 7.8 Hz, 1H), 7.46 (d, *J* = 8.0 Hz, 2H), 7.37 (d, *J* = 5.8 Hz, 1H), 7.22 (d, *J* = 8.6 Hz, 1H), 7.07 (d, *J* = 7.1 Hz, 1H), 5.07 (dd, *J* = 12.3, 4.8 Hz, 1H), 4.85 (d, *J* = 6.0 Hz, 2H), 2.94 - 2.83 (m, 1H), 2.57 (d, *J* = 35.0 Hz, 2H), 2.09 - 1.98 (m, 1H). UPLC-MS ( ESI ) calculated for $C_{24}H_{17}F_3N_4O_5S$ [M + H ]$^+$:531.48, found:531.19.

**Example 36: 4-((2-(2,4-Dimethoxyphenyl)-5-thiazolyl)methyl)amino)-2-(2,6-Dioxopiperidin-3-yl)isoindolin-1,3-dione**

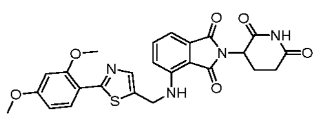

72 mg (0.29 mmol) of 2-(2,4-Dimethoxyphenyl)-5-thiazole-2-carbaldehyde and 80 mg (0.29 mmol) of racemic pomalidomide were used as raw materials. The synthetic route followed that of Example 28. After purification by HPLC, 19 mg of the yellow product was obtained, yielding 13%.$^1$H NMR (500 MHz, DMSO) δ 11.12 (s, 1H), 8.14 (d, *J* = 8.8 Hz, 1H), 7.83 (s, 1H), 7.60 - 7.55 (m, 1H), 7.26 (t, *J* = 5.7 Hz, 1H), 7.21 (d, *J* = 8.6 Hz, 1H), 7.07 (t, *J* = 7.1 Hz, 1H), 6.74 (d, *J* = 2.3 Hz,

1H), 6.68 (dd, *J* = 8.8, 2.4 Hz, 1H), 5.07 (dd, *J* = 12.8, 5.4 Hz, 1H), 4.80 (d, *J* = 5.0 Hz, 2H), 3.96 (s, 3H), 3.83 (s, 3H), 2.88 (ddd, *J* = 16.9, 13.9, 5.3 Hz, 1H), 2.58 (dd, *J* = 21.8, 4.3 Hz, 2H), 2.07 - 1.95 (m, 1H). UPLC-MS (ESI ) calculated for $C_{25}H_{22}N_4O_6S$ [M + H ]$^+$: 507.53, found:507.61.

**Example 37: 2-(2,6-Dioxopiperidin-3-yl)-4-((2-(4-(Trifluoromethyl)-phenyl)-5-thiazolyl)methyl)amino)-isoindolin-1,3-dione**

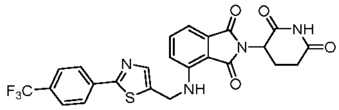

75 mg (0.29 mmol) of 2-(4-(Trifluoromethyl)-phenyl)-5-thiazole-2-carbaldehyde and 80 mg (0.29 mmol) of pomalidomide were used as raw materials. The synthetic route followed that of Example 28. After purification by HPLC, 6 mg of the yellow product was obtained, yielding 4%.$^1$H NMR (500 MHz, DMSO) δ 11.11 (s, 1H), 8.10 (d, *J* = 8.2 Hz, 2H), 8.00 (s, 1H), 7.82 (d, *J* = 8.4 Hz, 2H), 7.63 - 7.56 (m, 1H), 7.39 (t, *J* = 6.3 Hz, 1H), 7.23 (d, *J* = 8.6 Hz, 1H), 7.08 (d, *J* = 7.1 Hz, 1H), 5.07 (dd, *J* = 12.8, 5.3 Hz, 1H), 4.87 (d, *J* = 6.3 Hz, 2H), 2.94 - 2.82 (m, 1H), 2.59 (d, *J* = 20.0 Hz, 2H), 2.10 - 1.96 (m, 1H). UPLC-MS (ESI ) calculated for $C_{24}H_{17}F_3N_4O_4S$ [M + H ]$^+$:515.48, found:515.11.

**Example 38: 2-(2,6-Dioxopiperidin-3-yl)-4-((5-(Phenyl-d5)-1,3,4-thiadiazol-2-yl)methyl)amino)-isoindolin-1,3-dione**

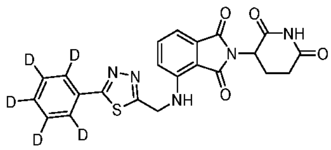

The synthetic route followed that of Example 32 to obtain the compound of Example 38.

**Example 39: (3-(1,3-Dioxy-4-((2-(4-(Trifluoromethyl)-phenyl)-5-thiazolyl)methyl)amino)-isoindol-2-yl)-2,6-Dioxopiperidin-1-yl)Neopentyl Methyl Ester**

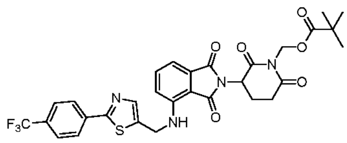

The compound from Example 37, 2-(2,6-Dioxopiperidin-3-yl)-4-((2-(4-(Trifluoromethyl)-phenyl)-5-thiazolyl) methyl)amino)-isoindolin-1,3-dione (70 mg, 0.11 mmol) was reacted with 16 μL (0.11 mmol) of neopentyl chloride and 43 mg (0.13 mmol) of cesium carbonate in 2 mL of N,N-dimethylformamide at room temperature. After one hour of heating at 50 °C and TLC monitoring of the reaction, the reaction mixture was cooled, quenched with water, and extracted with ethyl acetate. The organic phase was washed with saturated sodium chloride solution, dried, filtered, and concentrated. The crude product was purified by HPLC to yield 7.6 mg of the yellow solid, with a yield of 11%.$^1$H NMR (500 MHz, DMSO) δ 8.10 (d, J = 8.2 Hz, 2H), 7.99 (s, 1H), 7.82 (d, J = 8.4 Hz, 2H), 7.60 (dd, J = 8.5, 7.2 Hz, 1H), 7.36 (t, J = 6.3 Hz, 1H), 7.24 (d, J = 8.6 Hz, 1H), 7.09 (d, J = 7.1 Hz, 1H), 5.65 (s, 2H), 5.26 (dd, J = 13.0, 5.4 Hz, 1H), 4.88 (d, J = 6.2 Hz, 2H), 3.07 (ddd, J = 17.4, 14.0, 5.4 Hz, 1H), 2.87 - 2.80 (m, 1H), 2.65 - 2.51 (m, 1H), 2.11 (tdd, J = 7.6, 5.0, 2.6 Hz, 1H). $^{13}$C NMR (126 MHz, DMSO) δ 176.44, 171.12, 169.37, 168.42, 167.07, 164.71, 145.34, 142.47, 139.59, 136.59, 136.30, 132.25, 129.69, 126.65, 126.19, 126.17, 117.72, 111.41, 110.06, 63.18, 49.05, 38.46, 38.22, 31.05, 26.62, 21.07. UPLC-MS ( ESI ) calculated for $C_{30}H_{27}F_3N_4O_6S$ [M + H ]$^+$: 629.62, found:629.28.

**Example 40: (3-(4-(((2-(4-Chlorophenyl)-5-oxazolyl)methyl)amino)-1,3-dioxoisoindol-2-yl)-2,6-dioxopiperidin-1-yl)methyl Neopentyl Ester**

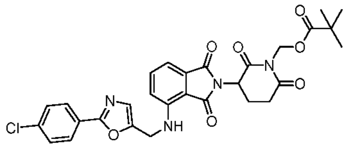

The synthetic route of Example 25 and Example 39 was used to obtain the compound of Example 40 as a yellow solid (50 mg, 26.7% yield). $^1$H NMR (500 MHz, DMSO) δ 7.91 (d, J = 8.6 Hz, 2H), 7.63 (dd, J = 8.4, 7.3 Hz, 1H), 7.61 - 7.57 (m, 2H), 7.30 (d, J = 8.6 Hz, 1H), 7.26 (s, 1H), 7.13 (d, J = 5.7 Hz, 1H), 7.11 (d, J = 7.1 Hz, 1H), 5.64 (s, 2H), 5.26 (dd, J = 13.0, 5.4 Hz, 1H), 4.74 (d, J = 5.8 Hz, 2H), 3.06 (ddd, J = 17.5, 14.0, 5.3 Hz, 1H), 2.83 (dd, J = 13.6, 2.8 Hz, 1H), 2.56 (dd, J = 13.4, 4.3 Hz, 1H), 2.12 - 2.04 (m, 1H), 1.09 (s, 9H). UPLC-MS ( ESI ) calculated for $C_{29}H_{27}ClN_4O_7$ [M + H ]$^+$: 579.16, found:579.36.

**Example 41: (3-(1,3-Dioxy-4-((2-(1-Propyvylpiperidin-4-yl)-5-oxazolyl)methyl)amino)-isoindol-2-yl)-2,6-Dioxopiperidin-1-yl)Propyl Methyl Ester**

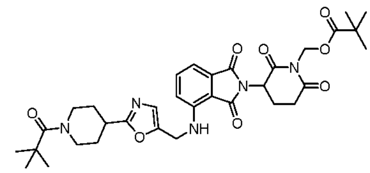

The synthetic route of Example 38 and Example 39 was followed to obtain the compound of Example 41. A yellow solid (56.9 mg) was obtained with a yield of 38%. $^1$H NMR (500 MHz, DMSO) δ 7.60 (dd, J = 8.5, 7.2 Hz, 1H), 7.22 (d, J = 8.6 Hz, 1H), 7.09 (d, J = 7.1 Hz, 1H), 7.02 (s, 1H), 6.98 (s, 1H), 5.64 (s, 2H), 5.26 (dd, J = 13.0, 5.4 Hz, 1H), 4.61 (d, J = 3.5 Hz, 2H), 4.17 (d, J = 13.5 Hz, 2H), 3.06 (ddd, J = 22.2, 12.9, 6.0 Hz, 2H), 2.99 (dd, J = 13.1, 10.8 Hz, 2H), 2.83 (dd, J = 13.6, 2.8 Hz, 1H), 2.62 - 2.54 (m, 1H), 2.09 (ddd, J = 14.9, 7.4, 4.9 Hz, 1H), 1.95 (dd, J = 13.4, 3.0 Hz, 2H), 1.55 - 1.46 (m, 2H), 1.17 (s, 9H), 1.10 (s, 9H). $^{13}$C NMR (201 MHz, DMSO) δ 176.45, 174.84, 171.11, 169.36, 168.53, 167.08, 165.57, 148.77, 145.69, 136.20, 132.11, 123.98, 117.74, 111.36, 109.90, 63.17, 49.05, 43.80, 40.43, 39.99, 38.23, 38.12, 36.82, 34.56, 31.04, 29.62, 28.07, 26.62, 21.08. UPLC-MS ( ESI ) calculated for $C_{33}H_{41}N_5O_8$ [M + H ]$^+$: 636.72, found:636.36.

**Example 42: 2-(2,6-Dioxopiperidin-3-yl)-4-((2-(4-(4-Fluorobenzyl)-cyclohexyl)-5-oxazolyl)methyl)amino)-isoindolin-1,3-dione**

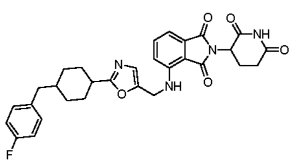

The synthetic route of Example 12 was followed to obtain the compound of Example 42. It was a mixture of the compounds from Example 105 and Example 106 before separation, with NMR and mass spectrometry data corresponding to the respective compounds.

**Example 43: 4-((2-(5-Chloropyridin-2-yl)-5-thiazolyl)methyl)amino)-2-(2,6-Dioxopyridin-3-yl)isoindolin-1,3-dione**

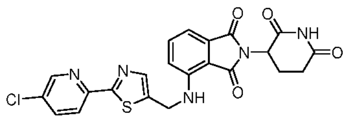

The synthetic route of Example 32 was followed to obtain the compound of Example 43, which was purified by HPLC to yield 58 mg, with a yield of 33%.$^1$H NMR (600 MHz, DMSO) δ 11.10 (s, 1H), 8.65 (d, J = 0.9 Hz, 1H), 8.09 - 8.02 (m, 2H), 7.97 (s, 1H), 7.59 (t, J = 7.8 Hz, 1H), 7.35 (t, J = 6.3 Hz, 1H), 7.22 (d, J = 8.6 Hz, 1H), 7.07 (d, J = 7.1 Hz, 1H), 5.07 (dd, J = 12.8, 5.4 Hz, 1H), 4.85 (d, J = 6.4 Hz, 2H), 2.93 - 2.84 (m, 1H), 2.62 - 2.51 (m, 2H), 2.08 - 2.00 (m, 1H). UPLC-MS ( ESI ) calculated for $C_{22}H_{16}ClN_5O_4S$ [M + H ]$^+$: 482.06, found:482.25.

**Example 44: 4-((2-(6-Chloropyridin-3-yl)-5-oxazolyl)methyl)amino)-2-(2,6-Dioxopiperidin-3-yl)isoindolin-1,3-dione**

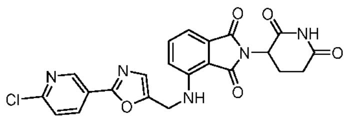

The synthetic route of Example 25 was followed to obtain the compound of Example 44. A yellow solid (49.3 mg) was obtained, with a yield of 53%.$^1$H NMR (500 MHz, DMSO) δ 11.10 (s, 1H), 8.91 (d, *J* = 2.0 Hz, 1H), 8.29 (dd, *J* = 8.4, 2.5 Hz, 1H), 7.69 (d, *J* = 8.4 Hz, 1H), 7.62 (dd, *J* = 8.4, 7.2 Hz, 1H), 7.32 (s, 1H), 7.29 (d, *J* = 8.6 Hz, 1H), 7.16 (t, *J* = 6.3 Hz, 1H), 7.10 (d, *J* = 7.1 Hz, 1H), 5.07 (dd, *J* = 12.8, 5.4 Hz, 1H), 4.75 (d, *J* = 6.2 Hz, 2H), 2.88 (ddd, *J* = 17.0, 13.9, 5.4 Hz, 1H), 2.65 - 2.52 (m, 2H), 2.03 (ddd, *J* = 10.5, 5.4, 3.1 Hz, 1H). $^{13}$C NMR (126 MHz, DMSO) δ 172.82, 170.07, 168.64, 167.22, 157.37, 151.58, 150.94, 146.91, 145.52, 136.60, 136.19, 132.21, 126.17, 124.97, 122.54, 117.72, 111.41, 110.16, 48.59, 36.94, 30.97, 22.12. UPLC-MS ( ESI ) calculated for $C_{22}H_{16}ClN_5O_5$ [M + H ]$^+$: 466.85, found:466.23.

**Example 45: 2-(2,6-Dioxopiperidin-3-yl)-4-((2-(Phenyl-d5)-5-oxazolyl)methyl)amino)isoindolin-1,3-dione**

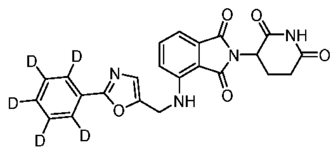

Following the synthetic route described in Example 22, the compound of Example 45 was obtained as a yellow solid (110 mg, 69% yield). $^1$H NMR (400 MHz, DMSO) δ 11.10 (s, 1H), 7.63 (s, 1H), 7.30 (d, J = 7.4 Hz, 1H), 7.23 (s, 1H), 7.10 (s, 2H), 5.07 (d, J = 8.0 Hz, 1H), 4.74 (s, 2H), 2.86 (d, J = 12.4 Hz, 1H), 2.59 (d, J = 17.8 Hz, 2H), 2.04 (s, 1H). UPLC-MS (ESI) calculated for $C_{23}H_{13}D_5N_4O_5$ [M + H ]$^+$: 436.16, found:436.28.

**Example 46: 2-(2,6-Dioxopiperidin-3-yl)-4-((2-(Phenyl-d5)-5-thiazolyl)methyl)amino)isoindolin-1,3-dione**

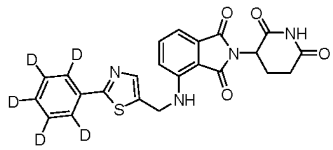

Following the synthetic route described in Example 32, the compound of Example 46 was obtained using 2-(Phenyl-d5)-5-thiazolylmethanal (134 mg, 0.69 mmol) and racemic pomalidomide (188 mg, 0.69 mmol) as starting materials. The compound was purified by HPLC to yield a yellow solid (88 mg, 28% yield). $^1$H NMR (600 MHz, DMSO) δ 11.11 (s, 1H), 7.90 (s, 1H), 7.59 (t, J = 7.8 Hz, 1H), 7.33 (t, J = 6.2 Hz, 1H), 7.22 (d, J = 8.6 Hz, 1H), 7.07 (d, J = 7.1 Hz, 1H), 5.07 (dd, J = 12.9, 5.4 Hz, 1H), 4.84 (d, J = 6.2 Hz, 2H), 2.89 (ddd, J = 17.3, 14.1, 5.3 Hz, 1H), 2.62 - 2.51 (m, 2H), 2.07 - 2.01

(m, 1H). UPLC-MS (ESI) calculated for $C_{23}H_{13}D_5N_4O_4S$ [M + H ]$^+$: 452.14, found:452.31.

**Example 47: 2-(2,6-Dioxopiperidin-3-yl)-4-(5-(Phenyl-d5)-1,3,4-oxadiazol-2-yl)methyl)amino)isoindolin-1,3-dione**

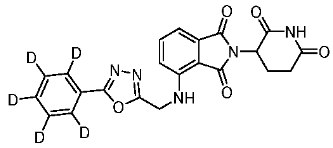

Following the synthetic route described in Example 32, the compound of Example 47 was obtained.

**Example 48: 2-(2,6-Dioxopiperidin-3-yl)-4-(((2-(4-((S)-Tetrahydrofuran-3-yl)oxy)phenyl)oxazol-5-yl)methyl)amino)iso-2,3-dihydroindolin-1,3-dione**

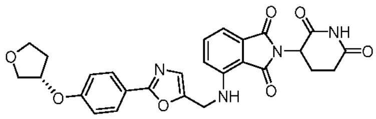

Following the synthetic route described in Example 22, the compound of Example 48 was obtained. A yellow solid (65.3 mg, 55% yield) was isolated. $^1$H NMR (500 MHz, DMSO) δ 11.08 (s, 1H), 7.83 (d, J = 8.9 Hz, 2H), 7.65 - 7.60 (m, 1H), 7.30 (d, J = 8.6 Hz, 1H), 7.17 (s, 1H), 7.10 (t, J = 6.5 Hz, 2H), 7.05 (d, J = 8.9 Hz, 2H), 5.12 - 5.08 (m, 1H), 5.06 (dd, J = 12.9, 5.5 Hz, 1H), 4.71 (d, J = 6.3 Hz, 2H), 3.90 (dd, J = 10.2, 4.5 Hz, 1H), 3.84 (dd, J = 15.4, 8.0 Hz, 1H), 3.79 (d, J = 10.3 Hz, 1H), 3.78 - 3.72 (m, 1H), 2.93 - 2.83 (m, 1H), 2.58 (dt, J = 18.0, 9.6 Hz, 2H), 2.25 (td, J = 14.3, 8.1 Hz, 1H), 2.06 - 2.00 (m, 1H), 1.99 - 1.94 (m, 1H). $^{13}$C NMR (126 MHz, DMSO) δ 172.77, 170.02, 168.66, 167.20, 160.35, 158.89, 149.12, 145.61, 136.11, 132.18, 127.48, 125.53, 119.65, 117.72, 115.78, 111.30, 110.05, 77.40, 72.20, 66.39, 48.57, 36.90, 32.40, 30.95, 22.09. UPLC-MS (ESI) calculated for $C_{27}H_{24}N_4O_7$ [M + H ]$^+$: 517.51, found:517.41.

**Example 49: 2-(2,6-Dioxopiperidin-3-yl)-4-(2-(4-(Oxetan-3-yloxy)phenyl)oxazol-5-yl)methyl)amino)isoindolin-1,3-dione**

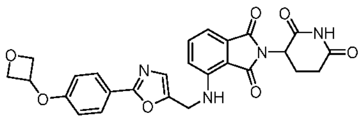

Following the synthetic route described in Example 22, the compound of Example 49 was obtained. A yellow solid (14.3 mg, 23% yield) was isolated. $^1$H NMR (500 MHz, DMSO) δ 11.09 (s, 1H), 7.83 (d, J = 8.9 Hz, 2H), 7.62 (dd, J = 8.3, 7.3 Hz, 1H), 7.29 (d, J = 8.6 Hz, 1H), 7.17 (s, 1H), 7.10 (t, J = 6.0 Hz, 2H), 6.92 (d, J = 8.9 Hz, 2H), 5.37 - 5.30 (m, 1H), 5.06 (dd, J = 12.8, 5.4 Hz, 1H), 4.94 (t, J = 6.8 Hz, 2H), 4.71 (d, J = 6.2 Hz, 2H), 4.55 (dd, J = 7.4, 5.1 Hz, 2H), 2.88 (ddd, J = 17.0, 13.8, 5.4 Hz, 1H), 2.57 (dt, J = 12.8, 9.4 Hz, 2H), 2.02 (ddd, J = 7.4, 5.2, 2.7 Hz, 1H). UPLC-MS (ESI) calculated for $C_{26}H_{22}N_4O_7$ [M + H ]$^+$: 503.48, found:503.39.

**Example 50: 2-(2,6-Dioxopiperidin-3-yl)-4-(2-(4-(2-Hydroxyethoxy)phenyl)oxazol-5-yl)methyl)amino)isoindolin-1,3-dione**

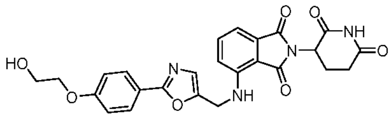

Following the synthetic route described in Example 22, the compound of Example 50 was obtained. A yellow solid (47 mg, 37% yield) was isolated. $^1H$ NMR (500 MHz, DMSO) δ 11.09 (s, 1H), 7.83 (d, J = 8.9 Hz, 2H), 7.62 (dd, J = 8.4, 7.2 Hz, 1H), 7.30 (d, J = 8.6 Hz, 1H), 7.16 (s, 1H), 7.09 (d, J = 7.0 Hz, 2H), 7.06 (d, J = 8.9 Hz, 2H), 5.06 (dd, J = 12.8, 5.4 Hz, 1H), 4.71 (d, J = 5.8 Hz, 2H), 4.04 (t, J = 4.9 Hz, 2H), 3.72 (t, J = 4.9 Hz, 2H), 2.88 (ddd, J = 17.0, 13.9, 5.4 Hz, 1H), 2.57 (ddd, J = 19.7, 14.6, 9.2 Hz, 2H), 2.06 - 2.00 (m, 1H). $^{13}C$ NMR (126 MHz, DMSO) δ 172.82, 170.07, 168.68, 167.23, 160.46, 160.44, 149.06, 145.62, 136.14, 132.20, 127.43, 125.54, 119.46, 117.74, 115.05, 111.31, 110.04, 69.75, 59.44, 48.58, 36.91, 30.97, 22.11. UPLC-MS (ESI) calculated for $C_{25}H_{22}N_4O_7$ [M + H ]$^+$: 491.15, found:491.41.

**Example 51: 4-(((2-(4-Cyclopropoxyphenyl)oxazol-5-yl)methyl)amino)-2-(2,6-Dioxopiperidin-3-yl)isoindolin-1,3-dione**

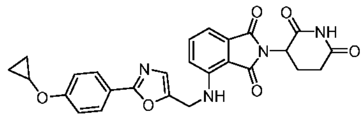

Following the synthetic route described in Example 22, the compound of Example 51 was obtained. A yellow solid (82.3 mg, 45% yield) was isolated. $^1H$ NMR (500 MHz, DMSO) δ 11.08 (s, 1H), 7.85 (d, J = 8.9 Hz, 2H), 7.63 (dd, J = 8.4, 7.2 Hz, 1H), 7.30 (d, J = 8.6 Hz, 1H), 7.19 - 7.15 (m, 3H), 7.09 (d, J = 7.0 Hz, 2H), 5.06 (dd, J = 12.8, 5.4 Hz, 1H), 4.71 (d, J = 5.2 Hz, 2H), 3.90 (tt, J = 6.0, 2.9 Hz, 1H), 2.88 (ddd, J = 17.1, 13.9, 5.4 Hz, 1H), 2.65 - 2.51 (m, 2H), 2.06 - 1.99 (m, 1H), 0.84 - 0.78 (m, 2H), 0.70 - 0.65 (m, 2H). $^{13}C$ NMR (126 MHz, DMSO) δ 172.82, 170.06, 168.68, 167.23, 160.41, 160.40, 149.15, 145.62, 136.14, 132.19, 127.38, 125.55, 119.95, 117.75, 115.50, 111.32, 110.05, 51.06, 48.58, 36.92, 30.97, 22.11, 5.94. UPLC-MS (ESI) calculated for $C_{26}H_{22}N_4O_6$ [M + H ]$^+$: 487.48 , found:487.39.

**Example 52: 2-(2,6-Dioxopiperidin-3-yl)-4-(2-(4-(2-Methoxyethoxy)phenyl)oxazol-5-yl)methyl)amino)isoindolin-1,3-dione**

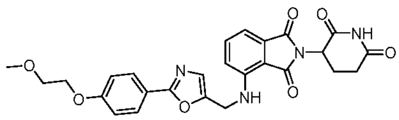

Following the synthetic route described in Example 22, the compound of Example 52 was obtained. A yellow solid (72.4 mg, 59% yield) was isolated. $^1H$ NMR (600 MHz, DMSO) δ 11.10 (s, 1H), 7.83 (d, J = 8.9 Hz, 2H), 7.62 (dd, J = 8.5, 7.2 Hz, 1H), 7.30 (d, J = 8.6 Hz, 1H), 7.16 (s, 1H), 7.13 - 7.03 (m, 4H), 5.06 (dd, J = 12.9, 5.4 Hz, 1H), 4.71 (d, J = 5.6 Hz, 2H), 4.16 - 4.12 (m, 2H), 3.69 - 3.64 (m, 2H), 3.30 (s, 3H), 2.88 (ddd, J = 17.1, 13.9, 5.4 Hz, 1H), 2.59 (dd, J = 17.2, 2.4 Hz, 1H), 2.56 - 2.51 (m, 1H), 2.03 (ddd, J = 10.4, 5.4, 3.0 Hz, 1H). $^{13}C$ NMR (151 MHz, DMSO) δ 172.86, 170.09, 168.71, 167.26, 160.45, 160.26, 149.12, 145.65, 136.18, 132.22, 127.47, 125.57, 119.61, 117.77, 115.06, 111.36, 110.07, 70.25, 67.16, 58.20, 48.61, 36.93, 30.99, 22.14. UPLC-MS (ESI) calculated for $C_{26}H_{24}N_4O_7$ [M + H ]$^+$: 505.50, found:505.41.

**Example 53: 2-(2,6-Dioxopiperidin-3-yl)-4-(2-(4-(Tetrahydro-2H-pyran-4-yl)oxy)phenyl)oxazol-5-yl)methyl)amino)isoindolin-1,3-dione**

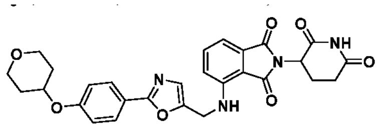

Following the synthetic route described in Example 22, the compound of Example 53 was obtained. A yellow solid (70 mg, 60% yield) was isolated. $^1$H NMR (500 MHz, DMSO) δ 11.09 (s, 1H), 7.82 (d, J = 8.8 Hz, 2H), 7.62 (dd, J = 8.4, 7.3 Hz, 1H), 7.29 (d, J = 8.6 Hz, 1H), 7.16 (s, 1H), 7.12 - 7.06 (m, 4H), 5.06 (dd, J = 12.8, 5.4 Hz, 1H), 4.71 (d, J = 4.6 Hz, 2H), 4.68 - 4.62 (m, 1H), 3.85 (dt, J = 11.4, 4.3 Hz, 2H), 3.51 - 3.47 (m, 2H), 2.88 (ddd, J = 17.1, 13.9, 5.4 Hz, 1H), 2.59 (dd, J = 17.7, 1.9 Hz, 1H), 2.53 (d, J = 4.4 Hz, 1H), 2.05 - 2.00 (m, 1H), 2.00 - 1.95 (m, 2H), 1.64 - 1.53 (m, 2H). $^{13}$C NMR (126 MHz, DMSO) δ 172.82, 170.06, 168.70, 167.24, 160.44, 158.76, 149.09, 145.64, 136.15, 132.20, 127.51, 125.54, 119.54, 117.75, 116.14, 111.34, 110.07, 71.55, 64.51, 48.61, 36.93, 31.66, 30.98, 22.13. UPLC-MS (ESI) calculated for $C_{28}H_{26}N_4O_7$ [M + H ]$^+$: 531.54, found:531.40.

**Example 54: 2-(2,6-Dioxopiperidin-3-yl)-4-(2-(4-((R)-Tetrahydrofuran-3-yl)oxy)phenyl)oxazol-5-yl)methyl)amino)isoindolin-1,3-dione**

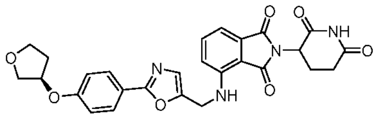

Following the synthetic route described in Example 22, the compound of Example 54 was obtained. A yellow solid (77.5 mg, 65% yield) was isolated. $^1$H NMR (600 MHz, DMSO) δ 11.10 (s, 1H), 7.83 (d, J = 8.9 Hz, 2H), 7.62 (dd, J = 8.5, 7.1 Hz, 1H), 7.30 (d, J = 8.6 Hz, 1H), 7.17 (s, 1H), 7.10 (dd, J = 10.4, 6.4 Hz, 2H), 7.05 (d, J = 8.9 Hz, 2H), 5.10 - 5.08 (m, 1H), 5.08 - 5.04 (m, 1H), 4.71 (d, J = 5.4 Hz, 2H), 3.89 (dd, J = 10.2, 4.5 Hz, 1H), 3.84 (dd, J = 15.3, 8.1 Hz, 1H), 3.79 (d, J = 10.2 Hz, 1H), 3.75 (td, J = 8.4, 4.6 Hz, 1H), 2.88 (ddd, J = 17.1, 13.9, 5.4 Hz, 1H), 2.62 - 2.56 (m, 1H), 2.53 (ddd, J = 9.8, 6.7, 3.2 Hz, 1H), 2.25 (dtd, J = 14.4, 8.2, 6.3 Hz, 1H), 2.06 - 2.00 (m, 1H), 1.96 (dt, J = 12.1, 5.7 Hz, 1H). $^{13}$C NMR (151 MHz, DMSO) δ 172.86, 170.10, 168.72, 167.26, 160.40, 158.93, 149.16, 145.65, 136.18, 132.22, 127.54, 125.58, 119.68, 117.77, 115.83, 111.36, 110.07, 77.44, 72.25, 66.45, 48.61, 36.93, 32.45, 30.99, 22.14. UPLC-MS ( ESI ) calculated for $C_{27}H_{26}N_4O_6$ [M + H ]$^+$: 517.51, found:517.26.

**Example 55: 2-(2,6-Dioxopiperidin-3-yl)-4-(2-(3-(Trifluoromethoxy)phenyl)oxazol-5-yl)methyl)amino)isoindolin-1,3-dione**

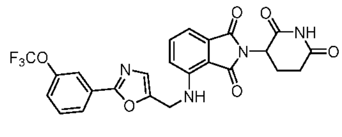

Following the synthetic route described in Example 22, the compound of Example 55 was obtained. A yellow solid (64.4 mg, 64% yield) was isolated. $^1$H NMR (500 MHz, DMSO) δ 11.08 (s, 1H), 7.94 (d, J = 7.9 Hz, 1H), 7.79 (s, 1H), 7.68 (t, J = 8.0 Hz, 1H), 7.62 (dd, J = 8.4, 7.3 Hz, 1H), 7.53 (d, J = 8.3 Hz, 1H), 7.30 (d, J = 8.6 Hz, 1H), 7.29 (s, 1H), 7.15 (t, J = 6.1 Hz, 1H), 7.10 (d, J = 7.1 Hz, 1H), 5.06 (dd, J = 12.7, 5.4 Hz, 1H), 4.75 (d, J = 5.6 Hz, 2H), 2.88 (ddd, J = 17.0, 13.9, 5.4 Hz, 1H), 2.64 - 2.52 (m, 2H), 2.03 (ddd, J = 10.4, 5.4, 3.2 Hz, 1H). $^{13}$C NMR (126 MHz, DMSO) δ 172.77, 170.02, 168.63, 167.19, 158.80, 150.65, 148.74, 145.55, 136.11, 132.19, 131.62, 128.82, 126.03, 124.68, 122.97, 117.73, 111.37, 110.15, 48.58, 36.95, 30.95, 22.09. UPLC-MS (ESI) calculated for $C_{24}H_{17}F_3N_4O_6$ [M + H ]$^+$: 515.42, found:515.33.

**Example 56: 2-(2,6-Dioxopiperidin-3-yl)-4-(2-(2-(Trifluoromethoxy)phenyl)oxazol-5-yl)methyl)amino)isoindolin-1,3-dione**

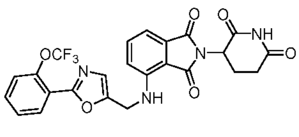

Following the synthetic route described in Example 22, the compound of Example 56 was obtained. A yellow solid

(66.4 mg, 66% yield) was isolated. $^1H$ NMR (500 MHz, DMSO) δ 11.08 (s, 1H), 8.04 (dd, J = 7.8, 1.6 Hz, 1H), 7.65 (td, J = 8.0, 1.6 Hz, 1H), 7.62 - 7.58 (m, 1H), 7.58 - 7.51 (m, 2H), 7.33 (s, 1H), 7.26 (d, J = 8.6 Hz, 1H), 7.12 (d, J = 6.3 Hz, 1H), 7.09 (d, J = 7.1 Hz, 1H), 5.07 (dd, J = 12.7, 5.4 Hz, 1H), 4.75 (d, J = 6.3 Hz, 2H), 2.89 (ddd, J = 17.0, 13.8, 5.4 Hz, 1H), 2.64 - 2.51 (m, 2H), 2.02 (ddd, J = 7.4, 5.4, 2.7 Hz, 1H). $^{13}C$ NMR (126 MHz, DMSO) δ 172.76, 169.98, 168.61, 167.21, 156.80, 150.52, 145.53, 144.89, 136.09, 132.28, 132.16, 129.96, 128.30, 125.92, 122.96, 120.82, 117.55, 111.33, 110.12, 48.56, 36.88, 30.94, 22.10. UPLC-MS (ESI) calculated for $C_{24}H_{17}F_3N_4O_6$ [M + H ]$^+$: 515.42, found:515.34.

**Example 57: 2-(2,6-Dioxopiperidin-3-yl)-4-(2-(4-(Trifluoromethyl)phenyl)oxazol-5-yl)methyl)amino)isoindolin-1,3-dione**

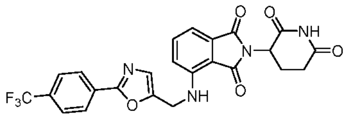

Following the synthetic route described in Example 22, the compound of Example 57 was obtained. A yellow solid (46.5 mg, 37% yield) was isolated. $^1H$ NMR (500 MHz, DMSO) δ 11.11 (s, 1H), 8.11 (d, J = 8.0 Hz, 2H), 7.89 (d, J = 7.9 Hz, 2H), 7.62 (t, J = 7.7 Hz, 1H), 7.32 (s, 1H), 7.30 (d, J = 8.6 Hz, 1H), 7.17 (t, J = 5.9 Hz, 1H), 7.10 (d, J = 6.9 Hz, 1H), 5.06 (dd, J = 12.8, 5.0 Hz, 1H), 4.76 (d, J = 5.5 Hz, 2H), 2.92 - 2.83 (m, 1H), 2.65 - 2.53 (m, 2H), 2.07 - 1.99 (m, 1H). $^{13}C$ NMR (151 MHz, DMSO) δ 172.88, 170.11, 168.69, 167.27, 159.06, 150.94, 145.59, 136.25, 132.25, 130.40, 130.15, 126.47, 126.30, 126.26, 124.86, 123.06, 117.76, 111.47, 110.18, 48.63, 37.02, 31.00, 22.16. UPLC-MS (ESI) calculated for $C_{24}H_{17}F_3N_4O_5$ [M + H ]$^+$: 499.42, found:499.35.

**Example 58: 2-(2,6-Dioxopiperidin-3-yl)-4-(2-(3-(Trifluoromethyl)phenyl)oxazol-5-yl)methyl)amino)isoindolin-1,3-dione**

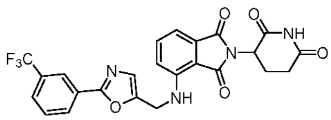

Following the synthetic route described in Example 22, the compound of Example 58 was obtained. A yellow solid (58.9 mg, 57% yield) was isolated. $^1H$ NMR (500 MHz, DMSO) δ 11.08 (s, 1H), 8.20 (d, J = 7.8 Hz, 1H), 8.15 (s, 1H), 7.89 (d, J = 7.9 Hz, 1H), 7.78 (t, J = 7.8 Hz, 1H), 7.62 (dd, J = 8.3, 7.3 Hz, 1H), 7.31 (t, J = 4.3 Hz, 2H), 7.17 (t, J = 6.2 Hz, 1H), 7.10 (d, J = 7.1 Hz, 1H), 5.07 (dd, J = 12.7, 5.4 Hz, 1H), 4.76 (d, J = 6.1 Hz, 2H), 2.88 (ddd, J = 16.9, 14.3, 5.4 Hz, 1H), 2.65 - 2.52 (m, 2H), 2.06 - 2.00 (m, 1H). $^{13}C$ NMR (126 MHz, DMSO) δ 172.77, 170.02, 168.62, 167.19, 158.88, 150.70, 145.54, 136.10, 132.20, 130.65, 129.52, 127.73, 126.98, 126.07, 124.83, 122.66, 121.92, 121.89, 117.74, 111.37, 110.16, 48.58, 36.96, 30.95, 22.10. UPLC-MS (ESI) calculated for $C_{24}H_{17}F_3N_4O_5$[M + H ]$^+$: 499.42, found:499.18.

**Example 59: 2-(2,6-Dioxopiperidin-3-yl)-4-(2-(2-(Trifluoromethyl)phenyl)oxazol-5-yl)methyl)amino)isoindolin-1,3-dione**

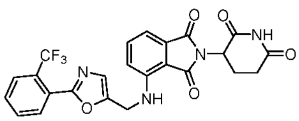

Following the synthetic route described in Example 22, the compound of Example 59 was obtained. A yellow solid (42.6 mg, 45% yield) was isolated. $^1H$ NMR (500 MHz, DMSO) δ 11.08 (s, 1H), 7.97 (d, J = 7.7 Hz, 1H), 7.91 (d, J = 7.8 Hz, 1H), 7.82 (t, J = 7.5 Hz, 1H), 7.75 (t, J = 7.6 Hz, 1H), 7.63 - 7.58 (m, 1H), 7.32 (s, 1H), 7.25 (d, J = 8.6 Hz, 1H), 7.10 (t, J = 6.7 Hz, 2H), 5.07 (dd, J = 12.7, 5.4 Hz, 1H), 4.75 (d, J = 6.0 Hz, 2H), 2.89 (ddd, J = 16.9, 13.8, 5.3 Hz, 1H), 2.65 - 2.52 (m, 2H), 2.06 - 1.99 (m, 1H). $^{13}C$ NMR (126 MHz, DMSO) δ 172.81, 170.03, 168.63, 167.23, 158.02, 151.05, 145.58, 136.10,

132.93, 132.16, 131.30, 131.05, 127.08, 127.03, 126.99, 126.95, 125.90, 117.71, 111.38, 110.15, 48.57, 36.87, 30.96, 22.12. UPLC-MS ( ESI ) calculated for $C_{24}H_{17}F_3N_4O_5$ [M + H ]$^+$: 499.42, found:499.34.

**Example 60: 2-(2,6-Dioxopiperidin-3-yl)-4-(5-(4-(Trifluoromethyl)phenyl)oxazol-2-yl)methyl)amino)isoindolin-1,3-dione**

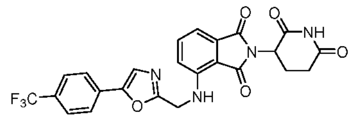

Following the synthetic route described in Example 24, the compound of Example 60 was obtained. A yellow solid (17.6 mg, 17% yield) was isolated. $^1$H NMR (500 MHz, DMSO) δ 11.10 (s, 1H), 7.88 (d, J = 8.4 Hz, 2H), 7.83 (t, J = 4.2 Hz, 3H), 7.61 (dd, J = 8.1, 7.5 Hz, 1H), 7.25 (d, J = 6.1 Hz, 1H), 7.22 (d, J = 8.6 Hz, 1H), 7.11 (d, J = 7.1 Hz, 1H), 5.08 (dd, J = 12.8, 5.4 Hz, 1H), 4.83 (d, J = 6.2 Hz, 2H), 2.94 - 2.85 (m, 1H), 2.63 - 2.58 (m, 1H), 2.57 - 2.51 (m, 1H), 2.06 - 2.01 (m, 1H).$^{13}$C NMR (126 MHz, DMSO) δ 172.78, 170.03, 168.65, 167.21, 162.01, 149.46, 145.71, 136.16, 132.10, 131.09, 130.09, 128.43, 128.17, 126.14, 126.11, 125.11, 124.81, 124.34, 122.95, 117.84, 111.50, 110.19, 48.62, 30.97, 22.11. UPLC-MS (ESI) calculated for $C_{24}H_{17}F_3N_4O_5$ [M + H ]$^+$: 499.42, found:499.35.

**Example 61: 2-(2,6-Dioxopiperidin-3-yl)-4-(5-(3-(Trifluoromethyl)phenyl)oxazol-2-yl)methyl)amino)isoindolin-1,3-dione**

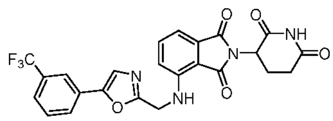

Following the synthetic route described in Example 24, the compound of Example 61 was obtained. A yellow solid (54.6 mg, 53% yield) was isolated.$^1$H NMR (500 MHz, DMSO) δ 11.10 (s, 1H), 7.99 (s, 1H), 7.96 (dd, J = 4.2, 3.1 Hz, 1H), 7.84 (s, 1H), 7.74 - 7.70 (m, 2H), 7.64 - 7.59 (m, 1H), 7.24 (dd, J = 12.9, 7.4 Hz, 2H), 7.11 (d, J = 7.0 Hz, 1H), 5.08 (dd, J = 12.8, 5.4 Hz, 1H), 4.82 (d, J = 6.3 Hz, 2H), 2.89 (ddd, J = 16.9, 13.8, 5.2 Hz, 1H), 2.60 (dd, J = 15.0, 3.3 Hz, 1H), 2.54 (dd, J = 13.5, 4.4 Hz, 1H), 2.04 (ddd, J = 12.4, 5.3, 2.2 Hz, 1H). $^{13}$C NMR (126 MHz, DMSO) δ 172.77, 170.01, 168.64, 167.20, 161.66, 149.36, 145.71, 136.12, 132.09, 130.39, 128.39, 127.56, 124.85, 124.20, 120.21, 120.18, 117.88, 111.48, 110.18, 48.61, 30.96, 22.10. UPLC-MS ( ESI ) calculated for $C_{24}H_{17}F_3N_4O_5$ [M + H ]$^+$: 499.42, found:499.31.

**Example 62: 2-(2,6-Dioxopiperidin-3-yl)-4-(5-(2-(Trifluoromethyl)phenyl)oxazol-2-yl)methyl)amino)isoindolin-1,3-dione**

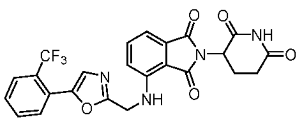

Following the synthetic route described in Example 24, the compound of Example 62 was obtained. A yellow solid (20.6 mg, 20% yield) was isolated. $^1$H NMR (500 MHz, DMSO) δ 11.09 (s, 1H), 7.88 (d, J = 7.9 Hz, 1H), 7.81 - 7.76 (m, 2H), 7.66 (dd, J = 10.9, 5.1 Hz, 1H), 7.62 - 7.57 (m, 1H), 7.43 (s, 1H), 7.23 (t, J = 6.3 Hz, 1H), 7.19 (d, J = 8.6 Hz, 1H), 7.11 (d, J = 7.0 Hz, 1H), 5.08 (dd, J = 12.7, 5.4 Hz, 1H), 4.82 (d, J = 6.3 Hz, 2H), 2.89 (ddd, J = 16.6, 13.7, 5.3 Hz, 1H), 2.60 (d, J = 18.4 Hz, 1H), 2.56 - 2.51 (m, 1H), 2.07 - 2.01 (m, 1H). $^{13}$C NMR (126 MHz, DMSO) δ 172.77, 169.99, 168.62, 167.21, 162.06, 147.66, 145.71, 136.09, 132.97, 132.07, 130.39, 129.73, 126.86, 126.81, 126.77, 126.72, 126.10, 126.08, 125.46, 124.70, 122.53, 117.77, 111.47, 110.21, 48.59, 30.95, 22.11. UPLC-MS ( ESI ) calculated for $C_{24}H_{17}F_3N_4O_5$ [M + H ]$^+$: 499.42, found:499.24.

**Example 63: 4-(((2-(3-Chlorophenyl)oxazol-5-yl)methyl)amino)-2-(2,6-Dioxopiperidin-3-yl)isoindolin-1,3-dione**

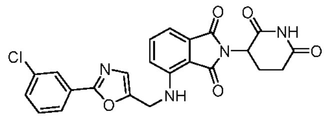

Following the synthetic route described in Example 22, the compound of Example 63 was obtained. A yellow solid (76.1 mg, 68% yield) was isolated. $^1$H NMR (500 MHz, DMSO) δ 11.08 (s, 1H), 7.89 (d, J = 1.7 Hz, 1H), 7.87 (dt, J = 7.0, 1.5 Hz, 1H), 7.63 (dd, J = 8.4, 7.2 Hz, 1H), 7.60 - 7.55 (m, 2H), 7.30 (d, J = 8.6 Hz, 1H), 7.28 (s, 1H), 7.15 (t, J = 5.9 Hz, 1H), 7.10 (d, J = 7.1 Hz, 1H), 5.07 (dd, J = 12.7, 5.4 Hz, 1H), 4.74 (d, J = 4.8 Hz, 2H), 2.88 (ddd, J = 16.9, 13.9, 5.4 Hz, 1H), 2.61 (ddd, J = 33.5, 17.4, 7.5 Hz, 2H), 2.03 (ddd, J = 12.1, 6.2, 3.9 Hz, 1H). $^{13}$C NMR (126 MHz, DMSO) δ 172.77, 170.02, 168.63, 167.20, 158.90, 150.48, 145.54, 136.13, 133.84, 132.20, 131.26, 130.33, 128.69, 126.00, 125.18, 124.29, 117.72, 111.37, 110.14, 48.58, 36.95, 30.95, 22.10. UPLC-MS (ESI) calculated for $C_{23}H_{17}ClN4O_5$ [M + H ]$^+$: 465.86, found:465.31.

**Example 64: 4-(((5-(4-Chlorophenyl)oxazol-2-yl)methyl)amino)-2-(2,6-Dioxopiperidin-3-yl)isoindolin-1,3-dione**

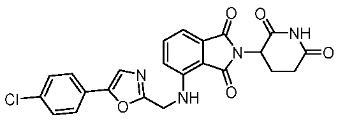

Following the synthetic route described in Example 24, the compound of Example 64 was obtained. A yellow solid (10.2 mg, 9% yield) was isolated. $^1$H NMR (500 MHz, DMSO) δ 11.10 (s, 1H), 7.68 (d, J = 8.7 Hz, 2H), 7.67 (s, 1H), 7.63 - 7.59 (m, 1H), 7.53 (d, J = 8.6 Hz, 2H), 7.25 - 7.20 (m, 2H), 7.11 (d, J = 7.0 Hz, 1H), 5.08 (dd, J = 12.7, 5.4 Hz, 1H), 4.80 (d, J = 6.3 Hz, 2H), 2.89 (ddd, J = 16.7, 13.6, 5.3 Hz, 1H), 2.57 (ddd, J = 18.0, 13.9, 3.5 Hz, 2H), 2.04 (ddd, J = 12.8, 5.6, 2.3 Hz, 1H). $^{13}$C NMR (126 MHz, DMSO) δ 172.78, 170.02, 168.65, 167.21, 161.23, 149.82, 145.72, 136.15, 132.87, 132.09, 129.20, 126.25, 125.53, 123.19, 117.83, 111.47, 110.16, 48.61, 30.96, 22.10. UPLC-MS (ESI) calculated for $C_{23}H_{17}ClN_4O_5$ [M + H ]$^+$: 465.86, found:465.29.

**Example 65: 4-(((5-(3-Chlorophenyl)oxazol-2-yl)methyl)amino)-2-(2,6-Dioxopiperidin-3-yl)isoindolin-1,3-dione**

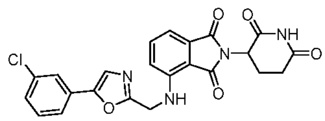

Following the synthetic route described in Example 24, the compound of Example 65 was obtained. A yellow solid (5.1 mg, 5% yield) was isolated. $^1$H NMR (500 MHz, DMSO) δ 11.10 (s, 1H), 7.76 - 7.73 (m, 2H), 7.63 - 7.60 (m, 2H), 7.49 (t, J = 7.9 Hz, 1H), 7.42 (dd, J = 8.0, 1.0 Hz, 1H), 7.23 (dd, J = 11.1, 7.4 Hz, 2H), 7.11 (d, J = 7.1 Hz, 1H), 5.08 (dd, J = 12.8, 5.4 Hz, 1H), 4.80 (d, J = 6.2 Hz, 2H), 2.95 - 2.83 (m, 1H), 2.60 (dd, J = 14.8, 3.5 Hz, 1H), 2.57 - 2.52 (m, 1H), 2.08 - 2.01 (m, 1H). $^{13}$C NMR (126 MHz, DMSO) δ 172.78, 170.02, 168.64, 167.21, 161.48, 149.39, 145.70, 136.14, 133.88, 132.09, 131.10, 129.33, 128.20, 123.88, 123.42, 122.30, 117.85, 111.47, 110.17, 48.61, 30.96, 22.10. UPLC-MS ( ESI ) calculated for $C_{23}H_{17}ClN_4O_5$ [M + H ]$^+$: 465.86, found:465.28.

**Example 66: 4-(((5-(2-Chlorophenyl)oxazol-2-yl)methyl)amino)-2-(2,6-Dioxopiperidin-3-yl)isoindolin-1,3-dione**

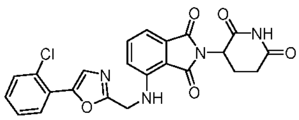

Following the synthetic route described in Example 24, the compound of Example 66 was obtained. A yellow solid (21.6 mg, 19% yield) was isolated. $^{1}H$ NMR (500 MHz, DMSO) δ 11.10 (s, 1H), 7.74 (dd, J = 7.8, 1.5 Hz, 1H), 7.72 (s, 1H), 7.64 - 7.60 (m, 1H), 7.59 (d, J = 1.0 Hz, 1H), 7.48 (td, J = 7.7, 1.2 Hz, 1H), 7.40 (td, J = 7.8, 1.6 Hz, 1H), 7.26 (d, J = 6.3 Hz, 1H), 7.22 (s, 1H), 7.11 (d, J = 7.1 Hz, 1H), 5.08 (dd, J = 12.8, 5.4 Hz, 1H), 4.83 (d, J = 6.3 Hz, 2H), 2.89 (ddd, J = 16.7, 13.7, 5.2 Hz, 1H), 2.63 - 2.57 (m, 1H), 2.57 - 2.52 (m, 1H), 2.06 - 2.02 (m, 1H). $^{13}C$ NMR (126 MHz, DMSO) δ 172.77, 170.02, 168.64, 167.21, 161.28, 147.37, 145.73, 136.15, 132.09, 130.67, 129.88, 129.44, 127.80, 126.61, 125.87, 117.88, 111.49, 110.20, 48.61, 30.96, 22.10. UPLC-MS ( ESI) calculated for $C_{23}H_{17}ClN_4O_5$ [M + H ]$^+$: 465.86, found:465.32.

**Example 67: 4-(((2-(4-Chlorophenyl)thiazol-5-yl)methyl)amino)-2-(2,6-Dioxopiperidin-3-yl)isoindolin-1,3-dione**

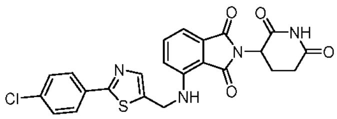

Following the synthetic route described in Example 32, the compound of Example 67 was obtained.

**Example 68: 4-(((2-(3-Chlorophenyl)thiazol-5-yl)methyl)amino)-2-(2,6-Dioxopiperidin-3-yl)isoindolin-1,3-dione**

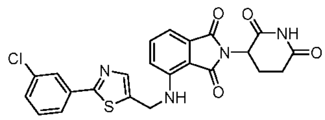

Following the synthetic route described in Example 32, the compound of Example 68 was obtained. A yellow solid (10.8 mg, 55% yield) was isolated. $^{1}H$ NMR (500 MHz, DMSO) δ 11.09 (s, 1H), 7.94 (s, 1H), 7.90 (d, J = 1.7 Hz, 1H), 7.85 - 7.81 (m, 1H), 7.62 - 7.56 (m, 1H), 7.54 - 7.46 (m, 2H), 7.35 (t, J = 6.3 Hz, 1H), 7.22 (d, J = 8.6 Hz, 1H), 7.08 (d, J = 7.1 Hz, 1H), 5.07 (dd, J = 12.7, 5.4 Hz, 1H), 4.86 (d, J = 6.3 Hz, 2H), 2.89 (ddd, J = 17.1, 13.8, 5.4 Hz, 1H), 2.64 - 2.52 (m, 2H), 2.04 (ddd, J = 17.4, 7.8, 4.8 Hz, 1H). $^{13}C$ NMR (126 MHz, DMSO) δ 172.78, 170.05, 168.57, 167.19, 164.75, 145.30, 142.13, 138.95, 136.20, 134.94, 133.91, 132.28, 131.15, 129.82, 125.20, 124.77, 117.61, 111.30, 110.11, 48.59, 38.45, 30.96, 22.11. UPLC-MS ( ESI ) calculated for $C_{23}H_{17}ClN_4O_4S$[M + H ]$^+$: 481.92, found:481.15.

**Example 69: 4-(((2-(2-Chlorophenyl)thiazol-5-yl)methyl)amino)-2-(2,6-Dioxopiperidin-3-yl)isoindolin-1,3-dione**

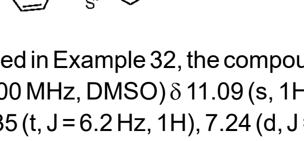

Following the synthetic route described in Example 32, the compound of Example 69 was obtained. A yellow solid (20.3 mg, 12% yield) was isolated. $^{1}H$ NMR (500 MHz, DMSO) δ 11.09 (s, 1H), 8.18 - 8.11 (m, 1H), 8.01 (s, 1H), 7.61 (ddd, J = 12.4, 6.5, 5.1 Hz, 2H), 7.50 - 7.44 (m, 2H), 7.35 (t, J = 6.2 Hz, 1H), 7.24 (d, J = 8.6 Hz, 1H), 7.08 (d, J = 7.0 Hz, 1H), 5.07 (dd, J = 12.7, 5.4 Hz, 1H), 4.88 (d, J = 5.9 Hz, 2H), 2.89 (ddd, J = 16.9, 13.8, 5.4 Hz, 1H), 2.64 - 2.53 (m, 2H), 2.07 - 1.99 (m, 1H).$^{13}C$ NMR (126 MHz, DMSO) δ 172.78, 170.05, 168.58, 167.20, 161.71, 145.36, 140.97, 139.38, 136.20, 132.30, 131.25, 131.03, 130.72, 130.47, 130.42, 127.71, 117.61, 111.27, 110.08, 48.59, 38.31, 30.96, 22.10. UPLC-MS ( ESI ) calculated for $C_{23}H_{17}ClN_4O_4S$ [M + H ]$^+$: 481.92, found:481.14.

**Example 70: 2-(2,6-Dioxopiperidin-3-yl)-4-(((2-(3-Fluorophenyl)oxazol-5-yl)methyl)amino)isoindolin-1,3-dione**

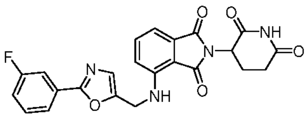

Following the synthetic route described in Example 22, the compound of Example 70 was obtained. A yellow solid (79.1 mg, 73% yield) was isolated. $^1$H NMR (500 MHz, DMSO) δ 11.09 (s, 1H), 7.76 (d, J = 7.8 Hz, 1H), 7.67 - 7.61 (m, 2H), 7.58 (td, J = 8.0, 6.0 Hz, 1H), 7.36 (td, J = 8.4, 2.1 Hz, 1H), 7.30 (d, J = 8.6 Hz, 1H), 7.27 (s, 1H), 7.14 (t, J = 5.9 Hz, 1H), 7.10 (d, J = 7.1 Hz, 1H), 5.07 (dd, J = 12.7, 5.4 Hz, 1H), 4.74 (d, J = 5.5 Hz, 2H), 2.88 (ddd, J = 16.9, 13.8, 5.3 Hz, 1H), 2.58 (dt, J = 17.7, 9.4 Hz, 2H), 2.03 (ddd, J = 10.8, 5.6, 3.3 Hz, 1H). $^{13}$C NMR (126 MHz, DMSO) δ 172.77, 170.02, 168.63, 167.19, 163.25, 161.31, 159.13, 159.11, 150.40, 145.55, 136.14, 132.19, 131.57, 131.50, 128.93, 128.86, 125.96, 121.87, 117.71, 117.53, 117.36, 112.39, 112.20, 111.37, 110.13, 48.58, 36.94, 30.95, 22.10. UPLC-MS ( ESI ) calculated for $C_{23}H_{17}FN_4O_5$ [M + H ]$^+$: 449.41, found: 449.32.

**Example 71: 2-(2,6-Dioxopiperidin-3-yl)-4-(((2-(2-Fluorophenyl)oxazol-5-yl)methyl)amino)isoindolin-1,3-dione**

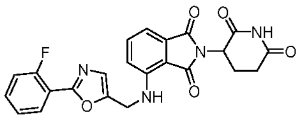

Following the synthetic route described in Example 22, the compound of Example 71 was obtained. A yellow solid (54.6 mg, 51% yield) was isolated. $^1$H NMR (500 MHz, DMSO) δ 11.08 (s, 1H), 7.95 (td, J = 7.7, 1.6 Hz, 1H), 7.62 (dd, J = 8.4, 7.3 Hz, 1H), 7.56 (tdd, J = 7.1, 5.1, 1.7 Hz, 1H), 7.42 - 7.37 (m, 1H), 7.35 (dd, J = 11.2, 3.9 Hz, 1H), 7.29 (t, J = 4.3 Hz, 2H), 7.13 (t, J = 5.8 Hz, 1H), 7.10 (d, J = 7.1 Hz, 1H), 5.06 (dd, J = 12.7, 5.4 Hz, 1H), 4.75 (d, J = 4.9 Hz, 2H), 2.88 (ddd, J = 17.0, 13.8, 5.4 Hz, 1H), 2.65 - 2.53 (m, 2H), 2.03 (ddd, J = 7.2, 5.3, 2.5 Hz, 1H). $^{13}$C NMR (126 MHz, DMSO) δ 172.77, 170.02, 168.64, 167.20, 159.99, 157.96, 156.70, 150.24, 145.56, 136.12, 132.65, 132.58, 132.17, 129.19, 125.72, 125.03, 117.72, 117.06, 116.89, 114.98, 114.89, 111.35, 110.09, 48.57, 36.91, 30.95, 22.09. UPLC-MS (ESI) calculated for $C_{23}H_{17}FN_4O_5$ [M + H ]$^+$: 449.41, found:449.27.

**Example 72: 2-(2,6-Dioxopiperidin-3-yl)-4-(((5-(4-Fluorophenyl)oxazol-2-yl)methyl)amino)isoindolin-1,3-dione**

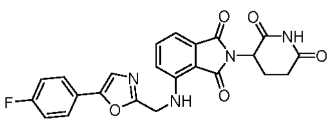

Following the synthetic route described in Example 24, the compound of Example 72 was obtained. A yellow solid (10.5 mg, 10% yield) was isolated. $^1$H NMR (500 MHz, DMSO) δ 11.10 (s, 1H), 7.73 - 7.68 (m, 2H), 7.61 (dd, J = 8.4, 7.3 Hz, 1H), 7.59 (s, 1H), 7.31 (t, J = 8.9 Hz, 2H), 7.22 (t, J = 6.7 Hz, 2H), 7.11 (d, J = 7.1 Hz, 1H), 5.08 (dd, J = 12.7, 5.4 Hz, 1H), 4.79 (d, J = 6.1 Hz, 2H), 2.89 (ddd, J = 16.8, 13.8, 5.2 Hz, 1H), 2.64 - 2.52 (m, 2H), 2.07 - 2.02 (m, 1H). $^{13}$C NMR (126 MHz, DMSO) δ 172.78, 170.03, 168.66, 167.21, 162.90, 160.94, 160.89, 150.03, 145.73, 136.15, 132.09, 126.12, 126.06, 124.08, 124.05, 122.31, 117.83, 116.28, 116.11, 111.46, 110.15, 48.61, 30.97, 22.11. UPLC-MS ( ESI ) calculated for $C_{23}H_{17}FN_4O_5$ [M + H ]$^+$: 449.41, found:449.38.

**Example 73: 4-(((2-(3,4-Dichlorophenyl)oxazol-5-yl)methyl)amino)-2-(2,6-Dioxopiperidin-3-yl)isoindolin-1,3-dione**

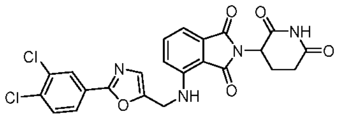

Following the synthetic route described in Example 22, the compound of Example 73 was obtained. A yellow solid (77 mg, 62% yield) was isolated. $^1$H NMR (500 MHz, DMSO) δ 11.11 (s, 1H), 8.06 (d, J = 1.9 Hz, 1H), 7.86 (dd, J = 8.4, 2.0 Hz, 1H), 7.80 (d, J = 8.4 Hz, 1H), 7.65 - 7.60 (m, 1H), 7.31-7.26 (m, 2H), 7.16 (t, J = 6.3 Hz, 1H), 7.10 (d, J = 7.0 Hz, 1H), 5.06 (dd, J = 12.8, 5.4 Hz, 1H), 4.74 (d, J = 6.1 Hz, 2H), 2.88 (ddd, J = 17.3, 14.0, 5.5 Hz, 1H), 2.64 - 2.53 (m, 2H), 2.02 (ddd, J = 7.7, 6.4, 2.1 Hz, 1H). $^{13}$C NMR (151 MHz, DMSO) δ 172.87, 170.11, 168.68, 167.26, 158.27, 150.80, 145.56, 136.22, 133.19, 132.25, 132.09, 131.70, 127.28, 127.24, 126.22, 125.79, 117.76, 111.46, 110.19, 48.63, 36.98, 31.00, 22.16. UPLC-MS ( ESI ) calculated for $C_{23}H_{16}Cl_2N_4O_5$ [M + H ]$^+$: 499.05 found:499.25.

**Example 74: 4-(((2-(2,3-Dichlorophenyl)oxazol-5-yl)methyl)amino)-2-(2,6-Dioxopiperidin-3-yl)isoindolin-1,3-dione**

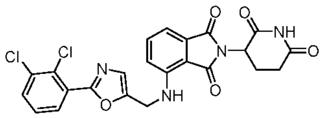

Following the synthetic route described in Example 22, the compound of Example 74 was obtained. A yellow solid (64.8 mg, 63% yield) was isolated. $^1$H NMR (500 MHz, DMSO) δ 11.08 (s, 1H), 7.89 (dd, J = 7.9, 1.5 Hz, 1H), 7.81 (dd, J = 8.1, 1.5 Hz, 1H), 7.62 (dd, J = 8.4, 7.3 Hz, 1H), 7.51 (t, J = 8.0 Hz, 1H), 7.34 (s, 1H), 7.30 (d, J = 8.6 Hz, 1H), 7.14 (t, J = 6.3 Hz, 1H), 7.10 (d, J = 7.1 Hz, 1H), 5.07 (dd, J = 12.7, 5.4 Hz, 1H), 4.77 (d, J = 6.0 Hz, 2H), 2.88 (ddd, J = 17.0, 13.8, 5.4 Hz, 1H), 2.58 (dt, J = 17.9, 9.3 Hz, 2H), 2.06 - 2.00 (m, 1H). $^{13}$C NMR (126 MHz, DMSO) δ 172.77, 170.01, 168.63, 167.19, 157.45, 150.77, 145.56, 136.13, 133.48, 132.28, 132.16, 129.55, 129.31, 128.63, 127.94, 125.73, 117.83, 111.39, 110.14, 48.58, 36.89, 30.95, 22.10. UPLC-MS ( ESI ) calculated for $C_{26}H_{24}N_4O_7$ [M + H ]+: 499.05, found: 499.12.

**Example 75: 4-(((2-(3,5-Dichlorophenyl)oxazol-5-yl)methyl)amino)-2-(2,6-Dioxopiperidin-3-yl)isoindolin-1,3-dione**

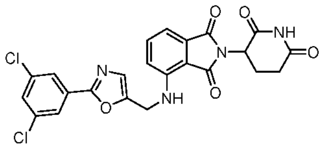

Following the synthetic route described in Example 22, the compound of Example 75 was obtained. A yellow solid (47.2 mg, 38% yield) was isolated. $^1$H NMR (500 MHz, DMSO) δ 11.11 (s, 1H), 7.86 (d, J = 1.9 Hz, 2H), 7.79 (t, J = 1.9 Hz, 1H), 7.62 (dd, J = 8.4, 7.3 Hz, 1H), 7.31 (s, 1H), 7.29 (d, J = 8.6 Hz, 1H), 7.17 (t, J = 6.3 Hz, 1H), 7.10 (d, J = 7.1 Hz, 1H), 5.06 (dd, J = 12.7, 5.4 Hz, 1H), 4.74 (d, J = 6.1 Hz, 2H), 2.88 (ddd, J = 17.0, 14.1, 5.5 Hz, 1H), 2.65 - 2.59 (m, 1H), 2.56 (dd, J = 13.2, 8.2 Hz, 1H), 2.03 (ddd, J = 10.4, 5.5, 3.1 Hz, 1H). $^{13}$C NMR (151 MHz, DMSO) δ 172.87, 170.11, 168.67, 167.26, 157.76, 151.12, 145.54, 136.21, 135.07, 132.26, 129.91, 129.87, 126.31, 124.15, 117.78, 111.47, 110.22, 48.63, 36.98, 31.00, 22.16. UPLC-MS ( ESI ) calculated for $C_{23}H_{16}Cl_2N_4O_5$ [M + H ]$^+$: 499.05, found: 499.28.

**Example 76: 4-(((2-(2,4-Dichlorophenyl)oxazol-5-yl)methyl)amino)-2-(2,6-Dioxopiperidin-3-yl)isoindolin-1,3-dione**

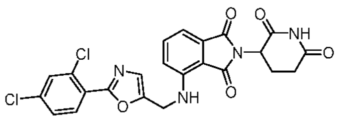

Following the synthetic route described in Example 22, the compound of Example 76 was obtained. A yellow solid (55.5 mg, 45% yield) was isolated. $^1$H NMR (500 MHz, DMSO) δ 11.11 (s, 1H), 7.94 (d, J = 8.5 Hz, 1H), 7.80 (d, J = 2.1 Hz, 1H), 7.63-7.54 (m, 2H), 7.32 (s, 1H), 7.30 (d, J = 8.6 Hz, 1H), 7.14 (t, J = 6.5 Hz, 1H), 7.10 (d, J = 7.1 Hz, 1H), 5.06 (dd, J = 12.8, 5.5 Hz, 1H), 5.06 (dd, J = 12.8, 5.5 Hz, 1H), 4.75 (d, J = 6.3 Hz, 2H), 2.88 (ddd, J = 17.0, 13.9, 5.4 Hz, 1H), 2.64 - 2.52 (m, 2H), 2.05 - 2.00 (m, 1H). $^{13}$C NMR (151 MHz, DMSO) δ 172.87, 170.09, 168.69, 167.27, 157.34, 150.69, 145.62, 136.20, 135.64, 132.20, 132.04, 131.90, 130.69, 128.02, 125.83, 124.58, 117.91, 111.46, 110.17, 48.63, 36.92, 31.00, 22.16. UPLC-MS (ESI) calculated for $C_{23}H_{16}Cl_2N_4O_5$ [M + H ]$^+$: 499.05, found: 499.13.

**Example 77: 4-((2-(2,5-Dichlorophenyl)oxazol-5-yl)methyl)amino)-2-(2,6-Dioxopiperidin-3-yl)isoindole-1,3-dione**

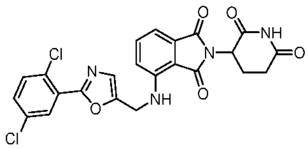

The compound of Example 77 was synthesized following the route described in Example 22. A yellow solid was obtained (53.1 mg, yield 51%).$^1$H NMR (500 MHz, DMSO) δ 11.08 (s, 1H), 7.95 (d, J = 2.5 Hz, 1H), 7.65 (d, J = 8.6 Hz, 1H), 7.63 - 7.58 (m, 2H), 7.34 (s, 1H), 7.30 (d, J = 8.6 Hz, 1H), 7.15 (t, J = 6.4 Hz, 1H), 7.10 (d, J = 7.1 Hz, 1H), 5.06 (dd, J = 12.7, 5.4 Hz, 1H), 4.76 (d, J = 6.2 Hz, 2H), 2.88 (ddd, J = 17.0, 13.9, 5.4 Hz, 1H), 2.62 - 2.53 (m, 2H), 2.06 - 2.00 (m, 1H). $^{13}$C NMR (126 MHz, DMSO) δ 172.77, 170.01, 168.62, 167.20, 156.79, 150.90, 145.56, 136.09, 132.93, 132.16, 132.08, 131.44, 129.81, 129.72, 127.03, 125.83, 117.87, 111.39, 110.17, 48.58, 36.88, 30.95, 22.10. UPLC-MS (ESI) calculated for $C_{23}H_{16}Cl_2N_4O_5$ [M + H ]$^+$: 499.05, found:499.29.

**Example 78: 4-((2-(2,6-Dichlorophenyl)oxazol-5-yl)methyl)amino)-2-(2,6-Dioxopiperidin-3-yl)isoindole-1,3-dione**

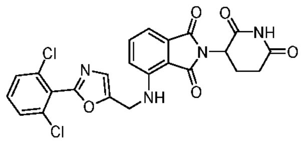

The compound of Example 78 was synthesized following the route described in Example 22.

**Example 79: 4-(((2-(((2R,6S)-2,6-Dimethylmorpholino)methyl)oxazol-5-yl)methyl)amino)-2-(2,6-Dioxopiperidin-3-yl)isoindoline-1,3-dione**

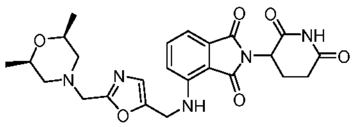

The compound 2-(((2R,6S)-2,6-Dimethylmorpholino)methyl)oxazol-5-carbaldehyde (30 mg, 0.1338 mmol) and Pomadime (37 mg, 0.1338 mmol) were dissolved in 3 mL acetic acid, and the reaction mixture was refluxed at 135 °C overnight. After cooling to room temperature, sodium borohydride (10 mg, 0.2675 mmol) was added and the reaction was allowed to proceed at room temperature for 1 hour. Upon completion, the reaction was quenched by adding water under ice

bath conditions. The resulting mixture was concentrated under reduced pressure, and the residue was dissolved in ethyl acetate. The organic layer was washed three times with water, then successively with saturated sodium bicarbonate solution and saturated sodium chloride solution. The organic phase was dried over anhydrous sodium sulfate, filtered, and the filtrate was concentrated under reduced pressure. $^1$H NMR (500 MHz, DMSO) δ 11.08 (s, 1H), 7.61 - 7.56 (m, 1H), 7.20 (d, J = 8.6 Hz, 1H), 7.08 (d, J = 7.1 Hz, 1H), 7.06 - 7.02 (m, 2H), 5.06 (dd, J = 12.8, 5.4 Hz, 1H), 4.64 (d, J = 6.3 Hz, 2H), 3.58 (s, 2H), 3.49 (dd, J = 13.8, 6.6 Hz, 2H), 2.88 (ddd, J = 17.2, 13.9, 5.4 Hz, 1H), 2.63 (d, J = 11.5 Hz, 2H), 2.61 - 2.51 (m, 2H), 2.05 - 1.99 (m, 1H), 1.70 (td, J = 10.7, 4.9 Hz, 2H), 0.97 (dd, J = 6.2, 1.9 Hz, 6H). $^{13}$C NMR (126 MHz, DMSO) δ 172.81, 170.04, 168.66, 167.21, 160.26, 149.55, 145.53, 136.09, 132.16, 124.27, 117.66, 111.26, 109.97, 70.82, 58.15, 53.54, 48.58, 36.74, 30.97, 22.12, 18.86. UPLC-MS ( ESI ) calculated for $C_{24}H_{27}N_5O_6$ [M + H ]$^+$: 482.51, found:482.20.

**Example 80: 2-(2,6-Dioxopiperidin-3-yl)-4-((2-((3-Morpholinoazo-1-yl)methyl)oxazol-5-yl)methyl)amino)isoindole-1,3-dione**

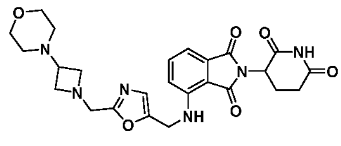

The compound of Example 80 was synthesized following the route described in Example 79. A yellow solid was obtained (16.8 mg, yield 6%).$^1$H NMR (500 MHz, DMSO) δ 11.09 (s, 1H), 7.59 (dd, J = 8.3, 7.4 Hz, 1H), 7.21 (d, J = 8.6 Hz, 1H), 7.07 (d, J = 7.0 Hz, 1H), 7.03 (t, J = 6.4 Hz, 1H), 7.01 (s, 1H), 5.05 (dd, J = 12.8, 5.4 Hz, 1H), 4.62 (d, J = 6.3 Hz, 2H), 3.61 (s, 2H), 3.54 - 3.50 (m, 4H), 3.44 - 3.37 (m, 2H), 2.93 (t, J = 6.9 Hz, 2H), 2.91 - 2.85 (m, 1H), 2.83 (dd, J = 12.9, 6.4 Hz, 1H), 2.62 - 2.56 (m, 1H), 2.56 - 2.51 (m, 1H), 2.17 (s, 4H), 2.05 - 1.98 (m, 1H). $^{13}$C NMR (126 MHz, DMSO) δ 172.81, 170.03, 168.69, 167.22, 160.53, 149.42, 145.60, 136.11, 132.17, 124.17, 117.67, 111.28, 109.99, 65.86, 57.83, 55.11, 53.79, 49.65, 48.60, 36.71, 30.98, 22.13. UPLC-MS (ESI) calculated for $C_{25}H_{28}N_6O_6$ [M + H ]$^+$: 509.54, found:509.85.

**Example 81: 4-(4-(5-((2-(2,6-Dioxopiperidin-3-yl)-1,3-dioxoisoindole-4-yl)amino)methyl)oxazol-2-yl)methyl)piperazine-1-yl)-3-fluorobenzonitrile**

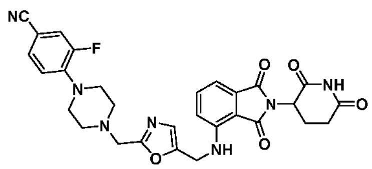

The compound of Example 81 was synthesized following the route described in Example 79. A yellow solid was obtained (48 mg, yield 19%).$^1$H NMR (500 MHz, DMSO) δ 11.09 (s, 1H), 7.66 (dd, J = 13.4, 1.9 Hz, 1H), 7.58 (dd, J = 8.3, 7.4 Hz, 1H), 7.55 (dd, J = 8.5, 1.7 Hz, 1H), 7.20 (d, J = 8.6 Hz, 1H), 7.11 - 7.03 (m, 4H), 5.05 (dd, J = 12.8, 5.4 Hz, 1H), 4.65 (d, J = 6.3 Hz, 2H), 3.67 (s, 2H), 3.16 - 3.09 (m, 4H), 2.88 (ddd, J = 17.1, 13.9, 5.4 Hz, 1H), 2.60 (d, J = 2.7 Hz, 1H), 2.58 - 2.55 (m, 4H), 2.52 (d, J = 4.5 Hz, 1H), 2.05 - 1.96 (m, 1H). $^{13}$C NMR (126 MHz, DMSO) δ 172.81, 170.06, 168.66, 167.20, 160.32, 154.11, 152.15, 149.60, 145.56, 143.75, 143.69, 136.10, 132.17, 129.89, 129.87, 124.29, 119.79, 119.59, 119.44, 119.42, 118.40, 117.68, 111.28, 110.02, 102.30, 102.22, 53.58, 51.80, 49.04, 48.58, 36.82, 30.97, 22.12. UPLC-MS ( ESI ) calculated for $C_{29}H_{26F}N_7O_5$ [M + H ]$^+$: 572.57, found:572.61.

**Example 82: 2-(2,6-Dioxopiperidin-3-yl)-4-(2-(Morpholinomethyl)oxazol-5-yl)methyl)amino)isoindole-1,3-dione**

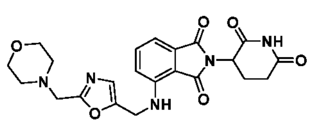

The compound of Example 82 was synthesized following the route described in Example 79. A yellow solid was obtained (5.4 mg, yield 7%).$^{1}$H NMR (600 MHz, DMSO) δ 11.10 (s, 1H), 7.58 (dd, J = 8.4, 7.2 Hz, 1H), 7.20 (d, J = 8.6 Hz, 1H), 7.08 (d, J = 7.0 Hz, 1H), 7.05 (d, J = 6.5 Hz, 2H), 5.06 (dd, J = 12.9, 5.4 Hz, 1H), 4.64 (d, J = 6.3 Hz, 2H), 3.78 - 3.58 (m, 2H), 3.51 (dd, J = 15.5, 12.3 Hz, 4H), 2.88 (ddd, J = 17.1, 13.9, 5.4 Hz, 1H), 2.62 - 2.57 (m, 1H), 2.53 (ddd, J = 6.0, 5.0, 3.7 Hz, 1H), 2.45 (dd, J = 23.4, 15.2 Hz, 4H), 2.03 (dtd, J = 7.5, 5.2, 2.1 Hz, 1H). $^{13}$C NMR (151 MHz, DMSO) δ 172.86, 170.10, 168.69, 167.25, 145.58, 136.15, 132.19, 124.38, 117.72, 111.35, 110.07, 69.80, 65.76, 52.50, 48.61, 36.83, 31.00, 22.15. UPLC-MS ( ESI ) calculated for $C_{22}H_{23}N_5O_6$ [M + H ]$^+$: 454.46, found:454.67.

**Example 83: 4-(4-(5-(((2-(2,6-Dioxopiperidin-3-yl)-1,3-dioxoisoindolyl-4-yl)amino)methyl)thiazol-2-yl)methyl)piperazine-1-yl)-3-fluorobenzonitrile**

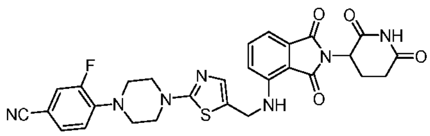

The compound 3-fluoro-4-(4-(5-formylthiazol-2-yl)piperazine-1-yl)methylbenzonitrile (83 mg, 0.303 mmol) and racemic Pomadime (96 mg, 0.303 mmol) were dissolved in 5 mL acetic acid, and the reaction mixture was refluxed at 130 °C overnight. After cooling to room temperature, NaBH4 (23 mg, 0.606 mmol) was added and the reaction was allowed to proceed for two hours. The mixture was concentrated under reduced pressure, and the resulting green solid (50 mg, yield 28.7%) was obtained via silica gel column chromatography. UPLC-MS ( ESI ) calculated for $C_{28}H_{24}FN_7O_4S$ [M + H ]$^+$: 574.60, found:574.44.

**Example 84: 4-(4-(5-(((2-(2,6-Dioxopiperidin-3-yl)-1,3-dioxoisoindole-4-yl)amino)methyl)oxazol-2-yl)methyl)piperazine-1-yl)-3-fluorobenzonitrile**

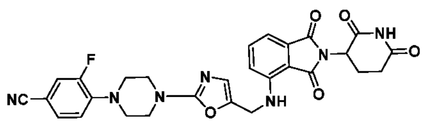

The compound of Example 84 was synthesized following the route described in Example 83.

**Example 85: 2-(2,6-Dioxopiperidin-3-yl)-4-(2-(3-Morpholinomethyl)oxazol-5-yl)methyl)amino)isoindole-1,3-dione**

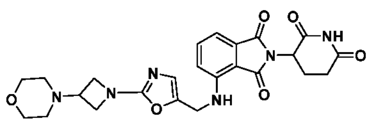

The compound of Example 85 was synthesized following the route described in Example 83.

**Example 86: 2-(2,6-Dioxopiperidin-3-yl)-4-(2-(3-Morpholinomethyl)thiazol-5-yl)methyl)amino)isoindole-1,3-dione**

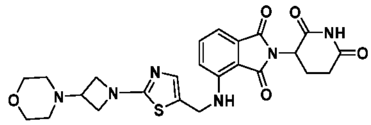

The compound of Example 86 was synthesized following the route described in Example 83.

**Example 87: 2-(2,6-Dioxopiperidin-3-yl)-4-(2-((4-Morpholinopiperazine-1-yl)methyl)oxazol-5-yl)methyl)amino) isoindole-1,3-dione**

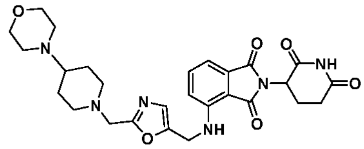

The compound of Example 87 was synthesized following the route described in Example 79. A yellow solid was obtained (65.8 mg, yield 23%).$^1$H NMR (500 MHz, DMSO) δ 11.13 (s, 1H), 7.60 (dd, J = 8.3, 7.3 Hz, 1H), 7.23 (s, 1H), 7.21 (d, J = 8.6 Hz, 1H), 7.11 (t, J = 6.6 Hz, 2H), 5.07 (dd, J = 12.9, 5.4 Hz, 1H), 4.68 (d, J = 6.0 Hz, 2H), 4.35 (s, 2H), 3.98 (dd, J = 14.9, 8.7 Hz, 2H), 3.75 - 3.59 (m, 2H), 3.53 - 3.26 (m, 5H), 3.18 - 3.00 (m, 2H), 2.95 - 2.79 (m, 3H), 2.63 - 2.57 (m, 1H), 2.53 (s, 1H), 2.23 (d, J = 11.6 Hz, 2H), 2.06 - 2.00 (m, 1H), 1.79 (dd, J = 24.6, 13.0 Hz, 2H). $^{13}$C NMR (201 MHz, DMSO) δ 172.84, 170.13, 168.68, 167.21, 145.47, 136.18, 132.20, 125.03, 117.60, 117.13, 115.66, 111.42, 110.10, 63.54, 50.36, 48.62, 40.43, 39.99, 36.75, 30.98, 22.13. UPLC-MS ( ESI ) calculated for $C_{27}H_{32}N_6O_6$ [M + H ]$^+$: 537.59, found:537.45.

**Example 88: 2-(2,6-Dioxopiperidin-3-yl)-6-fluoro-4-(2-phenyl-oxazol-5-yl)methyl)amino)isoindole-1,3-dione**

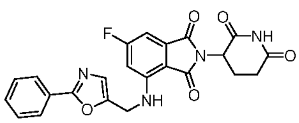

The compound of Example 88 was synthesized following the route described in Example 22.

**Example 89: 2-(2,6-Dioxopiperidin-3-yl-3-d)-4-((2-phenyl-oxazol-5-yl)methyl)amino)isoindole-1,3-dione**

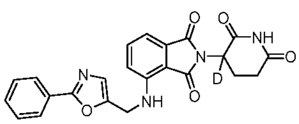

The compound of Example 89 was synthesized following the route described in Example 22.

**Example 90: 2-(3-Fluoro-2,6-Dioxopiperidin-3-yl)-4-((2-phenyl-oxazol-5-yl)methyl)amino)isoindole-1,3-dione**

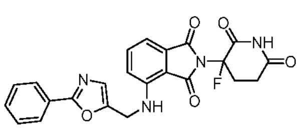

The compound of Example 90 was synthesized following the route described in Example 22.

**Example 91: 2-(2,6-Dioxopiperidine-3-yl)-4-((((2-((4-Methylpiperazine-1-yl)methyl)oxazol-5-yl)methyl)amino) isoindoline -1,3-dione**

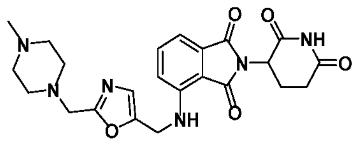

The compound of Example 91 was synthesized using the method outlined in Example 79. A yellow solid (21.1 mg) was obtained with a yield of 6%.$^1$H NMR (600 MHz, DMSO) δ 11.11 (s, 1H), 7.59 (dd, J = 8.5, 7.1 Hz, 1H), 7.20 (d, J = 8.6 Hz, 1H), 7.09 (d, J = 7.4 Hz, 1H), 7.08 (s, 1H), 7.05 (t, J = 6.1 Hz, 1H), 5.06 (dd, J = 12.9, 5.4 Hz, 1H), 4.64 (d, J = 5.6 Hz, 2H), 3.73 (s, 2H), 3.36 (d, J = 10.4 Hz, 2H), 2.98 (dd, J = 13.5, 10.0 Hz, 4H), 2.89 (ddd, J = 17.1, 14.0, 5.5 Hz, 1H), 2.76 (s, 3H), 2.63 - 2.57 (m, 1H), 2.56 - 2.51 (m, 1H), 2.45 (t, J = 10.4 Hz, 2H), 2.03 (dtd, J = 7.8, 5.3, 2.3 Hz, 1H). $^{13}$C NMR (151 MHz, DMSO) δ 172.85, 170.11, 168.71, 167.22, 159.73, 149.73, 145.54, 136.18, 132.20, 124.44, 117.60, 111.36, 110.03, 52.61, 52.44, 48.84, 48.61, 42.13, 36.79, 30.99, 22.14. UPLC-MS (ESI) calculated for $C_{23}H_{26}N_6O_S$ [M + H ]$^+$: 467.50, found:467.46.

**Example 92: 4-(((2-(2-Chlorophenyl)oxazol-5-yl)methyl)amino)-2-(2,6-Dioxopiperidine-3-yl) isoindoline -1,3-dione**

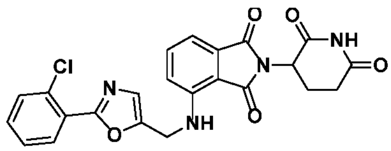

The compound of Example 92 was synthesized following the procedure of Example 22. A yellow solid (57.9 mg) was obtained with a yield of 52%.$^1$H NMR (500 MHz, DMSO) δ 11.08 (s, 1H), 7.92 (dd, J = 7.6, 1.8 Hz, 1H), 7.61 (dd, J = 12.5, 50 Hz, 2H), 7.52 (td, J = 7.7, 1.9 Hz, 1H), 7.48 (td, J = 7.5, 1.4 Hz, 1H), 7.31 (t, J = 4.0 Hz, 2H), 7.13 (t, J = 6.0 Hz, 1H), 7.10 (d, J = 7.1 Hz, 1H), 5.06 (dd, J = 12.7, 5.4 Hz, 1H), 4.76 (d, J = 4.8 Hz, 2H), 2.88 (ddd, J = 17.0, 13.9, 5.4 Hz, 1H), 2.64 - 2.52 (m, 2H), 2.03 (ddd, J = 10.3, 6.1, 3.9 Hz, 1H). $^{13}$C NMR (126 MHz, DMSO) δ 172.77, 170.01, 168.64, 167.20, 158.10, 150.38, 145.60, 136.11, 132.15, 131.81, 131.08, 131.05, 130.72, 127.60, 125.67, 125.60, 117.85, 111.37, 110.12, 48.57, 36.90, 30.95, 22.10. UPLC-MS ( ESI ) calculated for $C_{23}H_{17}ClN_4O_5$ [M + H ]$^+$: 465.86, found:465.33.

**Example 93: 2-(2,6-Dioxopiperidine-3-yl)-4-(((2-(4-Fluorophenyl)oxazol-5-yl)methyl)amino) isoindoline -1,3-dione**

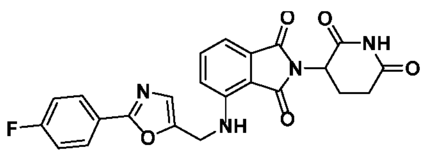

The compound of Example 93 was synthesized using the method of Example 22. A yellow solid (67.7 mg) was obtained with a yield of 58%.$^1$H NMR (500 MHz, DMSO) δ 11.09 (s, 1H), 7.98 - 7.94 (m, 2H), 7.62 (dd, J = 8.4, 7.3 Hz, 1H), 7.36 (t, J = 8.9 Hz, 2H), 7.30 (d, J = 8.6 Hz, 1H), 7.23 (s, 1H), 7.12 (t, J = 4.8 Hz, 1H), 7.10 (d, J = 7.1 Hz, 1H), 5.06 (dd, J = 12.7, 5.4 Hz, 1H), 4.73 (d, J = 4.8 Hz, 2H), 2.88 (ddd, J = 17.0, 13.9, 5.4 Hz, 1H), 2.57 (dt, J = 12.7, 9.1 Hz, 2H), 2.06 - 2.00 (m, 1H). $^{13}$C NMR (126 MHz, DMSO) δ 172.77, 170.02, 168.65, 167.20, 164.28, 162.30, 159.53, 149.91, 145.58, 136.14, 132.19, 129.63, 128.20, 128.13, 125.77, 123.56, 123.54, 117.70, 116.40, 116.23, 111.34, 110.10, 48.58, 36.92, 30.95, 22.10. UPLC-MS ( ESI ) calculated for $C_{23}H_{17}FN_4O_5$ [M + H ]$^+$: 449.41, found:449.35.

**Example 94: 2-(2,6-Dioxopiperidine-3-yl)-4-(((5-(3-Fluorophenyl)oxazol-2-yl)methyl)amino)isoindoline-1,3-dione**

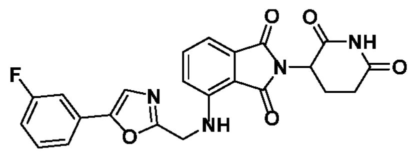

The compound of Example 94 was synthesized following the procedure of Example 22. A yellow solid (10.5 mg) was obtained with a yield of 10%.$^1$H NMR (500 MHz, DMSO) δ 11.11 (s, 1H), 7.72 (s, 1H), 7.62 (dd, J = 8.5, 7.2 Hz, 1H), 7.54 - 7.48 (m, 3H), 7.26 - 7.17 (m, 3H), 7.11 (d, J = 7.1 Hz, 1H), 5.09 (dd, J = 12.8, 5.4 Hz, 1H), 4.80 (d, J = 5.9 Hz, 2H), 2.89 (ddd, J = 16.6, 13.7, 5.2 Hz, 1H), 2.64 - 2.52 (m, 2H), 2.08 - 2.01 (m, 1H). $^{13}$C NMR (126 MHz, DMSO) δ 172.82, 170.06, 168.66, 167.24, 161.51, 161.43, 149.69, 149.67, 145.72, 136.17, 132.11, 131.44, 131.38, 129.56, 129.49, 123.82, 119.87, 119.86, 117.87, 115.35, 115.18, 111.49, 110.67, 110.48, 110.16, 48.62, 30.99, 22.12. UPLC-MS ( ESI ) calculated for $C_{23}H_{17}FN_4O_5$ [M + H ]$^+$: 449.41, found:449.31.

**Example 95: 2-(2,6-Dioxopiperidine-3-yl)-4-(((5-(2-Fluorophenyl)oxazol-2-yl)methyl)amino)isoindoline-1,3-dione**

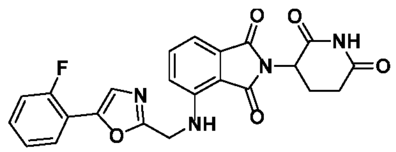

The compound of Example 95 was synthesized using the method of Example 22. A yellow solid (20.3 mg) was obtained with a yield of 17%. $^1$H NMR (500 MHz, DMSO) δ 11.11 (s, 1H), 7.70 (td, J = 7.7, 1.7 Hz, 1H), 7.62 (dd, J = 8.4, 7.2 Hz, 1H), 7.47 (d, J = 3.6 Hz, 1H), 7.46 - 7.41 (m, 1H), 7.40 - 7.31 (m, 2H), 7.25 (t, J = 6.5 Hz, 1H), 7.23 (d, J = 8.6 Hz, 1H), 7.11 (d, J = 7.1 Hz, 1H), 5.09 (dd, J = 12.8, 5.4 Hz, 1H), 4.83 (d, J = 6.3 Hz, 2H), 2.89 (ddd, J = 16.7, 13.7, 5.2 Hz, 1H), 2.63 - 2.51 (m, 2H), 2.05 (ddt, J = 12.7, 5.4, 2.6 Hz, 1H). $^{13}$C NMR (126 MHz, DMSO) δ 172.82, 170.07, 168.66, 167.24, 161.21, 158.85, 156.86, 145.74, 145.24, 145.23, 136.17, 132.11, 130.31, 130.25, 125.94, 125.85, 125.27, 125.25, 117.88, 116.33, 116.17, 115.51, 115.41, 111.50, 110.17, 48.62, 30.99, 22.12. UPLC-MS ( ESI ) calculated for $C_{23}H_{17}M_4O_5$ [M + H ]$^+$: 449.51, found:449.39.

**Example 96: 4-(((2-(Cyclopropoxyphenyl)oxazol-5-yl)methyl)amino)-2-(2,6-Dioxopiperidine-3-yl)isoindoline-1,3-dione**

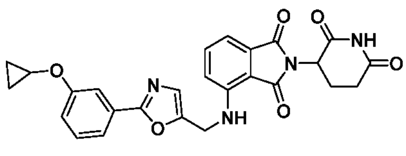

The compound of Example 96 was synthesized following the procedure of Example 22. A yellow solid (64.9 mg) was obtained with a yield of 61%. $^1$H NMR (500 MHz, DMSO) δ 11.11 (s, 1H), 7.62 (dd, J = 8.5, 7.2 Hz, 1H), 7.57 - 7.55 (m, 1H), 7.51 (d, J = 7.8 Hz, 1H), 7.43 (t, J = 8.0 Hz, 1H), 7.31 (d, J = 8.6 Hz, 1H), 7.23 (s, 1H), 7.18 - 7.13 (m, 2H), 7.09 (d, J = 7.1 Hz, 1H), 5.07 (dd, J = 12.8, 5.4 Hz, 1H), 4.74 (d, J = 3.3 Hz, 2H), 3.90 (ddd, J = 8.9, 5.9, 2.9 Hz, 1H), 2.88 (ddd, J = 17.0, 13.9, 5.4 Hz, 1H), 2.62 - 2.56 (m, 1H), 2.54 (dd, J = 8.5, 4.4 Hz, 1H), 2.06 - 1.99 (m, 1H), 0.80 (dt, J = 11.5, 5.8 Hz, 2H), 0.70 - 0.65 (m, 2H). $^{13}$C NMR (151 MHz, DMSO) δ 172.82, 170.06, 168.68, 167.23, 160.11, 158.98, 149.97, 145.62, 136.14, 132.21, 130.40, 128.04, 125.73, 118.41, 117.78, 117.53, 111.47, 111.36, 110.10, 50.90, 48.59, 36.95, 30.98, 22.12, 5.94. UPLC-MS (ESI) calculated for $C_{26}H_{22}N_4O_6$ [M + H ]$^+$: 487.48, found:487.31.

**Example 97: 4-(((2-(2-Cyclopropoxyphenyl)oxazol-5-yl)methyl)amino)-2-(2,6-Dioxopiperidine-3-yl)isoindoline-1,3-dione**

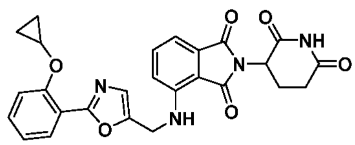

The compound of Example 97 was synthesized using the method of Example 22. A yellow solid (72.3 mg) was obtained with a yield of 68%. $^1$H NMR (500 MHz, DMSO) δ 11.11 (s, 1H), 7.75 (dd, J = 7.7, 1.5 Hz, 1H), 7.61 (dd, J = 8.4, 7.2 Hz, 1H), 7.51 - 7.47 (m, 1H), 7.44 (d, J = 7.7 Hz, 1H), 7.28 (d, J = 8.6 Hz, 1H), 7.20 (s, 1H), 7.13 - 7.04 (m, 3H), 5.07 (dd, J = 12.7, 5.4 Hz, 1H), 4.70 (d, J = 2.3 Hz, 2H), 3.90 (dq, J = 8.9, 2.8 Hz, 1H), 2.89 (ddd, J = 16.8, 13.8, 5.3 Hz, 1H), 2.59 (dd, J = 15.7, 3.7 Hz, 1H), 2.52 (d, J = 9.6 Hz, 1H), 2.03 (ddd, J = 10.1, 5.2, 3.1 Hz, 1H), 0.79 - 0.74 (m, 2H), 0.62 - 0.58 (m, 2H). $^{13}$C NMR (126 MHz, DMSO) δ 172.84, 170.05, 168.71, 167.26, 159.20, 156.46, 149.47, 145.65, 136.15, 132.19, 131.85, 129.84, 125.25, 120.95, 117.69, 116.14, 114.44, 111.27, 109.94, 51.31, 48.59, 40.43, 36.81, 30.98, 22.15, 6.02. UPLC-MS ( ESI ) calculated for $C_{26}H_{22}N_4O_6$ [M + H ]$^+$: 487.48, found:487.31.

**Example 98: 2-(2,6-Dioxopiperidine-3-yl)-4-(((5-Phenyl-1,3,4-oxadiazol-2-yl)methyl)amino)isoindoline-1,3-dione**

Synthetic Route:

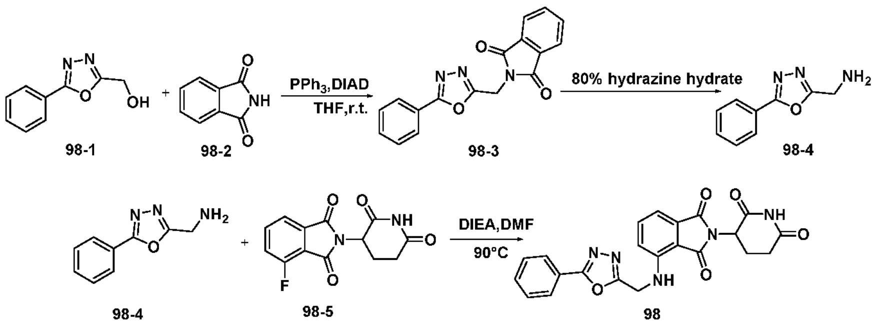


Step 1: Compound 98-1 (300 mg, 1.70 mmol), compound 98-2 (501 mg, 3.4 mmol), and triphenylphosphine (893 mg, 3.4 mmol) were dissolved in 10 mL of dry tetrahydrofuran (THF). DIAD (671 μL, 3.4 mmol) was added at room temperature under a nitrogen atmosphere. The reaction was allowed to proceed for 5 hours at room temperature. The reaction was monitored by TLC, and once it was complete, the mixture was concentrated under reduced pressure. The residue was purified by silica gel column chromatography to yield compound 98-3 as a pale yellow oil (600 mg). UPLC-MS (ESI) calculated for $C_{17}H_{11}N_3O_3$ [M + H ]$^+$: 306.08, found:306.15.

Step 2: Compound 98-3 (600 mg, 1.97 mmol) was dissolved in 8 mL of ethanol, and 80% hydrazine hydrate was added. The reaction was refluxed for 1 hour and monitored by LC-Mass. Once the reaction was complete, the mixture was cooled to room temperature, filtered, and the filtrate was concentrated under reduced pressure to yield the target compound 98-4 as a yellow oil (185 mg). calculated for calculated for $C_9H_9N_3O$ [M + H ]$^+$: 176.07, found:176.15.

Step 3: Compound 98-4 (86 mg, 0.49 mmol), compound 98-5 (147 mg, 0.53 mmol), and DIEA (160 μL, 0.97 mmol) were dissolved in 3 mL of DMF, and the reaction was heated to 90 °C overnight. The reaction was monitored by TLC, and once it was complete, the mixture was cooled to room temperature. The solution was then diluted with ethyl acetate and washed several times with saturated sodium chloride. The organic phase was dried over anhydrous sodium sulfate, filtered, and concentrated. The product was purified by HPLC to yield compound 98 as a yellow solid (13.1 mg), with a yield of 6%. $^1$H NMR (500 MHz, DMSO) δ 11.13 (s, 1H), 7.96 - 7.94 (m, 2H), 7.63 (dd, J = 9.3, 6.6 Hz, 2H), 7.61 (dd, J = 7.1, 4.6 Hz, 2H), 7.31 (t, J = 6.4 Hz, 1H), 7.27 (d, J = 8.6 Hz, 1H), 7.14 (d, J = 7.1 Hz, 1H), 5.09 (dd, J = 12.7, 5.4 Hz, 1H), 4.96 (d, J = 6.3 Hz, 2H), 2.89 (ddd, J = 16.8, 13.7, 5.2 Hz, 1H), 2.60 (dd, J = 15.5, 3.6 Hz, 1H), 2.54 (t, J = 6.8 Hz, 1H), 2.07 - 2.01 (m, 1H). $^{13}$C NMR (126 MHz, DMSO) δ 172.84, 170.07, 168.59, 167.22, 164.37, 145.46, 136.24, 132.15, 132.06, 129.60, 129.51, 126.93, 126.47, 123.25, 117.87, 111.74, 110.42, 48.64, 37.41, 30.99, 22.12. UPLC-MS ( ESI ) calculated for $C_{22}H_{17}N_5O_5$ [M + H ]$^+$: 432.41, found:432.21.

**Example 99: 2-(2,6-Dioxopiperidine-3-yl)-4-(((5-Phenyl-1,3,4-thiadiazol-2-yl)methyl)amino) isoindoline-1,3-dione**

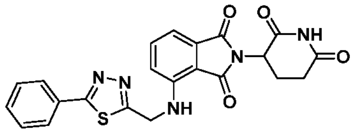

The synthetic route of Example 99 was the same as Example 98. Using (5-Phenyl-1,3,4-thiadiazol-2-yl) methylamine (200 mg, 1.04 mmol) and 2-(2,6-dioxopiperidine-3-yl)-4-fluoroindoline-1,3-dione (144 mg, 0.52 mmol) as starting materials, Example 98 was obtained. The product was purified by HPLC to yield 5 mg of yellow solid with a yield of 2%. $^{1}$H NMR (500 MHz, DMSO) δ 11.13 (s, 1H), 7.95 (dd, J = 7.9, 1.4 Hz, 2H), 7.62 - 7.59 (m, 3H), 7.55 - 7.53 (m, 2H), 7.17 (d, J = 8.6 Hz, 1H), 7.12 (d, J = 7.1 Hz, 1H), 5.09 (dd, J = 12.8, 5.4 Hz, 1H), 5.05 (d, J = 6.5 Hz, 2H), 2.90 (ddd, J = 16.8, 13.8, 5.3 Hz, 1H), 2.60 (dd, J = 15.5, 4.0 Hz, 1H), 2.54 (dd, J = 8.1, 5.5 Hz, 1H), 2.07 - 2.03 (m, 1H). $^{13}$C NMR (126 MHz, DMSO) δ 172.84, 170.02, 168.56, 167.19, 145.11, 136.39, 132.29, 132.06, 131.53, 131.45, 131.35, 129.43, 128.82, 128.72, 127.63, 117.57, 111.76, 110.46, 48.64, 41.44, 30.99, 22.12. UPLC-MS (ESI) calculated for $C_{22}H_{17}N_5O_4S$ [M + H ]$^+$: 448.47, found:448.20.

**Example 100: 2-(2,6-Dioxopiperidine-3-yl)-4-((((2-(3-(Oxetane-3-yl)phenyl)oxazol-5-yl)methyl)amino)isoindoline-1,3-dione**

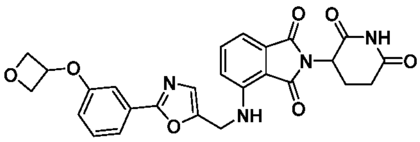

Following the synthetic route from Example 22, Example 100 was obtained. A yellow solid was obtained weighing 67.6 mg with a yield of 66%. $^{1}$H NMR (500 MHz, DMSO) δ 11.11 (s, 1H), 7.63 (dd, J = 8.4, 7.3 Hz, 1H), 7.52 (d, J = 7.7 Hz, 1H), 7.44 (t, J = 8.0 Hz, 1H), 7.30 (d, J = 8.6 Hz, 1H), 7.23 (d, J = 4.0 Hz, 2H), 7.15 (t, J = 5.9 Hz, 1H), 7.10 (d, J = 7.1 Hz, 1H), 6.94 (dd, J = 8.2, 2.3 Hz, 1H), 5.40 - 5.31 (m, 1H), 5.07 (dd, J = 12.8, 5.4 Hz, 1H), 4.93 (t, J = 6.7 Hz, 2H), 4.73 (d, J = 5.5 Hz, 2H), 4.56 (dd, J = 7.2, 5.0 Hz, 2H), 2.88 (ddd, J = 16.9, 13.9, 5.3 Hz, 1H), 2.62 - 2.55 (m, 1H), 2.54 - 2.51 (m, 1H), 2.02 (ddd, J = 7.3, 5.3, 2.9 Hz, 1H). $^{13}$C NMR (126 MHz, DMSO) δ 172.83, 170.08, 168.68, 167.23, 159.86, 156.66, 150.06, 145.60, 136.15, 132.22, 130.82, 128.35, 125.79, 118.75, 117.77, 116.77, 111.40, 110.12, 76.67, 70.12, 48.60, 36.94, 30.98, 22.12. UPLC-MS ( ESI ) calculated for $C_{26}H_{22}N_4O_7$ [M + H ]$^+$: 503.48, found:503.25.

**Example 101: 2-(2,6-Dioxopiperidine-3-yl)-4-(((2-(2-(Oxetane-3-yl)phenyl)oxazol-5-yl)methyl)amino) isoindoline-1,3-dione**

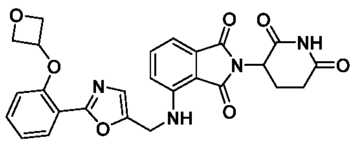

Following the synthetic route from Example 22, Example 101 was obtained. A yellow solid was obtained weighing 2.1 mg with a yield of 39%. $^{1}$H NMR (500 MHz, DMSO) δ 11.13 (s, 1H), 8.22 (s, 1H), 8.09 (dd, J = 8.2, 1.4 Hz, 1H), 7.77 - 7.72 (m, 1H), 7.70 - 7.65 (m, 1H), 7.39 - 7.33 (m, 3H), 7.28 (d, J = 8.4 Hz, 1H), 7.16 (d, J = 7.1 Hz, 1H), 5.09 (dd, J = 12.8, 5.4 Hz, 1H), 4.89 (d, J = 6.5 Hz, 2H), 4.82 (d, J = 15.1 Hz, 1H), 4.53 - 4.47 (m, 1H), 4.40 (dd, J = 15.1, 8.3 Hz, 1H), 3.85 (dd, J = 11.6, 4.8 Hz, 1H), 3.78 (dd, J = 11.6, 5.0 Hz, 1H), 2.89 (ddd, J = 17.2, 13.7, 5.2 Hz, 1H), 2.65 - 2.56 (m, 2H), 2.06 - 2.01 (m, 1H). $^{13}$C NMR (201 MHz, DMSO) δ 172.82, 170.07, 168.56, 167.17, 157.99, 157.91, 151.00, 145.06, 136.80, 136.34, 132.24, 129.43, 123.34, 121.53, 121.34, 117.78, 111.87, 110.56, 109.57, 77.64, 60.99, 52.53, 48.65, 36.53, 30.98, 22.13. UPLC-MS (ESI) calculated for $C_{26}H_{22}N_4O_7$ [M + H ]$^+$: 503.48,found:503.29.

**Example 102: 2-(2,6-Dioxopiperidine-3-yl)-4-((((2-(4-(Oxetane-3-yl)phenyl)thiazol-5-yl)methyl)amino) isoindoline-1,3-dione**

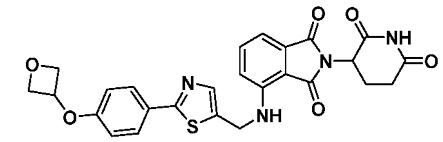

Following the synthetic route from Example 22, Example 102 was obtained. A yellow solid was obtained weighing 8.6 mg with a yield of 8%. $^1$H NMR (500 MHz, DMSO) δ 11.12 (s, 1H), 7.86 - 7.79 (m, 3H), 7.60 - 7.56 (m, 1H), 7.31 (t, J = 6.2 Hz, 1H), 7.21 (d, J = 8.6 Hz, 1H), 7.07 (d, J = 7.1 Hz, 1H), 6.87 (d, J = 8.8 Hz, 2H), 5.36 - 5.30 (m, 1H), 5.07 (dd, J = 12.8, 5.4 Hz, 1H), 4.93 (t, J = 6.7 Hz, 2H), 4.81 (d, J = 6.2 Hz, 2H), 4.55 (dd, J = 7.3, 5.0 Hz, 2H), 2.88 (ddd, J = 16.9, 13.9, 5.4 Hz, 1H), 2.62 - 2.52 (m, 2H), 2.07 - 2.00 (m, 1H). $^{13}$C NMR (126 MHz, DMSO) δ 172.84, 170.10, 168.62, 167.23, 166.34, 157.78, 145.40, 141.78, 136.91, 136.20, 132.28, 127.75, 126.68, 117.67, 115.13, 111.26, 110.05, 76.68, 70.15, 48.59, 38.47, 30.98, 22.13. UPLC-MS (ESI) calculated for $C_{26}H_{22}N_4O_6S$ [M + H ]$^+$: 519.54, found:519.22.

**Example 103: 4-(((2-(4-Cyclopropoxyphenyl)thiazol-5-yl)methyl)amino)-2-(2,6-Dioxopiperidine-3-yl)isoindoline-1,3-dione**

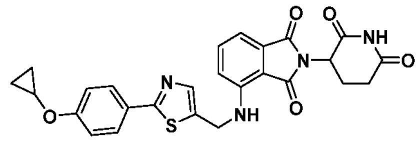

Following the synthetic route from Example 22, Example 103 was obtained. A yellow solid was obtained weighing 6.6 mg with a yield of 6%. $^1$H NMR (500 MHz, DMSO) δ 11.12 (s, 1H), 7.83 (s, 2H), 7.81 (d, J = 2.5 Hz, 1H), 7.59 (dd, J = 8.3, 7.4 Hz, 1H), 7.31 (t, J = 6.1 Hz, 1H), 7.22 (d, J = 8.6 Hz, 1H), 7.12 (d, J = 8.8 Hz, 2H), 7.07 (d, J = 7.1 Hz, 1H), 5.07 (dd, J = 12.8, 5.4 Hz, 1H), 4.81 (d, J = 5.9 Hz, 2H), 3.89 (ddd, J = 8.9, 6.0, 2.9 Hz, 1H), 2.89 (ddd, J = 17.0, 14.0, 5.4 Hz, 1H), 2.61 - 2.53 (m, 2H), 2.07 - 2.00 (m, 1H), 0.80 (q, J = 5.8 Hz, 2H), 0.69 - 0.64 (m, 2H). $^{13}$C NMR (201 MHz, DMSO) δ 172.83, 170.09, 168.61, 167.23, 166.57, 160.19, 145.40, 141.70, 136.71, 136.19, 132.28, 128.89, 127.48, 126.35, 117.67, 115.46, 111.25, 110.04, 51.02, 48.59, 38.47, 30.98, 22.13, 5.94. UPLC-MS ( ESI ) calculated for $C_{26}H_{22}N_4O_5S$ [M + H ]$^+$: 503.13, found:503.18.

**Example 104: 2-(2,6-Dioxopiperidine-3-yl)-4-((((2-(4-(2-Methoxyethoxy)phenyl)thiazol-5-yl)methyl)amino)isoindoline-1,3-dione**

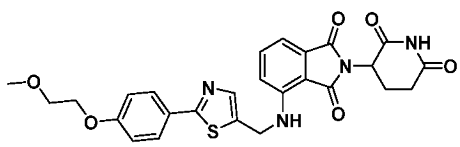

Following the synthetic route from Example 22, Example 104 was obtained. A yellow solid was obtained weighing 3.4 mg with a yield of 5%. $^1$H NMR (500 MHz, DMSO) δ 11.12 (s, 1H), 7.82 (s, 1H), 7.81 - 7.78 (m, 2H), 7.58 (dd, J = 8.3, 7.3 Hz, 1H), 7.31 (t, J = 6.2 Hz, 1H), 7.22 (d, J = 8.6 Hz, 1H), 7.07 (d, J = 7.0 Hz, 1H), 7.02 (t, J = 5.8 Hz, 2H), 5.07 (dd, J = 12.8, 5.5 Hz, 1H), 4.81 (d, J = 6.2 Hz, 2H), 4.13 (dd, J = 5.3, 3.7 Hz, 2H), 3.66 (dd, J = 5.3, 3.7 Hz, 2H), 3.30 (s, 3H), 2.89 (ddd, J = 17.3, 13.8, 5.3 Hz, 1H), 2.61 - 2.53 (m, 2H), 2.06 - 2.01 (m, 1H). $^{13}$C NMR (126 MHz, DMSO) δ 172.83, 170.10, 168.61, 167.23, 166.58, 160.01, 145.40, 141.70, 136.64, 136.18, 132.28, 127.52, 126.00, 117.68, 115.00, 111.25, 110.04, 70.25, 67.11, 58.18, 48.59, 38.47, 30.98, 22.13. UPLC-MS (ESI) calculated for $C_{26}H_{24}N_4O_6S$[M + H ]$^+$: 521.14, found:521.21.

**Example 105: 2-(2,6-Dioxopiperidine-3-yl)-4-(((2-((1S,4S)-4-(4-Fluorobenzyl)cyclohexyl)oxazol-5-yl)methyl)amino)isoindoline-1,3-dione**

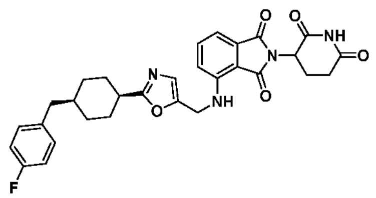

Following the synthetic route from Example 12, Example 105 was obtained. A yellow solid was obtained weighing 43.6 mg with a yield of 51%.$^1$H NMR (500 MHz, DMSO) δ 11.11 (s, 1H), 7.60 (dd, J = 8.5, 7.2 Hz, 1H), 7.22 (d, J = 8.6 Hz, 1H), 7.16 - 7.11 (m, 2H), 7.10 - 7.02 (m, 4H), 6.98 (s, 1H), 5.07 (dd, J = 12.7, 5.4 Hz, 1H), 4.62 (d, J = 1.4 Hz, 2H), 3.00 (p, J = 4.6 Hz, 1H), 2.88 (ddd, J = 16.9, 13.8, 5.4 Hz, 1H), 2.61 - 2.55 (m, 1H), 2.53 (d, J = 4.5 Hz, 1H), 2.39 (d, J = 7.4 Hz, 2H), 2.04 - 1.94 (m, 3H), 1.64 - 1.56 (m, 3H), 1.47 - 1.39 (m, 2H), 1.11 (td, J = 12.7, 2.7 Hz, 2H).

**Example 106: 2-(2,6-Dioxopiperidine-3-yl)-4-(((2-((1R,4R)-4-(4-Fluorobenzyl)cyclohexyl)oxazol-5-yl)methyl)amino)isoindoline-1,3-dione**

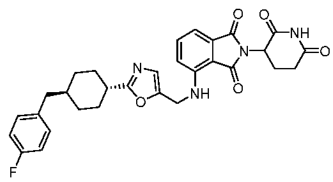

Following the synthetic route from Example 22, Example 106 was obtained. A yellow solid was obtained weighing 10.8 mg with a yield of 55%.$^1$H NMR (500 MHz, DMSO) δ 11.11 (s, 1H), 7.59 (dd, J = 8.4, 7.2 Hz, 1H), 7.18 (dd, J = 8.3, 6.2 Hz, 3H), 7.10 - 7.05 (m, 3H), 7.01 (t, J = 6.9 Hz, 1H), 6.93 (s, 1H), 5.06 (dd, J = 12.8, 5.5 Hz, 1H), 4.58 (d, J = 5.6 Hz, 2H), 2.88 (ddd, J = 17.2, 14.0, 5.4 Hz, 1H), 2.70 - 2.63 (m, 1H), 2.61 - 2.53 (m, 2H), 2.47 (d, J = 7.1 Hz, 2H), 2.06 - 1.94 (m, 3H), 1.67 (dd, J = 12.7, 1.9 Hz, 2H), 1.52 - 1.43 (m, 1H), 1.35 (ddd, J = 15.8, 13.2, 3.4 Hz, 2H), 1.04 (ddd, J = 15.7, 13.1, 3.3 Hz, 2H). UPLC-MS ( ESI ) calculated for $C_{30}H_{29}FN_4O_5$ [M + H ]$^+$: 545.48, found:545.32.

**Example Ctrl-1: 3-(1-oxo-4-(((2-(1-pivaloylpiperidin-4-yl)oxazol-5-yl)methyl)amino)isoindolin-2-yl)piperidine-2,6-dione**

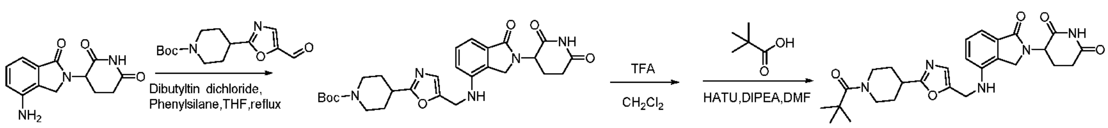


Lenalidomide (100 mg, 0.357 mmol), 4-(5-Formyloxazol-2-yl)piperidine-1-carboxylic acid tert-butyl ester (92 mg, 0.357 mmol), phenylsilane (44 μL, 0.357 mmol), and dibutyltin dichloride (108 mg, 0.357 mmol) were dissolved in 6 mL of tetrahydrofuran (THF). The mixture was heated to reflux and reacted overnight. After monitoring the reaction by TLC and confirming its completion, the reaction mixture was concentrated under reduced pressure, followed by column chromatography, yielding 4-(5-((2-(2,6-dioxopiperidine-3-yl)-1-oxoisoquinolin-4-yl)amino)methyl)oxazol-2-yl)piperidine-1-carboxylic acid tert-butyl ester as a colorless oily product (90 mg, 48% yield).

4-(5-((2-(2,6-dioxopiperidine-3-yl)-1-oxoisoquinolin-4-yl)amino)methyl)oxazol-2-yl)piperidine-1-carboxylic acid tert-butyl ester (90 mg, 0.17 mmol) was dissolved in 2 mL of dichloromethane. To this solution, 1 mL of trifluoroacetic acid was added, and the reaction was allowed to proceed for 30 minutes. Afterward, the mixture was concentrated under reduced pressure, and the crude product was dried under vacuum. The crude material was then dissolved in 2 mL of N,N-dimethylformamide (DMF), followed by the addition of trimethylacetic acid (19 mg, 0.187 mmol) and 2-(7-Azobenzotriazolyl)-N,N,N',N'-tetramethylurea hexafluorophosphate (71 mg, 0.187 mmol). The reaction was stirred, and N,N-diisopropylethylamine (296 μL, 1.7 mmol) was added. After stirring at room temperature for 1 hour, water was added to quench the reaction. The reaction mixture was extracted with ethyl acetate, separated, and the organic layer was washed several times with saturated sodium chloride solution. After drying, the mixture was filtered and concentrated, and the product was purified by HPLC to yield a white solid (7 mg, 8% yield). $^1$H NMR (400 MHz, DMSO) δ 11.01 (s, 1H), 7.28 (d, J = 7.7 Hz, 1H), 6.98 (d, J = 10.2 Hz, 2H), 6.88 (d, J = 8.0 Hz, 1H), 6.21 (s, 1H), 5.11 (d, J = 8.3 Hz, 1H), 4.40 (d, J = 5.3 Hz, 2H), 4.29 - 4.08 (m, 4H), 3.15 - 2.86 (m, 5H), 2.68 - 2.57 (m, 2H), 2.37 - 2.21 (m, 1H), 2.10 - 1.89 (m, 4H), 1.52 (dd, J = 21.5, 10.8 Hz, 2H), 1.18 (s, 9H). UPLC-MS ( ESI ) calculated for $C_{27}H_{33}N_5O_5$ [M + H ]$^+$:508.59, found:508.32.

**Example Ctrl-2: N-((2-(4-chlorophenyl)oxazol-5-yl)methyl)-N-(2-(2,6-dioxopiperidin-3-yl)-1,3-dioxoisoindolin-4-yl)formamide**

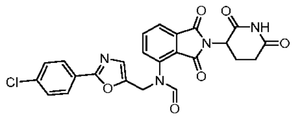

4-((2-(4-Chlorophenyl)oxazol-5-yl)methyl)amino)-2-(2,6-dioxopiperidine-3-yl)isoindole-1,3-dione (50 mg, 0.11 mmol) was dissolved in 1 mL of formic acid. To this solution, 300 μL of acetic anhydride was added, and the reaction mixture was heated to 60 °C and reacted for 1 hour. After monitoring the reaction by LC-Mass and confirming its completion, the mixture was cooled to room temperature. The product was purified by HPLC, yielding a yellow solid (14 mg, 26% yield). $^1$H NMR (400 MHz, DMSO) δ 11.14 (s, 1H), 8.51 (s, 1H), 7.90 (dd, J = 16.7, 7.2 Hz, 5H), 7.50 (d, J = 8.0 Hz, 2H), 7.18 (s, 1H), 5.30 - 5.19 (m, 2H), 5.18 - 5.06 (m, 1H), 2.94 - 2.79 (m, 1H), 2.63 (d, J = 31.6 Hz, 1H), 2.03 - 1.90 (m, 1H). UPLC-MS ( ESI ) calculated for $C_{24}H_{17}ClN_4O_6$ [M + H ]$^+$: 493.87, found:493.59.

**Example Ctrl-3: 4-(((2-(4-chlorophenyl)oxazol-5-yl)methyl)(methyl)amino)-2-(2,6-dioxopiperidin-3-yl)isoindoline-1,3-dione**

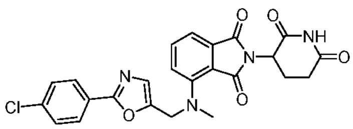

1-(2-(4-Chlorophenyl)oxazol-5-yl)-N-methylmethanamine (100 mg, 0.45 mmol) was used as the starting material. It was dissolved in 5 mL of DMSO, and 2-(2,6-dioxopiperidine-3-yl)-4-fluoroisoindole-1,3-dione (101 mg, 0.367 mmol) was added. To the mixture, N,N-diisopropylethylamine (182 μL, 0.367 mmol) was added under stirring. The reaction was heated to 100 °C and stirred overnight. After confirming the completion of the reaction, the mixture was diluted with ethyl acetate, washed successively with water, saturated brine, and dried over anhydrous sodium sulfate. The solvent was removed under reduced pressure, and the product was purified by HPLC to yield a yellow-green solid (130 mg, 74% yield). $^1$H NMR (400 MHz, DMSO) δ 11.11 (s, 1H), 7.95 (d, J = 8.4 Hz, 2H), 7.68 (t, J = 7.7 Hz, 1H), 7.50 (d, J = 8.1 Hz, 2H), 7.35 (t, J = 8.2 Hz, 2H), 7.19 (s, 1H), 5.14 (dd, J = 12.2, 4.9 Hz, 1H), 4.96 (q, J = 16.4 Hz, 2H), 3.06 (s, 3H), 2.89 (t, J = 13.1 Hz, 1H), 2.60 (d, J = 16.3 Hz, 2H), 2.18 - 1.96 (m, 1H). UPLC-MS ( ESI ) calculated for $C_{24}H_{19}ClN_4O_5$ [M + H ]$^+$:479.89, found:479.63.

**Example Ctrl-4: 3-(4-((1-(4-Chlorophenyl)-1H-1,2,3-triazol-4-yl)methyl)amino)-1-oxisoquinolin-2-yl)piperidine-2,6-dione**

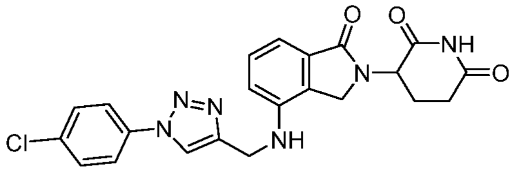

1-Azido-4-chlorobenzene and 3-(1-oxy-4-(prop-2-in-1-oxy)isoindole-2-yl)piperidine-2,6-dione were reacted via a click reaction to obtain Example Ctrl-4. $^{1}$H NMR (400 MHz, DMSO) δ 11.03 (s, 1H), 8.76 (s, 1H), 7.94 (d, J = 8.9 Hz, 2H), 7.66 (d, J = 8.9 Hz, 2H), 7.29 (t, J = 7.8 Hz, 1H), 6.98 (d, J = 7.3 Hz, 1H), 6.88 (d, J = 8.0 Hz, 1H), 6.30 (s, 1H), 5.13 (dd, J = 13.3, 5.1 Hz, 1H), 4.52 (s, 2H), 4.29 (d, J = 17.2 Hz, 1H), 4.18 (d, J = 17.2 Hz, 1H), 2.99 - 2.88 (m, 1H), 2.67 - 2.58 (m, 1H), 2.30 (ddd, J = 26.5, 13.3, 4.4 Hz, 1H), 2.08 - 1.99 (m, 1H). UPLC-MS ( ESI ) calculated for $C_{22}H_{19}ClN_6O_3$ $[M + H]^+$:451.88, found:451.70.

**2. Test Implementation Examples**

This invention also tested the activity of the multi-substituted isoindoline compounds from the implementation examples on three major types of hematologic tumor cell lines. The representative cell lines include: multiple myeloma cell line (MM.1S), mantle cell lymphoma cell line (Mino), and acute myeloid leukemia cell line (MV-4-11). The proliferation inhibition activity of the compounds on these three representative cell lines was evaluated. In this invention, compounds 39, 40, and 41 from the implementation examples are prodrug compounds of the corresponding compounds after ester bond hydrolysis. Except for special instructions, the experimental materials required for pharmacological experiments were commercially available.

**1. Effect of the Compound on MM.1S Cell Proliferation**

MM.1S cells were cultured in 1640 medium supplemented with 10% fetal bovine serum, then collected and diluted to the desired cell concentration after 7 days of treatment. A total of 180 μL of cell suspension was added to each well in a 96-well cell plate, ensuring 20,000 cells per well. For the control group, 20 μL of 0.2% DMSO was added to each well. Compounds were diluted in a 5-step gradient from a 10 mM stock solution, and 20 μL of each dilution was added to the compound-containing wells (with a final DMSO concentration of 0.2%). The cells were incubated in a 37 °C, 5% CO2 incubator for 7 days. Afterward, 20 μL of the MTS reagent (Promega, G5430) was added to each well, followed by incubation for 3-4 hours in a 37 °C, 5% CO2 incubator. Absorbance at 490 nm was measured using a microplate reader, with 690 nm absorbance as the background. The final raw data was calculated as OD490 - OD690. The compound inhibition rate was calculated using the formula:

$$\text{Inhibition rate} = (\text{OD_DMSO} - \text{OD_compound}) / (\text{OD_DMSO} - \text{OD_blank}) \times 100\%.$$

The IC50 for the compound's proliferative inhibition was fitted using GraphPad Prism 5.0. The experiment was repeated three times, and the average and standard deviation were calculated using three parallel experiments per trial.

The results of the cell viability test are shown in Table 1: A: Cell activity $IC_{50}$ < 15 nM, B: Cell activity 15 nM < $IC_{50}$ < 30 nM, C: Cell activity 30 nM < $IC_{50}$ < 150 nM, D: Cell activity $IC_{50}$ > 150 nM.

**Table 1. Inhibition Activity of Compounds on MM.1S Cell Proliferation**

| Compound Number | MM.1S Cells Inhibition Activity $IC_{50}$ | Compound Number | MM.1S Cells Inhibition Activity $IC_{50}$ |
|---|---|---|---|
| 1 | A | 53 | A |
| 2 | A | 54 | A |
| 3 | A | 55 | B |
| 4 | A | 56 | C |
| 5 | B | 57 | A |
| 6 | C | 58 | B |
| 7 | B | 59 | C |
| 8 | B | 60 | B |

(continued)

| Compound Number | MM.1S Cells Inhibition Activity $IC_{50}$ | Compound Number | MM.1S Cells Inhibition Activity $IC_{50}$ |
|---|---|---|---|
| 9 | A | 61 | C |
| 10 | A | 62 | D |
| 11 | A | 63 | B |
| 12 | C | 64 | A |
| 13 | C | 65 | C |
| 14 | B | 66 | C |
| 15 | A | 69 | C |
| 16 | A | 70 | B |
| 17 | A | 71 | A |
| 18 | A | 72 | A |
| 19 | A | 73 | B |
| 20 | A | 74 | A |
| 21 | A | 76 | C |
| 22 | A | 77 | C |
| 23 | A | 79 | D |
| 24 | B | 80 | D |
| 25 | A | 81 | C |
| 26 | A | 82 | D |
| 27 | A | 87 | C |
| 28 | C | 91 | D |
| 29 | A | 92 | A |
| 30 | B | 93 | A |
| 31 | A | 94 | C |
| 32 | A | 95 | C |
| 33 | A | 96 | A |
| 34 | C | 97 | A |
| 35 | A | 98 | B |
| 36 | B | 99 | C |
| 37 | A | 100 | A |
| 39 | B | 102 | C |
| 40 | C | 103 | A |
| 41 | B | 104 | B |
| 43 | A | 105 | D |
| 44 | C | 106 | C |
| 45 | B | Lenalidomide | C |
| 46 | B | Pomalidomide | C |
| 48 | A | CC-220 | A |
| 49 | B | | |
| 50 | A | | |

(continued)

| Compound Number | MM.1S Cells Inhibition Activity $IC_{50}$ | Compound Number | MM.1S Cells Inhibition Activity $IC_{50}$ |
|---|---|---|---|
| 51 | A | | |
| 52 | A | | |

Based on the cell growth inhibition test results of the compounds mentioned above, some embodiments of the compounds in this invention exhibited excellent inhibition activity against multiple myeloma MM.1S cell growth. The majority of the compounds showed better activity than the marketed drugs Lenalidomide or Pomalidomide, and were comparable to the currently clinical investigational compound CC-220. On the other hand, the development of these structurally diverse compounds provided a broader material basis and new structures for obtaining drug molecules with higher activity and better pharmacological properties. Therefore, the compounds of this invention could be used for the prevention and treatment of diseases related to the regulation of CRBN (CRL4CRBN E3 ubiquitin ligase) activity, such as multiple myeloma or other potential tumor diseases, pain, neurological disorders, and immune system diseases.

**2. Inhibition of Cell Proliferation of Mino Cells by the Compounds**

Mino cells were cultured with RPMI-1640 medium supplemented with 15% fetal bovine serum, and collected. The cell concentration was diluted according to a 3-day treatment period, and 90 μL of the cell suspension was added to each well of a 96-well plate, resulting in 8000 cells per well. For control wells, 10 μL of DMSO at a final concentration of 0.2% was added. The compounds were diluted in a 5-fold gradient from a 10 mM stock solution, and 10 μL of each compound solution was added to the experimental wells (with a final DMSO concentration of 0.2%). The cells were incubated in a 37 °C, 5% CO2 incubator for 3 days. After preparing the reaction mixture using the MTS reagent kit (Promega, G5430), 20 μL was added to each well, and the cells were incubated for 3-4 hours at 37 °C, 5% CO2.

The absorbance at 490 nm was read using a microplate reader, with the absorbance at 690 nm used as the background. The final raw data was calculated as OD490 - OD690. The inhibition rate of the compounds was calculated using the formula: Inhibition rate = (OD DMSO - OD compound) / (OD DMSO - OD blank) × 100%. The IC50 values for cell proliferation inhibition of the compounds were fitted using GraphPad Prism 5.0. The experiment was repeated three times, with the average and standard deviation calculated from three parallel experiments each time.

The cell activity test results are shown in Table 2: A indicates $IC_{50}$ < 60 nM, B indicates 60 nM < $IC_{50}$ < 120 nM, C indicates $IC_{50}$ > 120 nM.

**Table 2. Inhibition of Cell Proliferation of Mino Cells by the Compounds**

| Compound Number | Mino Cells Inhibition Activity $IC_{50}$ | Compound Number | Mino Cells Inhibition Activity $IC_{50}$ |
|---|---|---|---|
| 1 | A | 53 | A |
| 2 | A | 54 | A |
| 3 | A | 57 | C |
| 4 | A | 58 | C |
| 5 | C | 59 | C |
| 6 | B | 60 | C |
| 7 | C | 61 | C |
| 8 | B | 62 | C |
| 9 | C | 63 | C |
| 10 | B | 64 | C |
| 11 | A | 65 | C |
| 12 | C | 66 | C |
| 13 | C | 68 | C |
| 14 | B | 69 | C |

(continued)

| Compound Number | Mino Cells Inhibition Activity $IC_{50}$ | Compound Number | Mino Cells Inhibition Activity $IC_{50}$ |
|---|---|---|---|
| 15 | B | 71 | A |
| 16 | A | 72 | C |
| 17 | B | 73 | C |
| 18 | A | 74 | C |
| 19 | A | 75 | C |
| 20 | A | 76 | C |
| 21 | C | 77 | C |
| 22 | A | 79 | C |
| 23 | C | 80 | C |
| 24 | C | 81 | C |
| 25 | A | 82 | C |
| 26 | A | 87 | C |
| 27 | B | 91 | C |
| 28 | C | 92 | C |
| 29 | C | 93 | A |
| 30 | C | 94 | C |
| 31 | A | 95 | C |
| 32 | B | 96 | C |
| 33 | B | 97 | C |
| 34 | C | 98 | C |
| 35 | A | 99 | C |
| 36 | C | 100 | C |
| 37 | A | 103 | C |
| 39 | B | 104 | C |
| 40 | C | 105 | C |
| 41 | C | 106 | C |
| 43 | C | Lenalidomide | C |
| 44 | C | Pomalidomide | C |
| 45 | A | CCC-220 | B |
| 46 | C | | |
| 48 | A | | |
| 49 | A | | |
| 50 | A | | |
| 51 | A | | |
| 52 | A | | |

Based on the cell growth inhibition activity test results of the compounds in the above examples, some of the compounds in this invention showed good inhibitory activity against the growth of Mino cells, a model of mantle cell lymphoma. The majority of these compounds exhibited better activity than the marketed drugs, Lenalidomide or Pomalidomide, and most of the compounds in this invention performed better than the currently clinically researched

compound, CC-220. On the other hand, the development of these structurally diverse compounds provides a foundation and source of molecules for obtaining higher activity and better pharmacological properties. Therefore, the compounds of this invention broaden the application range of thalidomide-related drugs in the treatment of hematologic malignancies, expanding their potential use to other indications in hematologic cancers. For example, they can serve as active molecules for the treatment and prevention of mantle cell lymphoma or be used as diagnostic reagents for such diseases. Consequently, the compounds of this invention can act as potent novel CRBN modulators for the prevention and treatment of diseases related to CRBN (CRL4CRBNE3 ubiquitin ligase) activity, such as multiple myeloma, mantle cell lymphoma, and potentially other tumor diseases, as well as pain, neurological disorders, and immune system diseases.

### 3. Inhibition of MV-4-11 Cell Proliferation by the Compounds

MV-4-11 cells were cultured in IMDM with 10% fetal bovine serum, collected, and diluted to the appropriate cell concentration for a 7-day treatment period. A 96-well plate was used, and 180 μL of cell suspension was added to each well, resulting in a cell count of 2,000 per well. For the control wells, 20 μL of DMSO at a final concentration of 0.2% was added. The compounds were diluted from a 10 mM stock solution in a five-fold gradient, and 20 μL was added to the compound wells (with a final DMSO concentration of 0.2%). The cells were incubated in a 37 °C, 5% CO2 incubator for 7 days. After preparing the reaction solution using the MTS reagent kit (Promega, G5430), 20 μL was added to each well, and the cells were incubated in the incubator for 3-4 hours. The absorbance at 490 nm was measured using a microplate reader, with the absorbance at 690 nm as the background value. The final data was calculated as OD490 - OD690. The inhibition rate of the compounds was calculated using the formula: Inhibition Rate = (OD_DMSO - OD_compound) / (OD_DMSO - OD_blank) × 100%. The IC50 values for proliferation inhibition were calculated using GraphPad Prism 5.0. The experiments were repeated three times, with three parallel experiments for each, and the mean and standard deviation were calculated.

The cell activity test results are shown in Table 3: A indicates $IC_{50}$ < 1 μM, B indicates 1 μM ≤ $IC_{50}$ ≤ 20 μM, and C indicates $IC_{50}$ > 20 μM.

**Table 3. Inhibition of MV-4-11 Cell Proliferation by the Compounds**

| Compound Number | MV-4-11 Cells Inhibition Activity $IC_{50}$ | Compound Number | MV-4-11 Cells Inhibition Activity $IC_{50}$ |
|---|---|---|---|
| 1 | B | 53 | A |
| 2 | A | 54 | A |
| 3 | B | 55 | A |
| 4 | A | 56 | B |
| 5 | B | 57 | A |
| 6 | B | 58 | A |
| 7 | B | 59 | B |
| 8 | A | 60 | B |
| 9 | A | 61 | C |
| 10 | B | 62 | B |
| 11 | A | 63 | A |
| 12 | A | 64 | A |
| 13 | B | 65 | A |
| 14 | A | 66 | A |
| 15 | A | 68 | A |
| 16 | A | 69 | A |
| 17 | B | 70 | B |
| 18 | A | 71 | A |
| 19 | A | 72 | A |
| 20 | A | 73 | A |

(continued)

| Compound Number | MV-4-11 Cells Inhibition Activity $IC_{50}$ | Compound Number | MV-4-11 Cells Inhibition Activity $IC_{50}$ |
|---|---|---|---|
| 21 | B | 74 | B |
| 22 | A | 75 | A |
| 23 | A | 76 | A |
| 24 | A | 77 | B |
| 25 | A | 79 | A |
| 26 | A | 80 | A |
| 27 | A | 81 | B |
| 28 | B | 82 | A |
| 29 | A | 87 | C |
| 30 | B | 91 | B |
| 31 | A | 92 | A |
| 32 | A | 93 | A |
| 33 | A | 94 | C |
| 34 | A | 95 | C |
| 35 | A | 96 | A |
| 36 | A | 97 | B |
| 37 | A | 98 | B |
| 39 | A | 99 | B |
| 40 | A | 100 | B |
| 41 | B | 101 | B |
| 43 | A | 102 | B |
| 44 | C | 103 | B |
| 45 | A | 104 | A |
| 46 | B | 105 | B |
| 48 | A | 106 | A |
| 49 | A | Lenalidomide | C |
| 50 | A | Pomalidomide | C |
| 51 | A | CCC-220 | C |
| 52 | A | | |

Based on the cell growth inhibition activity test results of the compounds in the above examples, some of the compounds in this invention showed very good inhibitory activity against the acute leukemia MV-4-11 cells. Several compounds exhibited $IC_{50}$ values in the nanomolar range, with the best activity of the compounds in the table reaching $IC_{50}$ < 100 nM. However, positive compounds, including marketed drugs such as Lenalidomide or Pomalidomide, and currently clinically researched compounds such as CC-220 or CC-122, all showed $IC_{50}$ values greater than 20 μM in MV-4-11 acute leukemia cells. Therefore, the compounds of this invention, through the design of intramolecular hydrogen bond formation and optimization of substituents, achieved unexpected results in MV-4-11 cells, with activity much superior to the marketed drugs and currently researched drugs.

### 4. Pharmacokinetic Experiments

**Mouse Pharmacokinetic Experimental Scheme** ICR mice, female, 18-22 g, randomly divided into groups with

4 mice per group. The test compounds were administered by oral gavage at a dose volume of 10 mL/kg. The oral solution was prepared using DMSO/PEG-400/water (5:30:65, v/v/v). Mice were fasted for 12 hours prior to the experiment with free access to water. After drug administration, they were allowed to eat uniformly at 2 hours post-dose. The test compounds (20 mg/kg) were orally administered to 4 mice. Blood samples (50 μL) were collected from the orbital vein at the following time points: 5 min, 15 min, 30 min, 1 h, 2 h, 4 h, 6 h, and 24 h. The blood was centrifuged at 12,000 rpm for 2 minutes to separate plasma, which was then stored at -20 °C for blood concentration measurement.

The pharmacokinetic test results showed that compounds capable of forming intramolecular hydrogen bonds (Class C1), such as compounds 22, 37, 45, and 51, exhibited Cmax (ng/mL) > 2000 and AUClast (h*ng/mL) > 10000. These pharmacokinetic properties were significantly superior to structurally comparable compounds without intramolecular hydrogen bonds (Class B1 or B2). Similar results were not limited to the above-mentioned examples.

The compounds of this invention demonstrated better pharmacokinetics, and the compounds tested in this invention were superior to those disclosed in prior art that lacked intramolecular hydrogen bonds. These examples demonstrate the advantages of forming intramolecular hydrogen bonds, but the invention is not limited to these examples, as other compounds in this invention may also exhibit similar advantages.

## Discussion

During the research process, the inventors unexpectedly discovered that the compounds of Formula I in this invention exhibited excellent anti-cell proliferation activity and pharmacokinetic properties. Through computational and activity testing, it was found that the substitution at the 4-position amino group of the Pomalidomide core structure, where the hydrogen on the amino group can form a stable six-membered ring intramolecular hydrogen bond with the oxygen atom on the indoline ring (C1-type compounds, such as compounds 25, 9, etc., see Figure 1), plays a key role. This intramolecular hydrogen bond imposes certain structural constraints on the molecule, and in turn, the Pomalidomide core structure forms an optimal dihedral angle with the five-membered heteroaryl ring through the aliphatic linkage (as shown in Figure 1). As a result, the five-membered heteroaryl group and its substituent group can effectively interact with the hydrophobic pocket of the target protein, significantly improving the cell activity of such compounds. Moreover, molecular activity testing showed that when the 4-position amino group did not contain a hydrogen bond donor (hydrogen atom) (compounds D1-D2, where the NH group was substituted with methyl or formyl groups), or when the five-membered heteroaryl ring was replaced by other groups such as a six-membered aromatic ring, six-membered heteroaryl ring, or cyclohexyl group, the activity was not satisfactory (Table 4). As seen in Table 4, compounds with an amino group at the 4-position capable of forming an intramolecular hydrogen bond with the oxygen atom on the indoline ring (such as Examples 9, 15, 25, etc.) exhibited significantly higher activity than compounds that could not form such hydrogen bonds (e.g., Examples Ctrl-1 to Ctrl-4). Similar examples are not limited to those in Table 4.Further in vivo metabolism experiments verified that C1-type compounds, which can form intramolecular hydrogen bonds, exhibited significantly improved drug metabolism properties in the metabolic tests.

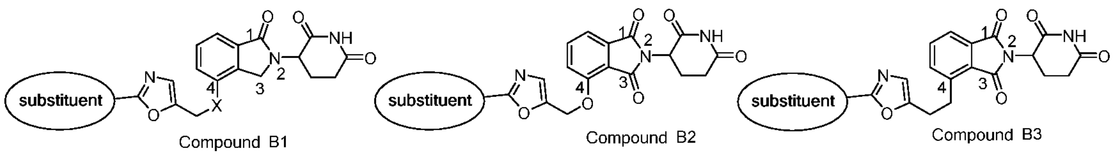

Compound B1 Compound B2 Compound B3

X=O or NH

Compounds with O or C substitution at the 4-position

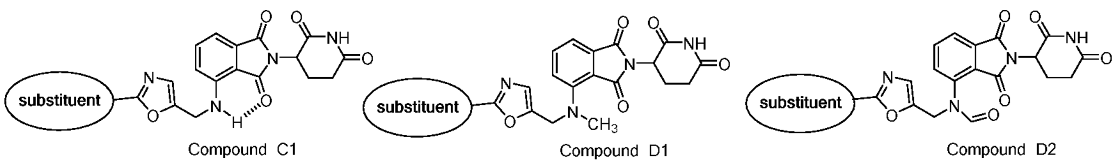

Compound C1 Compound D1 Compound D2

Can form intramolecular hydrogen bonding.

No intramolecular hydrogen bonding

The inventors analyzed the crystal structures of the protein complexes of Lenalidomide, CC-885, CC-220 with CRBN (PDB IDs: 4CI2, 5HXB, 5V3O). The side chains of both CC-220 and CC-885 molecules interact with a groove, with the terminal fragments pointing toward a nearby hydrophobic pocket. The F150 residue in the hydrophobic pocket is located adjacent to the fragments of CC-220 and CC-885. By comparing the crystal structure of Lenalidomide with CRBN, it was found that the position of F150 had shifted, which might be a result of subtle conformational adjustments in CRBN protein, leading to the degradation of different substrates. Based on the crystal structure analysis, it was speculated that

the conformation of F150 is easily altered due to the high flexibility of the β-loop region, which can be influenced by external forces. Therefore, the CRL4CRBN E3 ligase molecular glue degraders of Formula I in this application, due to their specific dihedral angle structure, significantly enhance the binding affinity with CRBN and may potentially generate new degradation substrates, thereby greatly improving tumor treatment efficacy and enabling new indications for therapy.

Additionally, the inventors employed molecular dynamics simulation methods to analyze the structural dynamics and binding site interactions between the model molecules and the E3 ligase interface. By combining molecular docking and a pharmacophore matching strategy based on the complex, and using scoring functions to evaluate the binding modes and interactions of the compounds at the E3 ligase active site, the inventors performed computational simulations and optimizations in structural design. This led to the discovery of novel, specific small-molecule modulators for $CRL4^{CRBN}$ E3 ligase.

**Table 4. Comparison of the Impact of Intramolecular Hydrogen Bond Formation on Compound Activity**

| Compoun ds Number | Compound Structure | Conditions for Intramolecul ar Hydrogen Bond Formation | Inhibitory Activity of Compounds on Cell Proliferation** | | |
|---|---|---|---|---|---|
| | | | MM.1S | Mino | MV-4-11 |
| **Example 9** | 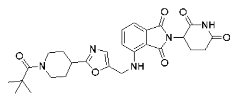 | Present | $IC_{50}$ = 8.8 nM | $IC_{50}$ = 141.2 nM | $IC_{50}$ = 781.7 nM |
| **Example Ctrl-1** | 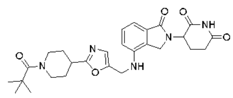 | Absent | $IC_{50}$ = 419.5 nM | $IC_{50}$ > 20000 nM | $IC_{50}$ = 14374 nM |
| **Example 15** | 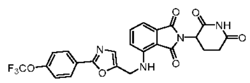 | Present | $IC_{50}$ = 6.4 nM | $IC_{50}$ = 60.5 nM | $IC_{50}$ = 750 nM |
| **Prior Art Dis-closed Compoun d-1** | 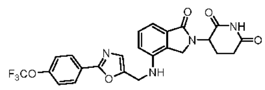 | Absent | $IC_{50}$ > 20 nM | $IC_{50}$ > 4800 nM | $IC_{50}$ > 20000 nM |
| **Prior Art Dis-closed Compoun d-2** | 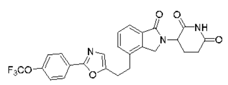 | Absent | $IC_{50}$ = 73.0 nM | $IC_{50}$ = 800.0 nM | $IC_{50}$ > 20000 nM |
| **Prior Art Dis-closed Compoun d-3** | 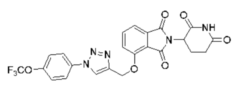 | Absent | $IC_{50}$ > 20000 nM | $IC_{50}$ = 680.0 nM | $IC_{50}$ > 20000 nM |
| **Prior Art Dis-closed Compoun d-4** | 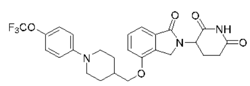 | Absent | $IC_{50}$ > 41 nM | $IC_{50}$ = 7500 nM | $IC_{50}$ > 10000 nM |
| **Example 25** | 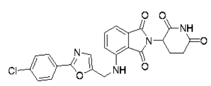 | Present | A $IC_{50}$ < 15 nM | A $IC_{50}$ < 60 nM | A $IC_{50}$ < 1 μM |
| **Example Ctrl-2** | 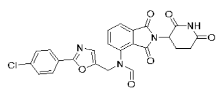 | Absent | C $IC_{50}$ > 30 nM | C IC 50 **>** 120 nM | B 1μM≤$IC_{50}$≤ 20 μM |
| **Example Ctrl-3** | 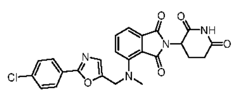 | Absent | C $IC_{50}$ > 30 nM | C IC 50 **>** 120 nM | B 1μM≤$IC_{50}$≤ 20 μM |
| **Example Ctrl-4** | 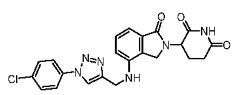 | Absent | $IC_{50}$ > 15 nM | $IC_{50}$> 5000 nM | $IC_{50}$> 20000 nM |

It can be seen that the test results of the compounds in this application in multiple myeloma cell line (MM.1S), mantle cell lymphoma cell line (Mino), and acute myeloid leukemia cell line (MV-4-11) indicated that some of the novel small-molecule modulators exhibited better cell inhibition activity compared to the already marketed or clinical-stage drugs, such as Lenalidomide, Pomalidomide, and CC-220. Particularly in the acute myeloid leukemia cell line (MV-4-11), Lenalidomide, Pomalidomide, and CC-220 showed poor activity, demonstrating micromolar-level activity, whereas some of the compounds designed in this invention had IC50 values in the nanomolar range, with the best active compound in the cell activity test reaching an IC50 of <100 nM. Compared to compounds in the prior art, the best active compound could improve activity by more than 100-fold. Therefore, the compounds in this invention broadened the application scope in the treatment of hematological malignancies and can serve as candidate drug molecules for new indications in blood cancers.

In summary, the compounds in this invention expanded the application range of immunomodulatory drugs in the treatment of hematological malignancies and can be applied to other indications of blood cancers, such as being inhibitors for acute leukemia and as drug candidates for treating these diseases. Therefore, the compounds in this invention can serve as potent new CRBN modulators for the prevention and treatment of diseases related to the regulation of CRBN ($CRL4^{CRBN}$ E3 ligase) activity, such as multiple myeloma, mantle cell lymphoma, acute leukemia, and potentially other tumor diseases, as well as pain, neurological diseases, and immune system diseases.

All references cited in the documents mentioned in this invention are incorporated by reference into this application as if each reference were individually cited. Furthermore, it should be understood that, after reading the above teachings of the invention, those skilled in the art can make various modifications or alterations to the invention. These equivalent forms also fall within the scope defined by the claims attached to this application.

## Claims

1. A compound of Formula (I), or a pharmaceutically acceptable salt, a tautomer, a stereoisomer, or a prodrug thereof:

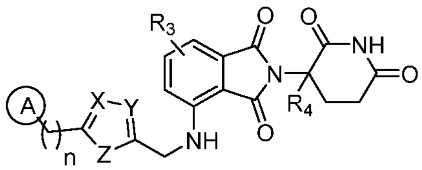

(I)

wherein, n is 0 or 1;
X and Y are each independently selected from CH and N, and X and Y are not both CH simultaneously;
Z is selected from O, S, and N;

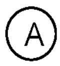

is selected from: C6-C10 aryl, C4-C10 cycloalkyl, 4-10 membered heterocyclyl, 5-10 membered heteroaryl, wherein the C6-C10 aryl, C4-C10 cycloalkyl, 4-10 membered heterocyclyl, and 5-10 membered heteroaryl are optionally substituted with one or more substituents selected from the group consisting of: hydrogen, deuterium, halogen, cyano, nitro, hydroxy, carboxy, aminocarbonyl, 4-10 membered heterocyclyl, $C_1$-$C_6$ alkyl, $C_1$-$C_6$ alkoxy, halogen-substituted $C_1$-$C_6$ alkyl, halogen-substituted $C_1$-$C_6$ alkoxy, substituted or unsubstituted phenyl, $C_2$-$C_6$ alkenyl, C3-C8 heterocyclyl-O-, C3-C8 cycloalkyl-O-, hydroxy-substituted $C_1$-$C_6$ alkoxy, $C_1$-$C_6$ alkoxy-substituted $C_1$-$C_6$ alkoxy, -C(O)-$R_1$, and -(CH2)m-$R_2$; wherein the substituted or unsubstituted phenyl is optionally substituted with one or more substituents selected from the group consisting of: hydrogen, halogen, cyano, nitro, hydroxy, carboxy, 4-10 membered heterocyclyl, C1-C6 alkyl, C1-C6 alkoxy, halogen-substituted C1-C6 alkyl, halogen-substituted C1-C6 alkoxy;

$R_1$ is selected from: halogen-substituted or unsubstituted C1-C6 alkyl, C4-C12 cycloalkyl, C6-C10 aryl, 4-10 membered heterocyclyl, and -CH2-(C4-C12 cycloalkyl), wherein the C4-C12 cycloalkyl, C6-C10 aryl, 4-10 membered heterocyclyl, and -CH2-(C4-C12 cycloalkyl) are optionally substituted with one or more substituents selected from the group consisting of: hydrogen, deuterium, halogen, cyano, nitro, hydroxy, carboxy, 4-10 membered heterocyclyl, C1-C6 alkyl, C1-C6 alkoxy, halogen-substituted C1-C6 alkyl, halogen-substituted C1-C6 alkoxy, and halogen-substituted or unsubstituted phenyl;

$R_2$ is C6-C10 aryl, and the C6-C10 aryl is optionally substituted with one or more substituents selected from the group consisting of: hydrogen, halogen, cyano, nitro, hydroxy, carboxy, 4-10 membered heterocyclyl, $C_1$-$C_6$ alkyl, $C_1$-$C_6$ alkoxy, halogen-substituted $C_1$-$C_6$ alkyl, and halogen-substituted $C_1$-$C_6$ alkoxy;
m is 0 or 1;
$R_3$ is hydrogen or halogen; and
$R_4$ is hydrogen, deuterium, or fluorine.

**2.** The compound according to claim 1, or a pharmaceutically acceptable salt, a tautomer, a stereoisomer, or a prodrug thereof, wherein the compound has the structure shown in formula II:

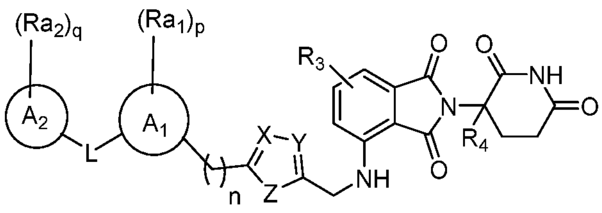

(II)

wherein, n is 0 or 1;
X and Y are independently selected from: CH or N, and X and Y are not both CH simultaneously;
Z is selected from: O, S, and NH;
$R_3$ is hydrogen or halogen;
$R_4$ is hydrogen, deuterium, or fluorine;
$A_1$ is selected from: phenyl, C4-C6 cycloalkyl, 4-6 membered heterocyclyl and 5-6 membered heteroaryl;
L is absent, CO, or $CH_2$;
$A_2$ is selected from: absent, C1-C6 alkyl, phenyl, C4-C10 cycloalkyl, 4-6 membered heterocyclyl, and 5-6 membered heteroaryl.
$Ra_1$ and $Ra_2$ are independently selected from: hydrogen, deuterium, halogen, cyano, nitro, hydroxyl, carboxyl, amino carbonyl, 4-10 membered heterocyclyl, $C_1$-$C_6$ alkyl, $C_1$-$C_6$ alkoxy, halogen-substituted $C_1$-$C_6$ alkyl, halogen-substituted $C_1$-$C_6$ alkoxy, substituted or unsubstituted phenyl, $C_2$-$C_6$ alkenyl, C3-C8 heterocyclyl-O-, C3-C8 cycloalkyl-O-, hydroxyl-substituted $C_1$-$C_6$ alkoxy, and $C_1$-$C_6$ alkoxy-substituted $C_1$-$C_6$ alkoxy; wherein the substituted or unsubstituted phenyl is optionally substituted by one or more substituents selected from the group consisting of: hydrogen, halogen, cyano, nitro, hydroxyl, carboxyl, 4-10 membered heterocyclyl, C1-C6 alkyl, C1-C6 alkoxy, halogen-substituted C1-C6 alkyl, and halogen-substituted C1-C6 alkoxy ;
p and q are independently 0, 1, 2, 3, 4, or 5.

**3.** The compound according to claim 1, or a pharmaceutically acceptable salt, a tautomer, a stereoisomer, or a prodrug thereof, wherein the compound has the structure shown in formula (I-1) to (I-4):

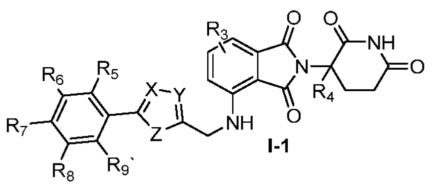

I-1

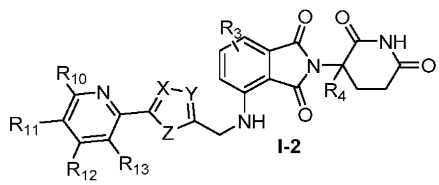

I-2

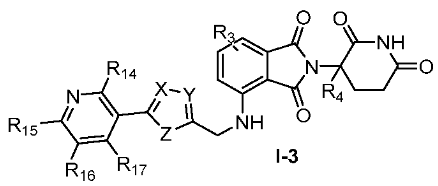

I-3

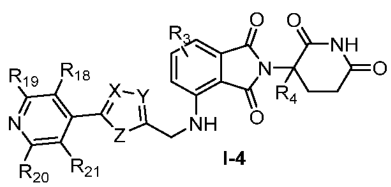

I-4

wherein

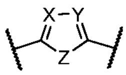

is selected from:

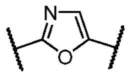, 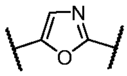, 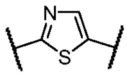, 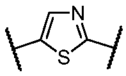,

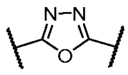 and 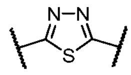;

$R_3$ is hydrogen or halogen;
$R_4$ is hydrogen, deuterium, or fluorine;
$R_5$, $R_6$, $R_7$, $R_8$, $R_9$, $R_{10}$, $R_{11}$, $R_{12}$, $R_{13}$, $R_{14}$, $R_{15}$, $R_{16}$, $R_{17}$, $R_{18}$, $R_{19}$, $R_{20}$ and $R_{21}$ are each independently selected from: hydrogen, deuterium, halogen, cyano, C1-C6 alkyl, C1-C6 alkoxy, halogen-substituted C1-C6 alkyl, halogen-substituted C1-C6 alkoxy, phenyl, vinyl, C3-C8 heterocyclyl-O-, C3-C8 cycloalkyl-O-, hydroxyl-substituted $C_1$-$C_6$ alkoxy, $C_1$-$C_6$ alkoxy-substituted $C_1$-$C_6$ alkoxy;

**4.** The compound according to claim 1, or a pharmaceutically acceptable salt, a tautomer, a stereoisomer, or a prodrug thereof, wherein the compound has the structure shown in (I-5) or (I-6):

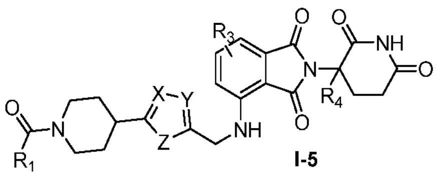

**I-5**

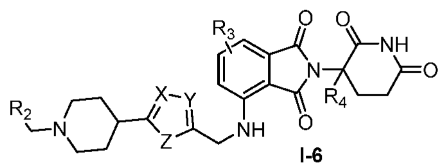

**I-6**

wherein

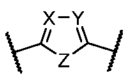

is selected from:

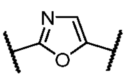, 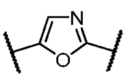, 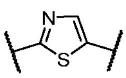, 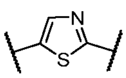,

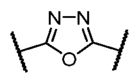 and 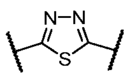;

$R_1$ is selected from: tert-butyl, cyclobutyl, cyclohexyl, phenyl,

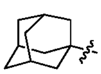 and 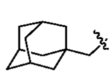,

and the cyclobutyl, cyclohexyl, and phenyl are optionally substituted with one or more substituents selected from the group consisting of: halogen, cyano, C1-C3 alkyl, trifluoromethyl, trifluoromethoxy, halogen-substituted or unsubstituted phenyl;
$R_2$ is phenyl, and the phenyl is optionally substituted with one or more substituents selected from the group consisting of: fluoro, cyano and trifluoromethoxy;
$R_3$ is hydrogen or halogen;
$R_4$ is hydrogen, deuterium, or fluorine.

**5.** The compound according to claim 1, or a pharmaceutically acceptable salt, a tautomer, a stereoisomer, or a prodrug thereof, wherein

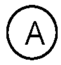

is selected from:

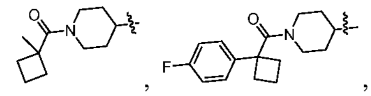
, ,

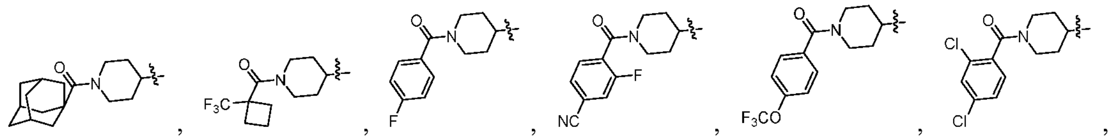
, , , , , ,

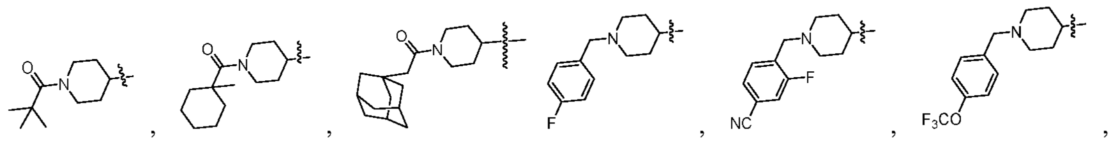
, , , , , ,

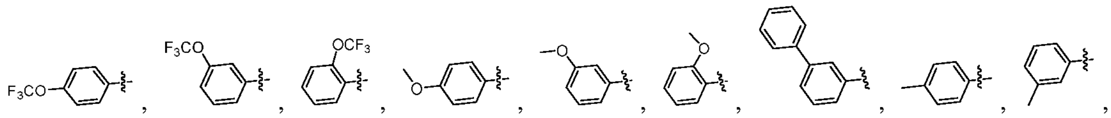
, , , , , , , , ,

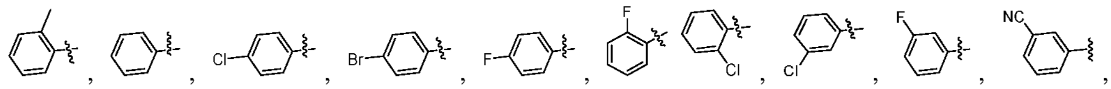
, , , , , , , , , ,

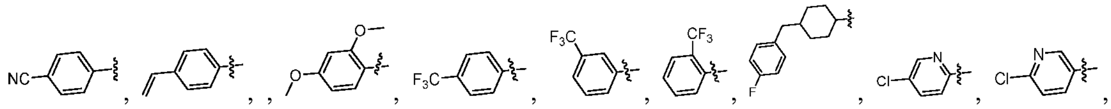
, , , , , , , , , ,

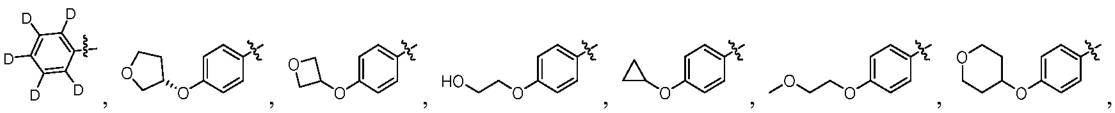
, , , , , , ,

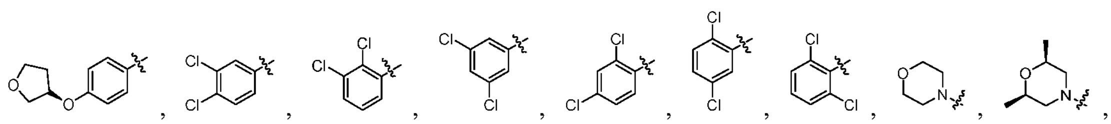
, , , , , , , , ,

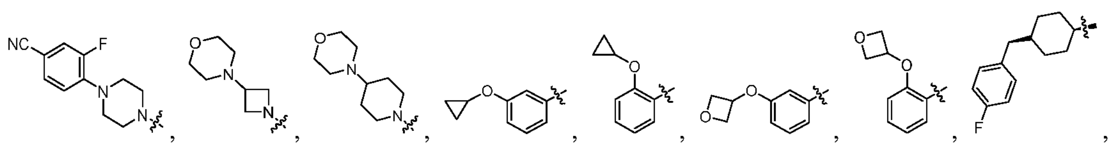
, , , , , , , ,

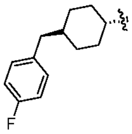 and 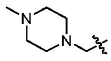.

6. The compound according to claim 1, or a pharmaceutically acceptable salt, a tautomer, a stereoisomer, or a prodrug thereof, wherein the compound is selected from the following compounds:

| Comp. No. | Compound structure | Comp. No. | Compound structure |
|---|---|---|---|
| 1 | 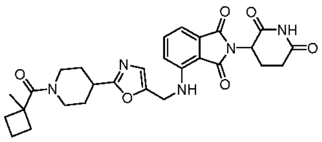 | 2 | 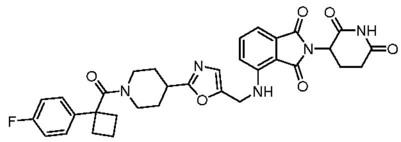 |
| 3 | 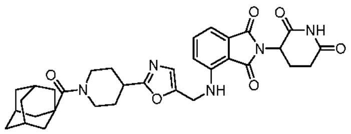 | 4 | 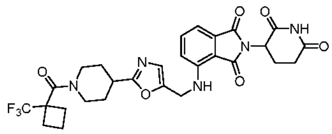 |
| 5 | 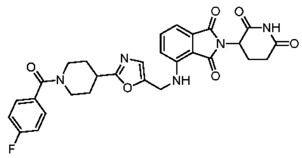 | 6 | 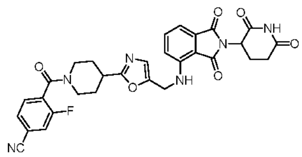 |
| 7 | 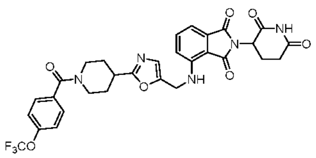 | 8 | 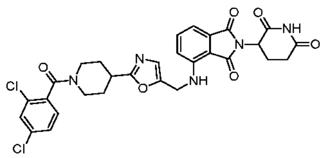 |
| 9 | 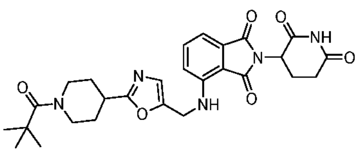 | 10 | 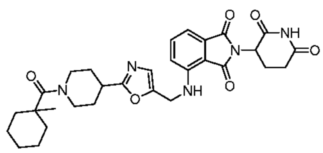 |
| 11 | 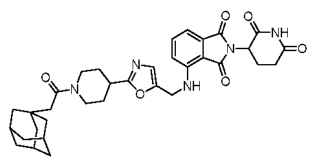 | 12 | 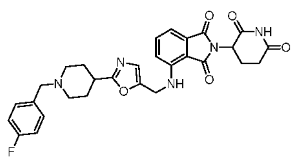 |
| 13 | 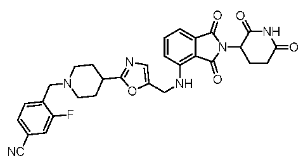 | 14 | 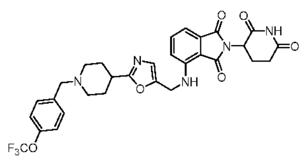 |
| 15 | 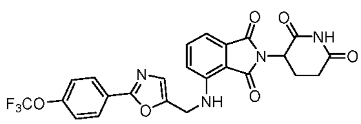 | 16 | 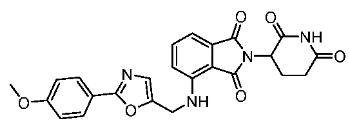 |

(continued)

| Comp. No. | Compound structure | Comp. No. | Compound structure |
|---|---|---|---|
| **17** | 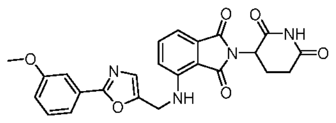 | **18** | 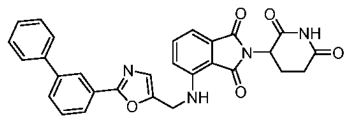 |
| **19** | 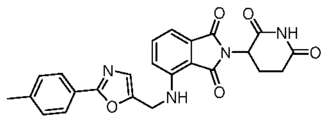 | **20** | 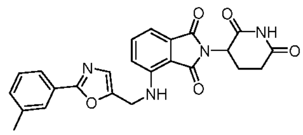 |
| **21** | 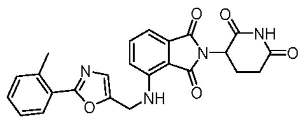 | **22** | 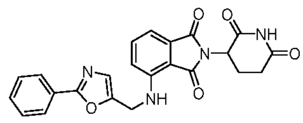 |
| **23** | 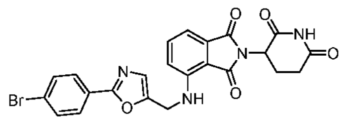 | **24** | 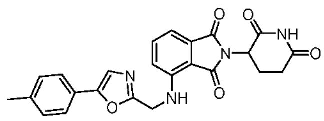 |
| **25** | 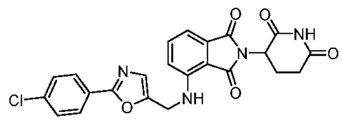 | **26** | 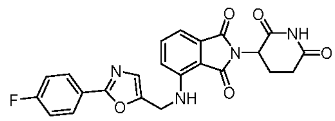 |
| **27** | 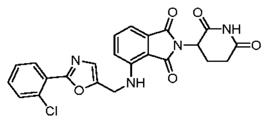 | **28** | 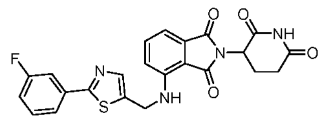 |
| **29** | 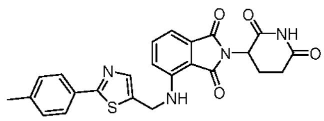 | **30** | 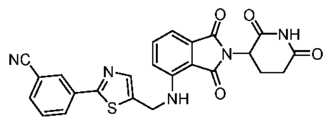 |
| **31** | 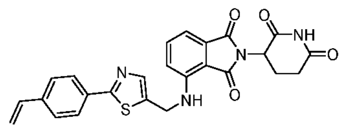 | **32** | 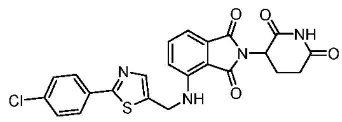 |
| **33** | 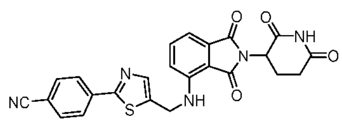 | **34** | 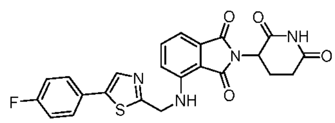 |
| **35** | 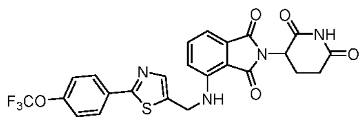 | **36** | 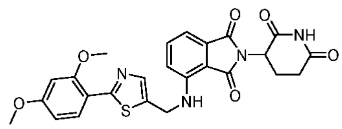 |
| **37** | 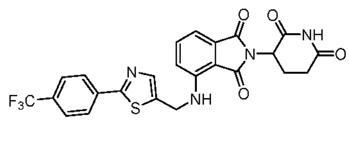 | **38** | 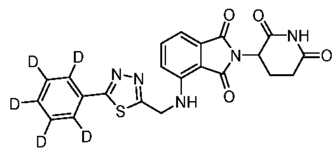 |

(continued)

| Comp. No. | Compound structure | Comp. No. | Compound structure |
|---|---|---|---|
| 39 | 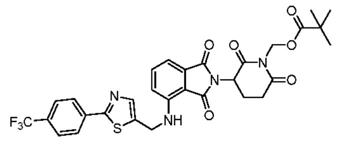 | 40 | 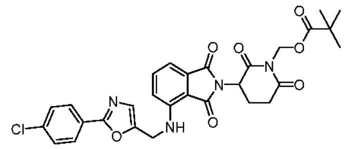 |
| 41 | 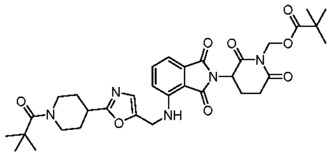 | 42 | 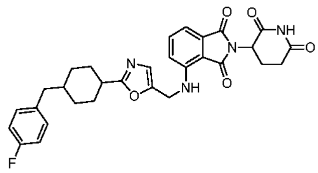 |
| 43 | 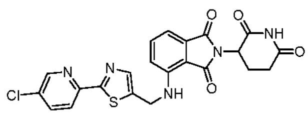 | 44 | 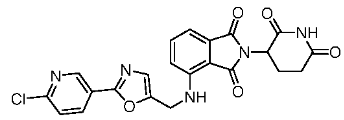 |
| 45 | 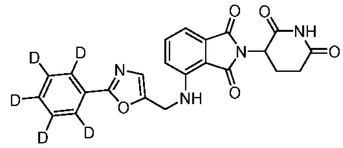 | 46 | 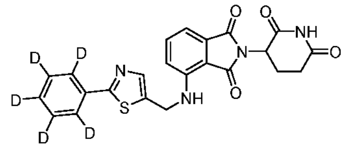 |
| 47 | 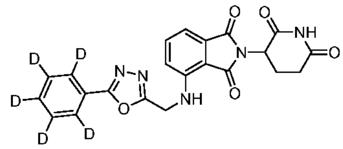 | 48 | 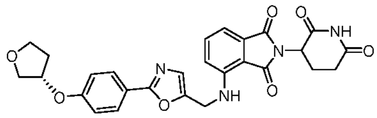 |
| 49 | 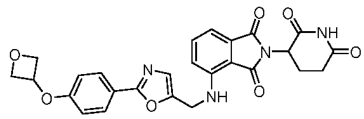 | 50 | 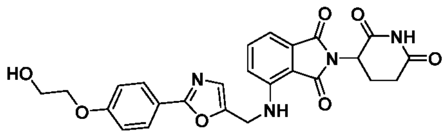 |
| 51 | 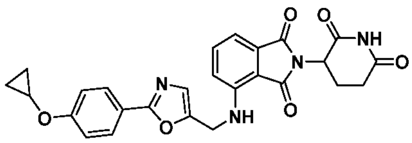 | 52 | 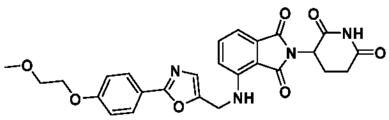 |
| 53 | 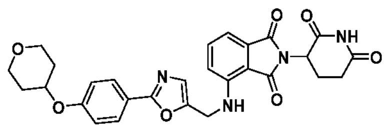 | 54 | 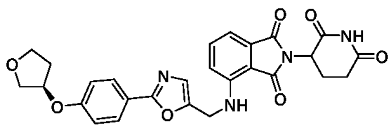 |
| 55 | 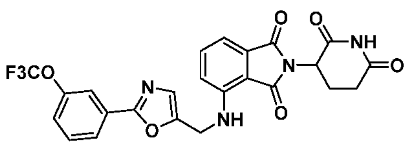 | 56 | 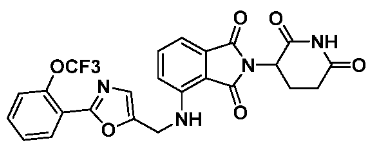 |
| 57 | 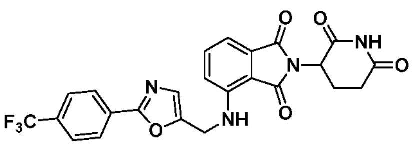 | 58 | 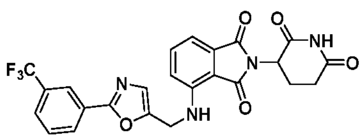 |

(continued)

| Comp. No. | Compound structure | Comp. No. | Compound structure |
|---|---|---|---|
| 59 | 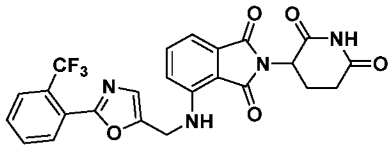 | 60 | 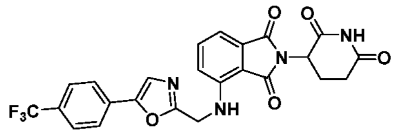 |
| 61 | 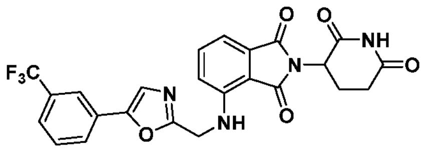 | 62 | 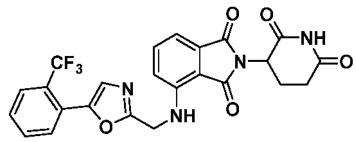 |
| 63 | 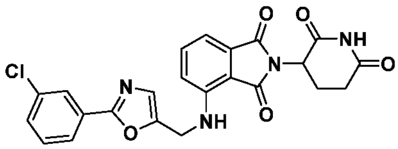 | 64 | 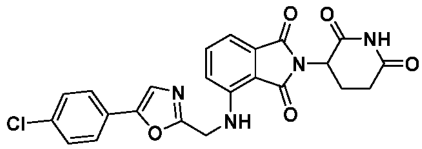 |
| 65 | 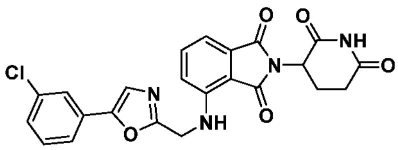 | 66 | 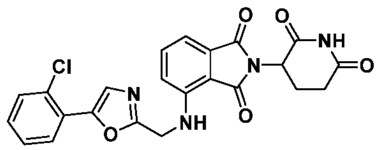 |
| 67 | 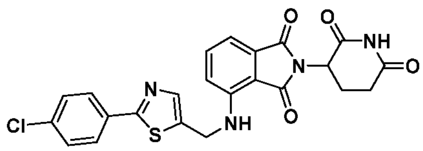 | 68 | 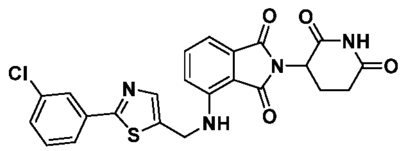 |
| 69 | 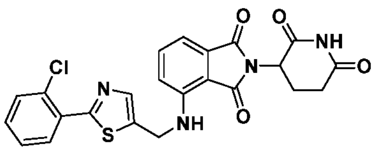 | 70 | 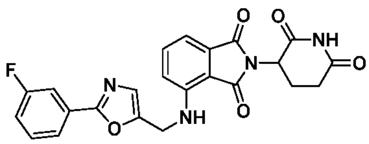 |
| 71 | 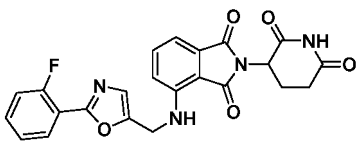 | 72 | 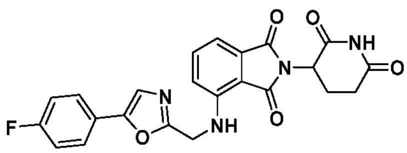 |
| 73 | 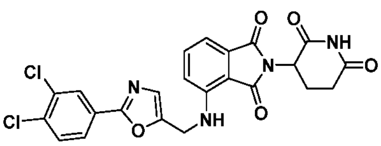 | 74 | 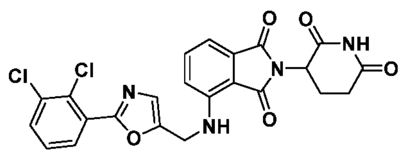 |
| 75 | 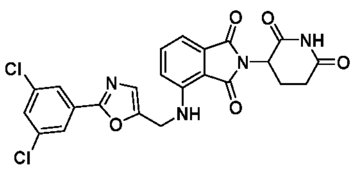 | 76 | 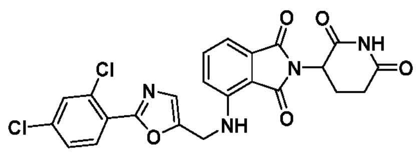 |

(continued)

| Comp. No. | Compound structure | Comp. No. | Compound structure |
|---|---|---|---|
| 77 | 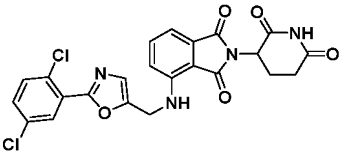 | 78 | 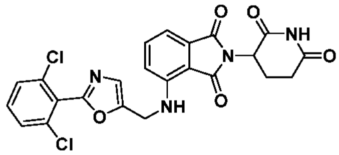 |
| 79 | 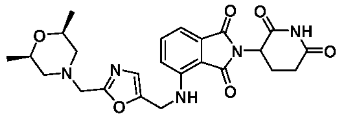 | 80 | 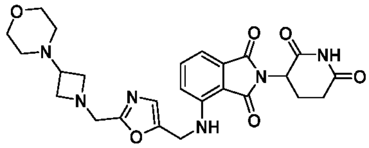 |
| 81 | 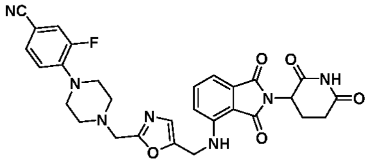 | 82 | 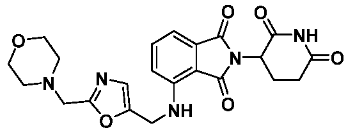 |
| 83 | 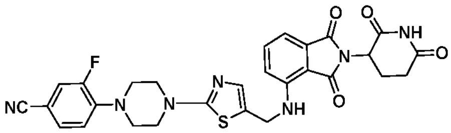 | 84 | 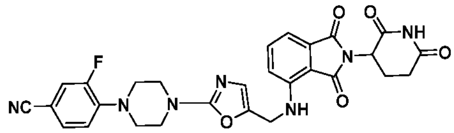 |
| 85 | 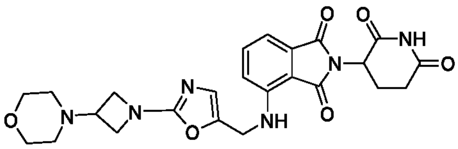 | 86 | 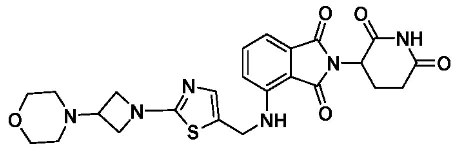 |
| 87 | 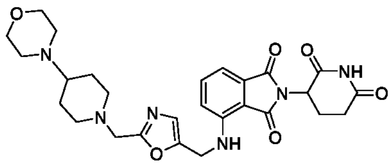 | 88 | 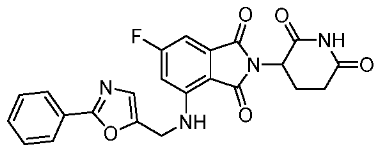 |
| 89 | 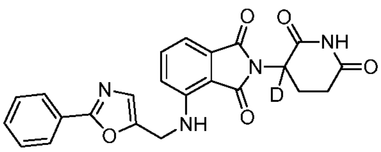 | 90 | 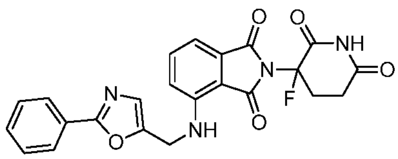 |
| 91 | 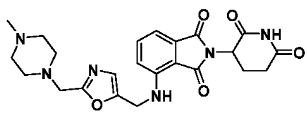 | 92 | 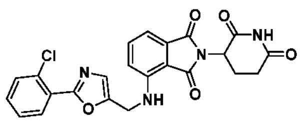 |
| 93 | 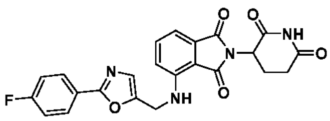 | 94 | 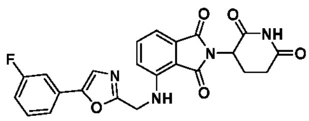 |
| 95 | 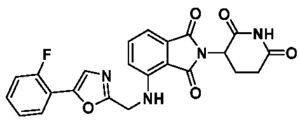 | 96 | 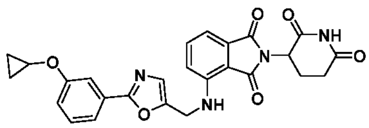 |

(continued)

| Comp. No. | Compound structure | Comp. No. | Compound structure |
|---|---|---|---|
| 97 | 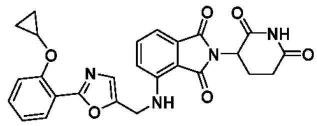 | 98 | 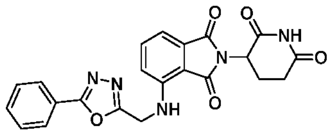 |
| 99 | 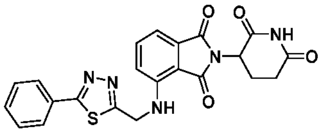 | 100 | 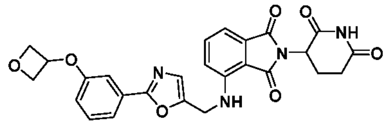 |
| 101 | 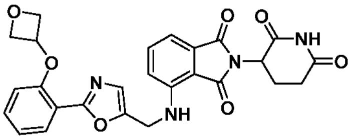 | 102 | 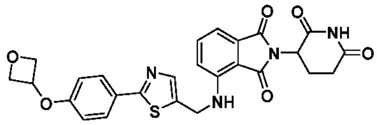 |
| 103 | 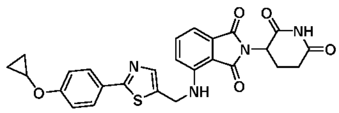 | 104 | 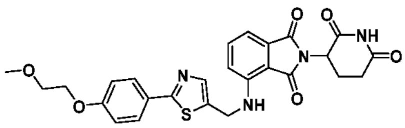 |
| 105 | 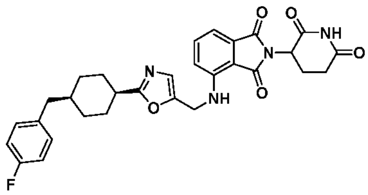 | 106 | 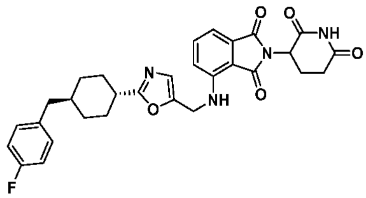. |

**7.** A method for preparing the compound according to any one of claims 1-6, wherein the method is selected from one of the following methods:
Synthesis Method 1:

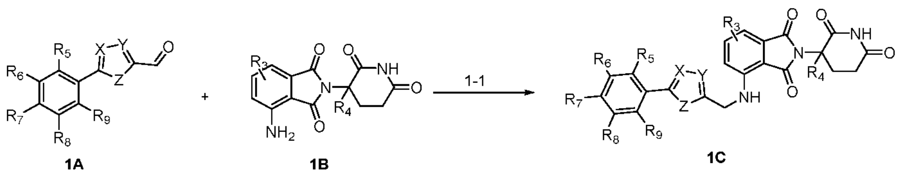


X and Y are each selected from CH and N, and X and Y are not both CH simultaneously;
Z is selected from O, S, and NH;
$R_3$, $R_4$, $R_5$, $R_6$, $R_7$, $R_8$, and $R_9$ are as defined in claim 3;
Step 1-1: Compound 1A and compound 1B react in the presence of phenylsilane and dibutyltin dichloride to yield compound 1C; or compound 1A and compound 1B react in the presence of acetic acid and sodium borohydride to yield compound 1C;
Synthesis Method 2:

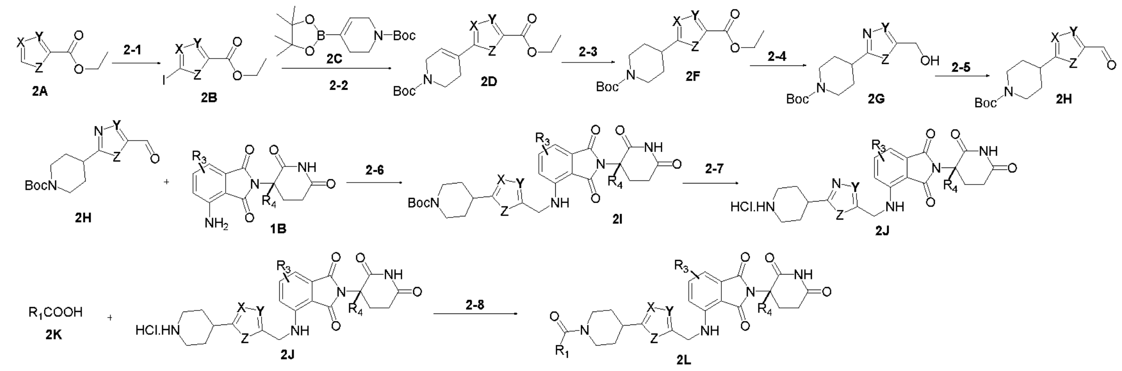


X is N, Y is CH;
Z is O;
$R_1$, $R_3$, and $R_4$ are as defined in claim 4;
Step 2-1: Compound 2A reacts with 1,2-diiodoethane to yield compound 2B;
Step 2-2: Compound 2B undergoes a coupling reaction with compound 2C to yield compound 2D;
Step 2-3: Compound 2D is hydrogenated to yield compound 2F;
Step 2-4: Compound 2F is reduced with sodium borohydride to yield compound 2G;
Step 2-5: Compound 2G reacts with an oxidizing agent to yield compound 2H;
Step 2-6: Compound 2H reacts with compound 1B in the presence of phenylsilane and dibutyltin dichloride to yield compound 2I;
Step 2-7: Compound 2I reacts in the presence of hydrochloric acid to yield compound 2J;
Step 2-8: Compound 2K and compound 2J react in the presence of HATU and N,N-diisopropylethylamine to yield compound 2L;
Synthesis Method 3:

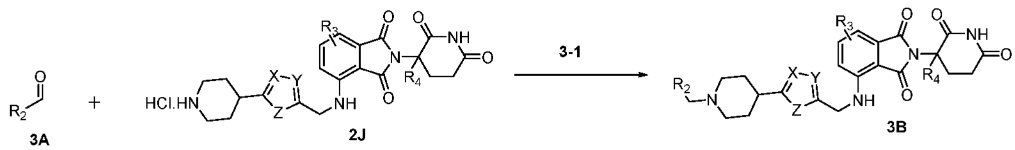


X is N, Y is CH;
Z is O;
$R_2$, $R_3$, and $R_4$ are as defined in claim 4;
Step 3-1: Compound 3A reacts with compound 2J in the presence of zinc chloride, triethylamine, and sodium cyanoborohydride to yield compound 3B;
Synthesis Method 4:

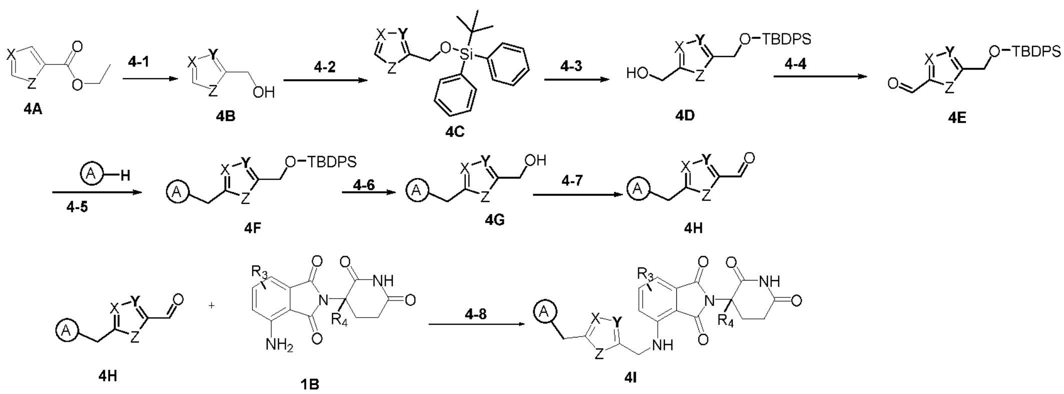


X is N, Y is CH;
Z is O;

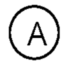

is as defined in claim 1, preferably,

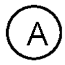

is a nitrogen-containing 4-membered or 6-membered heterocyclyl;

Step 4-1: Compound 4A reacts with lithium aluminum hydride to yield compound 4B;

Step 4-2: Compound 4B reacts with tert-butyl diphenylchlorosilane to yield compound 4C;

Step 4-3: In the presence of a borane-tetrahydrofuran complex and n-butyl lithium, compound 4C reacts with 4-formylmorpholine to yield compound 4D;

Step 4-4: Compound 4D is oxidized using Dess-Martin reagent to yield compound 4E;

Step 4-5: Compound 4E reacts with

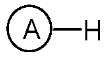

in the presence of sodium borohydride in acetic acid through reductive amination to yield compound 4F;

Step 4-6: In the presence of tetrabutylammonium fluoride in tetrahydrofuran solution, compound 4F undergoes deprotection to yield compound 4G;

Step 4-7: Compound 4G is oxidized using Dess-Martin reagent to yield compound 4H;

Step 4-8: In the presence of acetic acid and sodium borohydride, compound 4H reacts with compound 1B to yield compound 4I;

Synthesis Method 5:

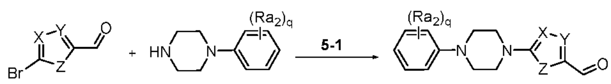


5A 5B 5C

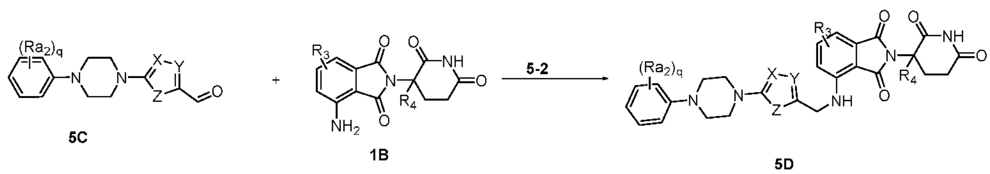


5C 1B 5D

X and Y are each selected from CH and N, and X and Y are not both be CH simultaneously;

Z is selected from O, S, and NH;

$R_3$, $R_4$, $Ra_2$, and q are as defined in claim 2;

Step 5-1: In the presence of a palladium catalyst, compound 5A undergoes a Buchwald-Hartwig amination reaction with compound 5B to yield compound 5C;

Step 5-2: In the presence of acetic acid and sodium borohydride, compound 5C undergoes reductive amination with compound 1B to yield compound 5D.

**8.** A pharmaceutical composition, comprising a therapeutically effective amount of the compound according to any one of claims 1-6, or a pharmaceutically acceptable salt, a tautomer, a stereoisomer, a prodrug, or a pharmaceutically acceptable salt thereof, and pharmaceutically acceptable carriers.

**9.** The pharmaceutical composition according to claim 8, wherein the pharmaceutical composition further comprises one or more selected from dexamethasone, rituximab, trastuzumab, PD-1 inhibitors, PDL-1 inhibitors, pemetrexed, topotecan, doxorubicin, bortezomib, gemcitabine, dacarbazine, clarithromycin, vincristine, cytarabine, prednisone, docetaxel, chlorambucil injection, HDAC inhibitors, -androgen receptor inhibitors, androgen biosynthesis inhibitors, BTK inhibitors, erythropoiesis-stimulating agents, minocycline, Elotuzumab, Palbociclib, Nivolumab, Pembrolizumab, Panobinostat, Ublituximab, Romidepsin, Eltrombopag, CAR-T, and melphalan.

**10.** A use of the compound according to any one of claims 1-6, or a pharmaceutically acceptable salt, a tautomer, a stereoisomer, a prodrug, or a pharmaceutically acceptable salt thereof in the preparation of a medicament for the prevention or treatment of diseases associated with the $CRL4^{CRBN}$ E3 ubiquitin ligase, preferably, the diseases associated with the $CRL4^{CRBN}$ E3 ubiquitin ligase are cancer, pain, central nervous system diseases, or immune system diseases.

**11.** The use according to claim 10, wherein the diseases are solid tumors or hematological malignancies, preferably, the diseases are selected from: myelodysplastic syndrome, multiple myeloma, mantle cell lymphoma, non-Hodgkin lymphoma, chronic lymphocytic leukemia, chronic myelomonocytic leukemia, myelofibrosis, Burkitt lymphoma, Hodgkin lymphoma, diffuse large B-cell lymphoma, follicular lymphoma, ciliary body and chronic melanoma, iris melanoma, recurrent bilateral ocular melanoma, T-cell lymphoma, erythroid lymphoma, monocytic and mononuclear cell leukemia, myeloid leukemia, central nervous system lymphoma, meningioma, spinal cord tumors, non-small cell lung cancer, ovarian cancer, skin cancer, renal cell carcinoma, astrocytoma, amyloidosis, type I complex regional pain syndrome, malignant melanoma, radiculopathy, glioblastoma, gliosarcoma, malignant glioma, refractory plasma cell myeloma, extraocular extending melanoma, papillary and follicular thyroid carcinoma, breast cancer, prostate cancer, hepatocellular carcinoma, and primary macroglobulinemia.

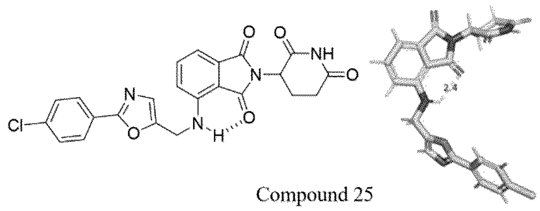

Compound 25

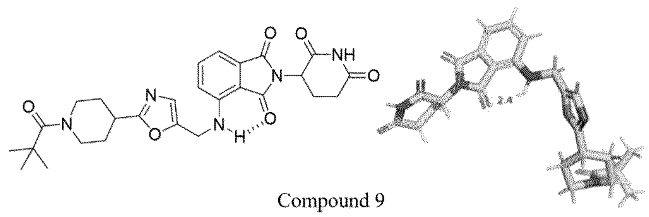

Compound 9

Structural characteristics of the compounds capable of forming intramolecular hydrogen bond according to the present invention

Figure 1

## INTERNATIONAL SEARCH REPORT

International application No. **PCT/CN2023/102216**

**A. CLASSIFICATION OF SUBJECT MATTER**

C07D413/14(2006.01)i; C07D417/14(2006.01)i; A61K31/422(2006.01)i; A61K31/427(2006.01)i; A61K31/433(2006.01)i; A61P37/02(2006.01)i; A61P35/02(2006.01)i; A61P25/00(2006.01)i; A61P29/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNTXT; ENTXT; CNABS; VEN; CNKI; STN(Registry, CAplus, Casreact): 中国科学院上海药物研究所, E3泛素连接酶, CRBN, 分子胶, 分子内氢键, 恶唑, 异吲哚林, 哌啶酮, E3 ubiquitin ligase?, molecular glue, intramolecular hydrogen bond, +oxazole+, +isoindoline+, +piperidone+, 根据权利要求1表达式进行结构检索, structure search conducted according to expression formula of claim 1

**C. DOCUMENTS CONSIDERED TO BE RELEVANT**

| Category* | Citation of document, with indication, where appropriate, of the relevant passages | Relevant to claim No. |
|---|---|---|
| X | CN 111902141 A (C4 THERAPEUTICS, INC.) 06 November 2020 (2020-11-06)<br>description, paragraphs 0019-0033, 0663-0691, 0701-0731, 0807-0853 and 1306-1344 | 1-11 |
| A | CN 111285850 A (SHANGHAI INSTITUTE OF MATERIA MEDICA, CHINESE ACADEMY OF SCIENCES) 16 June 2020 (2020-06-16)<br>description, paragraphs 0033-0061 and 0143-0144 | 1-11 |
| A | CN 109311900 A (THE REGENTS OF THE UNIVERSITY OF MICHIGAN) 05 February 2019 (2019-02-05)<br>description, paragraphs 0011-0040 and 0347 | 1-11 |
| A | CN 109790143 A (C4 THERAPEUTICS, INC.) 21 May 2019 (2019-05-21)<br>description, paragraphs 0026-0065, 0087 and 2776-2779 | 1-11 |
| A | CN 112745298 A (SHANGHAI INSTITUTE OF MATERIA MEDICA, CHINESE ACADEMY OF SCIENCES) 04 May 2021 (2021-05-04)<br>description, paragraphs 0036-0145 | 1-11 |

☐ Further documents are listed in the continuation of Box C.   ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 October 2023** | **10 October 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/102216**

| Patent document cited in search report | | | Publication date (day/month/year) | Patent family member(s) | | | Publication date (day/month/year) |
|---|---|---|---|---|---|---|---|
| CN | 111902141 | A | 06 November 2020 | EP | 3773576 | A1 | 17 February 2021 |
| | | | | EP | 3773576 | A4 | 29 December 2021 |
| | | | | US | 2021009559 | A1 | 14 January 2021 |
| | | | | WO | 2019191112 | A1 | 03 October 2019 |
| | | | | JP | 2021519337 | A | 10 August 2021 |
| | | | | KR | 20210018199 | A | 17 February 2021 |
| CN | 111285850 | A | 16 June 2020 | CA | 3122317 | A1 | 11 June 2020 |
| | | | | AU | 2019392231 | A1 | 29 July 2021 |
| | | | | AU | 2019392231 | B2 | 20 October 2022 |
| | | | | WO | 2020114482 | A1 | 11 June 2020 |
| | | | | EP | 3896062 | A1 | 20 October 2021 |
| | | | | EP | 3896062 | A4 | 15 June 2022 |
| | | | | US | 2022041578 | A1 | 10 February 2022 |
| | | | | CN | 111285850 | B | 22 April 2022 |
| CN | 109311900 | A | 05 February 2019 | WO | 2017176958 | A1 | 12 October 2017 |
| | | | | US | 2019119289 | A1 | 25 April 2019 |
| | | | | US | 10759808 | B2 | 01 September 2020 |
| | | | | JP | 2022050469 | A | 30 March 2022 |
| | | | | CA | 3020281 | A1 | 12 October 2017 |
| | | | | US | 2021002289 | A1 | 07 January 2021 |
| | | | | AU | 2017246453 | A1 | 08 November 2018 |
| | | | | EP | 3440082 | A1 | 13 February 2019 |
| | | | | AU | 2021232732 | A1 | 14 October 2021 |
| | | | | JP | 2019513746 | A | 30 May 2019 |
| | | | | JP | 7001614 | B2 | 03 February 2022 |
| CN | 109790143 | A | 21 May 2019 | WO | 2017197051 | A1 | 16 November 2017 |
| | | | | US | 2022313827 | A1 | 06 October 2022 |
| | | | | US | 2019076539 | A1 | 14 March 2019 |
| | | | | EP | 3455219 | A1 | 20 March 2019 |
| | | | | EP | 3455219 | A4 | 18 December 2019 |
| CN | 112745298 | A | 04 May 2021 | WO | 2021083328 | A1 | 06 May 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 111285850 A
- WO 2017193063 A1


### Non-patent literature cited in the description


- **J. RAUTIO et al.** *Nature Reviews Drug Discovery*, 2008, vol. 7, 255-270
- Prodrugs: Challenges and Rewards. Springer, 2007
- **GOODMAN** ; **GILMAN**. The Pharmacological Basis of Therapeutics. Pergamon
- Remington's, Pharmaceutical Sciences. Mack Publishing Co.